# EUROPEAN PATENT APPLICATION

(11) **EP 4 840 856 A1**
(43) Date of publication of application: **18.11.2026**
(21) Application number: 25739105.2
(22) Date of filing: 09.01.2025
(51) Int. Cl.: C07D 417/04, A61K 31/427, A61K 31/4439, A61K 31/422, A61P 19/02, C07D 417/14, C07D 277/46, C07D 413/04

(54) **NOVEL COMPOUND AND USES THEREOF**

(30) Priority: 09.01.2024 KR 20240003692
(71) Applicant: Proteina Co., Ltd., Seoul 08380 (KR)
(72) Inventor: CHOI, Byung San, Seoul 08223 (KR); YOON, Tae Young, Seoul 06288 (KR); KIM, Jin-Hong, Seoul 06320 (KR); YOO, Dongwon, Seoul 06583 (KR); SEO, Youngran, Suwon-si, Gyeonggi-do 16689 (KR); KIM, Tae Gyun, Seoul 08830 (KR); AHN, Jeong Hyun, Seoul 06248 (KR); AN, Hyoung Tae, Namyangju-si, Gyeonggi-do 12141 (KR); KIM, Ji Eun, Incheon 22801 (KR); CHO, Yongsik, Seoul 03306 (KR); JEONG, Sehan, Seoul 08788 (KR)
(74) Representative: ABG Intellectual Property Law, S.L.
(86) International application number: PCT/KR2025/000546
(87) International publication number: WO 2025/150943

(57) **Abstract**

The present invention provides a novel compound and a pharmaceutical composition comprising the same for preventing or treating diseases associated with reduced expression of SOX9. The compound and the composition can effectively prevent or treat diseases associated with reduced expression of SOX9.

## Description

### Technical Field

The present invention relates to a novel compound, or a stereoisomer, a solvate, or a pharmaceutically acceptable salt thereof, and to its use for preventing or treating diseases associated with the reduced expression of SOX9.

### Background Art

Osteoarthritis (OA), also widely known as degenerative arthritis, is a disease that currently afflicts more than 7% of the world's population (approximately 528 million people). Conventional treatments for osteoarthritis are largely focused on artificial joint replacement surgery and pain-relief therapies based on prescriptions of cartilage-protecting agents, such as hyaluronic acid, and anti-inflammatory drugs. However, due to population aging, the incidence of osteoarthritis is increasing worldwide. As a result, there is a demand for the development of direct osteoarthritis therapeutics rather than mere pain relief. One of the causative factors of osteoarthritis is the gradual loss of cartilage that arises from destruction of articular cartilage tissue caused by dysregulation of the enzymes of the anabolic and catabolic pathways of articular cartilage proteins. Accordingly, research is being conducted to inhibit the progression of osteoarthritis through the development of disease-modifying OA drugs (DMOADs) that target these regulatory factors. The recently developed Lorecivivint (SM04690) is one example of an osteoarthritis DMOAD.

SOX9 (SRY-Box Transcription Factor 9), one of the atypical proteins that play a key role in regulating the expression of extracellular matrix genes within chondrocytes, is known to regulate the expression of cartilaginous extracellular matrix genes, such as collagens, which are essential for articular cartilage. In particular, in osteoarthritis patient groups, reductions in the expression and transcriptional activity of SOX9 have been identified. The expression levels of SOX9 and its target proteins have been observed to decrease markedly with the severity of the disease. One of the mechanisms by which the activity of a transcription factor is regulated is the condensation of transcription factors. As condensation is induced, the resulting transcription factor condensates can regulate the expression of genes. That is, when the condensation of the SOX9 transcription factor is induced, the expression and transcriptional effect of SOX9 can be increased through the condensates.

Therefore, there is a need for research and development of DMOADs capable of directly treating osteoarthritis by inducing the condensation of the SOX9 transcription factor and thereby increasing the transcriptional efficiency of SOX9.

### Detailed Description of the Invention

### Technical Problem

One object of the present invention is to provide a novel compound, or a stereoisomer, a solvate, or a pharmaceutically acceptable salt thereof, that is capable of inducing the condensation of the SOX9 transcription factor.

Another object of the present invention is to provide a pharmaceutical composition for preventing or treating diseases associated with reduced expression of SOX9, by using a novel compound, or a stereoisomer, a solvate, or a pharmaceutically acceptable salt thereof, that is capable of inducing condensation of the SOX9 transcription factor.

### Solution to the Problem

Each description and embodiment disclosed in the present specification can be applied to other descriptions and embodiments. That is, all combinations of the various elements disclosed in the present specification fall within the scope of the present invention. Further, the scope of the present invention is not to be regarded as limited by the specific descriptions provided below.

One aspect of the present invention provides a compound represented by Formula 1 below, or a stereoisomer, a solvate, or a pharmaceutically acceptable salt thereof.

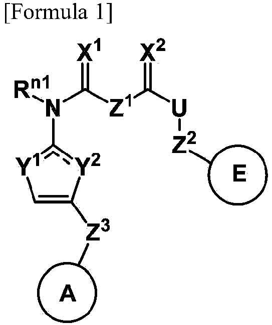

In Formula 1, one of Y¹ and Y² is N, and the other is S, O, or NR^{a1}.

In Formula 1, X¹ and X² are each independently O, S, or NR^{a2}.

In Formula 1, U is NRⁿ², a 5- to 7-membered heterocyclyl containing 1 to 3 heteroatoms N, or a moiety in which these are linked to each other.

In Formula 1, Z¹ is C₁₋₆ alkylene.

In Formula 1, Z² is a direct bond, C₁₋₆ alkylene, -NRⁿ³CO-, or a 5- to 7-membered heteroaryl containing 1 to 3 heteroatoms selected from N, O, and S.

In Formula 1, R^{a1} and R^{a2} are each independently H or C₁₋₆ alkyl.

In Formula 1, Rⁿ¹, Rⁿ², and Rⁿ³ are each independently H or C₁₋₆ alkyl.

In Formula 1, when Z² is C₁₋₆ alkylene, any carbon of the alkylene is optionally substituted with halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl.

In Formula 1, Z³ is a direct bond or -C(=O)-.

In Formula 1, Ring A is a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S.

In Formula 1, Ring A is optionally substituted with 1 to 3 R^{A}.

R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo.

In Formula 1, Ring E is a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl.

In Formula 1, Ring E is optionally substituted with 1 to 3 R^{E}.

R^{E} is selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano.

In Formula 1, one of Y¹ and Y² may be N, and the other may be S, O, or NR^{a1}. For example, Y¹ may be N, and Y² may be S, O, or NR^{a1}; or Y² may be N, and Y¹ may be S, O, or NR^{a1}. In some embodiments, one of Y¹ and Y² may be N, and the other may be S or O. For example, Y¹ may be N, and Y² may be S; or Y¹ may be S or O, and Y² may be N.

In Formula 1, X¹ and X² may each independently be O, S, or NR^{a2}. X¹ and X² may be the same or different. For example, X¹ and X² may each be O.

In Formula 1, U may be NRⁿ², a 5- to 7-membered heterocyclyl containing 1 to 3 heteroatoms N, or a moiety in which these are linked to each other. In some embodiments, U may be NRⁿ², a 5- or 6-membered heterocyclyl containing 1 or 2 heteroatoms N, or a moiety in which these are linked to each other.

In some embodiments, U may be selected from NRⁿ²; piperidinediyl; piperazinediyl; or a moiety in which NR²ⁿ is linked to piperidinediyl or piperazinediyl.

In some embodiments, U may be selected from the group consisting of NRⁿ² and the structures shown below. In each of the structures below, *¹ may be connected to the carbon atom of -C(=X²)- in Formula 1, and *² may be connected to Z².

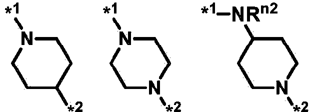

R^{a1} and R^{a2} may each independently be H or C₁₋₆ alkyl. In some embodiments, R^{a1} and R^{a2} may each independently be H or C₁₋₄ alkyl. For example, R^{a1} and R^{a2} may each independently be H or methyl.

In Formula 1, Rⁿ¹, Rⁿ², and Rⁿ³ may each independently be H or C₁₋₆ alkyl. In some embodiments, Rⁿ¹, Rⁿ², and Rⁿ³ may each independently be H or C₁₋₄ alkyl. For example, Rⁿ¹, Rⁿ², and Rⁿ³ may each independently be H or methyl.

In Formula 1, Z¹ may be C₁₋₆ alkylene. In some embodiments, Z¹ may be C₁₋₄ alkylene. In some embodiments, Z¹ may be a linear alkylene. In some embodiments, Z¹ may be methylene, ethylene, propylene, butylene, pentylene, or hexylene.

In Formula 1, Z² may be a direct bond, C₁₋₆ alkylene, -NRⁿ³CO-, or a 5- to 7-membered heteroaryl containing 1 to 3 heteroatoms selected from N, O, and S. -NRⁿ³CO- may be *³-NRⁿ³CO-*⁴, where *³ may be connected to U in Formula 1, and *⁴ may be connected to Ring E. In some embodiments, Z² may be a direct bond, C₁₋₄ alkylene, -NRⁿ³CO-, or a 5- or 6-membered heteroaryl containing 1 to 3 heteroatoms selected from N, O, and S. For example, Z² may be a direct bond, methylene, ethylene, propylene, butylene, -NHCO-, or dithiazolediyl.

In Formula 1, when Z² is C₁₋₆ alkylene, any carbon of the alkylene may be optionally substituted with halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl. In some embodiments, any carbon of the alkylene may be optionally substituted with carboxy, C₁₋₆ alkoxycarbonyl, or C₆₋₁₂ aryl. For example, any carbon of the alkylene may be substituted with methoxycarbonyl or phenyl. The carbon atom of the alkylene of Z² that is adjacent to Ring E may be substituted.

In some embodiments, when U is a 5- to 7-membered heterocyclyl containing 1 to 3 heteroatoms N, or a moiety in which NRⁿ² and a 5- to 7-membered heterocyclyl containing 1 to 3 heteroatoms N are linked to each other, Z² may be a direct bond or C₁₋₆ alkylene.

In Formula 1, Z³ may be a direct bond or -C(=O)-.

In some embodiments, the compound represented by Formula 1 may be selected from the compounds represented by Formula 2 or Formula 3 below.

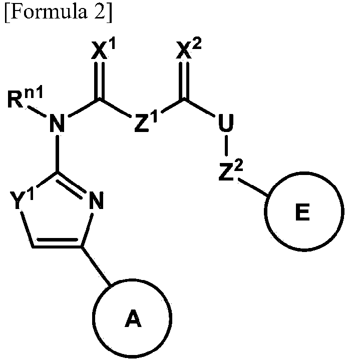

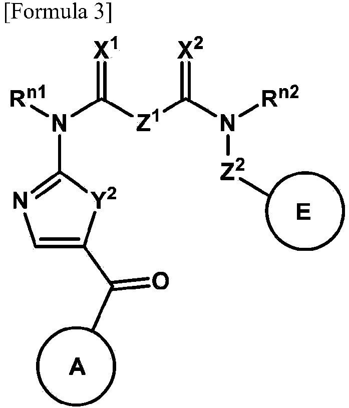

In Formulae 2 and 3, Z¹, Z², X¹, X², U, Rⁿ¹, Rⁿ², Ring A, and Ring E are as defined in Formula 1.

In some embodiments, Y¹ in Formula 2 and Y² in Formula 3 may each independently be S or O.

In Formula 1, Ring A may be a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S. In some embodiments, Ring A may be a 6- to 12-membered aryl, a partially unsaturated 9- or 10-membered bicyclic carbocyclyl, a 6- to 10-membered heteroaryl containing 1 or 2 heteroatoms selected from O, N, or S, a 5- to 7-membered heterocyclyl containing 1 or 2 heteroatoms selected from O, N, or S, or a fused benzoheterocyclyl in which a benzene ring is fused to a 5- to 7-membered heterocyclyl containing 1 or 2 heteroatoms selected from O, N, or S.

In some embodiments, Ring A may be phenyl, biphenylyl, tetrahydronaphthyl, pyranyl, pyranonyl (oxopyranyl), benzopyranyl, or benzopyronyl. The benzopyronyl may be coumarinyl.

In some embodiments, Ring A may be 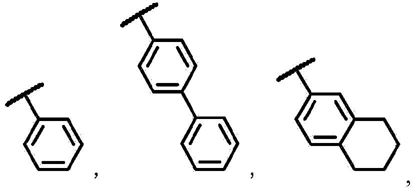 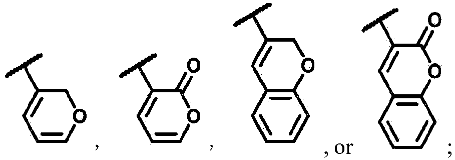 and Ring A may be optionally substituted with 1 to 3 R^{A}.

In some embodiments, Z³ may be a direct bond, and Ring A may be

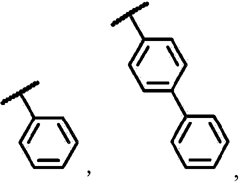

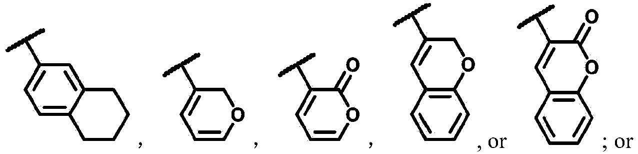

Z³ may be -C(=O)-, and Ring A may be

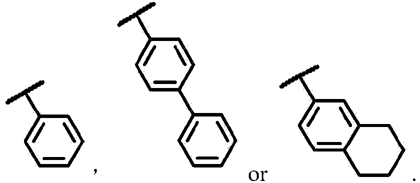

In Formula 1, Ring A may be optionally substituted with 1 to 3 R^{A}. R^{A} may be selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo. In some embodiments, R^{A} may be selected from the group consisting of halo, hydroxy, C₁₋₆ alkoxy, amino, C₁₋₆ alkylamino, di(C₁₋₆ alkyl)amino, C₁₋₆ alkyl, and oxo.

In some embodiments, R^{A} may be selected from the group consisting of halo, hydroxy, C₁₋₄ alkoxy, amino, C₁₋₄ alkylamino, di(C₁₋₄ alkyl)amino, C₁₋₄ alkyl, cyano, nitro, and oxo.

In some embodiments, R^{A} may be selected from the group consisting of F, Cl, Br, hydroxy, methoxy, dimethylamino, methyl, and oxo.

In some embodiments, Ring A may be selected from the following chemical structures:

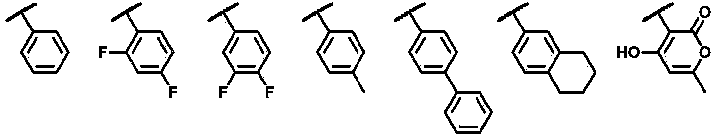

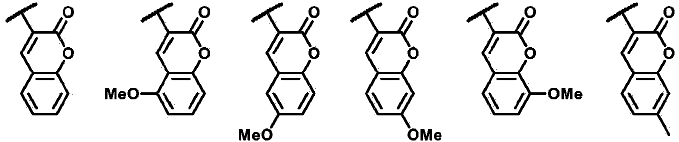

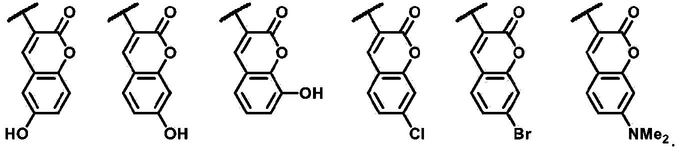

In Formula 1, Ring E may be a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl. In some embodiments, Ring E may be a 6- to 12-membered aryl, a partially unsaturated 9- to 12-membered bicyclic carbocyclyl, a 5- to 12-membered heteroaryl containing 1 or 2 heteroatoms selected from O, N, or S, a 5- to 8-membered heterocyclyl containing 1 or 2 heteroatoms selected from O, N, or S, or a fused heterocycloaryl in which a 5- to 7-membered heterocyclic ring containing 1 or 2 heteroatoms selected from O, N, or S is fused to phenyl. In some embodiments, Ring E may be a 6- to 10-membered aryl, a partially unsaturated 9- or 10-membered bicyclic carbocyclyl, a 5- to 10-membered heteroaryl containing 1 or 2 heteroatoms selected from O, N, or S, a 5- to 7-membered heterocyclyl containing 1 or 2 heteroatoms selected from O, N, or S, or a fused heterocycloaryl in which a 5- or 6-membered heterocyclic ring containing 1 or 2 heteroatoms selected from O, N, or S is fused to phenyl.

In some embodiments, Ring E may be phenyl, naphthalenyl (naphthyl), indanyl, pyridinyl, pyridazinyl, pyrimidinyl, pyrazinyl, imidazolyl, pyrazolyl, triazolyl, thiophenyl, thiazolyl, isothiazolyl, oxazolyl, isoxazolyl, morpholinyl, thiomorpholinyl, piperidinyl, hexahydroxypyridazinyl, hexahydroxypyrimidinyl, piperazinyl, indolyl, indazolyl, benzoimidazolyl, quinolinyl, isoquinolinyl, cinnolinyl, quinazolinyl, quinoxalinyl, phthalazinyl, or benzodioxolyl. For example, Ring E may be phenyl, naphthalenyl (naphthyl), pyridinyl, pyridazinyl, pyrimidinyl, imidazolyl, thiophenyl, morpholinyl, thiomorpholinyl, piperazinyl, indolyl, quinolinyl, or benzodioxolyl.

In some embodiments, Ring E may be selected from the following chemical structures; and Ring E may be optionally substituted with 1 to 3 R^{E}.

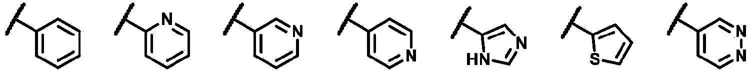

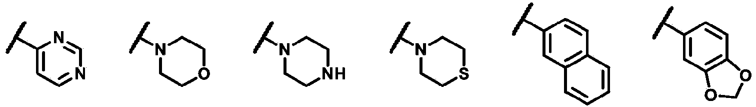

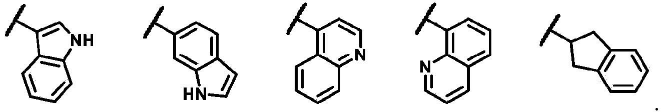

In Formula 1, Ring E may be optionally substituted with 1 to 3 R^{E}. When two or more R^{E}s are present, each R^{E} may be the same as, or different from, one another.

R^{E} may be selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano. In some embodiments, R^{E} is selected from the group consisting of halo; hydroxy; C₁₋₄ alkoxy; amino; C₁₋₄ alkylamino; di(C₁₋₄ alkyl)amino; carboxy; C₁₋₄ alkoxycarbonyl; carbamoyl; C₁₋₄ alkylcarbamoyl; di(C₁₋₄ alkyl)carbamoyl; halosulfonyl; sulfoxy; C₁₋₆ alkylsulfonyl; cyano; nitro; oxo; or C₁₋₄ alkyl optionally substituted with one or more selected from halo, hydroxy, C₁₋₄ alkoxy, amino, C₁₋₄ alkylamino, di(C₁₋₄ alkyl)amino, nitro, and cyano.

In some embodiments, R^{E} may be selected from the group consisting of halo; hydroxy; C₁₋₆ alkoxy; amino; C₁₋₆ alkylamino; di(C₁₋₆ alkyl)amino; halosulfonyl; sulfoxy; C₁₋₆ alkylsulfonyl; cyano; nitro; oxo; C₁₋₆ alkyl, C₁₋₆ haloalkyl, and C₁₋₆ hydroxyalkyl. In some embodiments, R^{E} may be selected from the group consisting of halo; hydroxy; C₁₋₄ alkoxy; amino; C₁₋₄ alkylamino; di(C₁₋₄ alkyl)amino; halosulfonyl; sulfoxy; C₁₋₄ alkylsulfonyl; cyano; nitro; oxo; C₁₋₄ alkyl, C₁₋₄ haloalkyl, and C₁₋₄ hydroxyalkyl.

In some embodiments, R^{E} may be selected from the group consisting of methoxy, hydroxy, amino, F, Cl, Br, trifluoromethyl, cyano, hydroxymethyl, methyl, fluorosulfonyl, nitro, and oxo.

In some embodiments, Ring E may be selected from the following chemical structures:

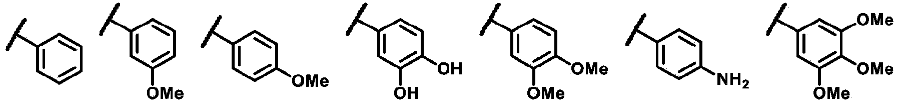

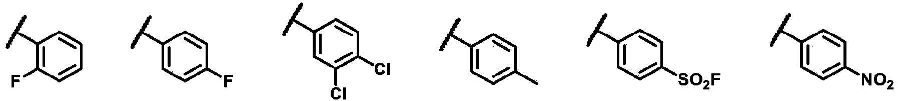

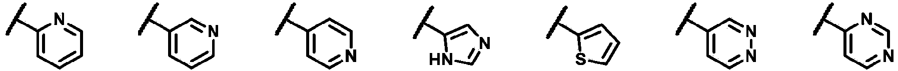

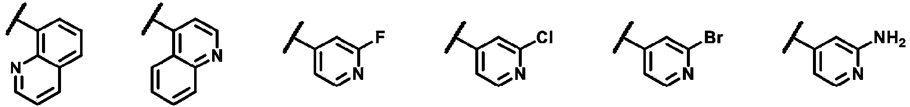

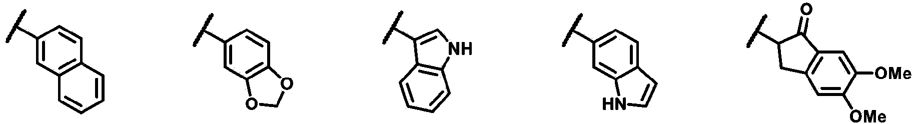

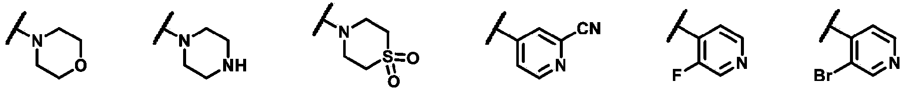

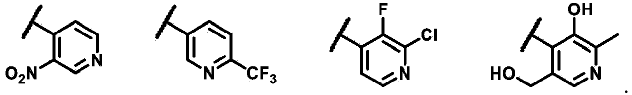

In some embodiments, the compound represented by Formula 1 may be represented by Formula I to Formula III below.

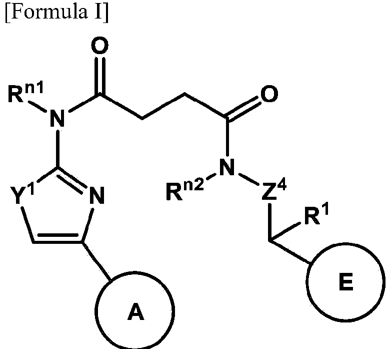

In Formula I, Y¹ is S, O, or NR^{a1}; R^{a1} is H or C₁₋₆ alkyl; Rⁿ¹ and Rⁿ² are each independently H or C₁₋₆ alkyl; Z⁴ is a direct bond or C₁₋₄ alkylene; R¹ is H, halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl.

In Formula I, Ring A is a 6- to 14-membered aryl, a partially unsaturated 9- to 14-membered bicyclic carbocyclyl, a 6- to 12-membered heteroaryl containing 1 to 3 heteroatoms selected from O, N, or S, a 5- to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S; Ring A is optionally substituted with 1 to 3 R^{A}; and R^{A} may be selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo.

In Formula I, Ring E is a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl; Ring E is optionally substituted with 1 to 3 R^{E}; and R^{E} may be selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano.

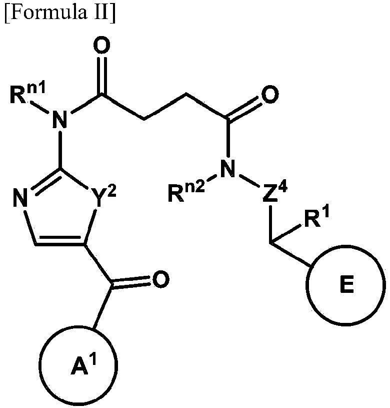

In Formula II, Y² is S, O, or NR^{a1}; R^{a1} is H or C₁₋₆ alkyl; Rⁿ¹ and Rⁿ² are each independently H or C₁₋₆ alkyl; Z⁴ is a direct bond or C₁₋₄ alkylene; R¹ is H, halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl.

In Formula II, Ring A¹ is a 6- to 14-membered aryl or a partially unsaturated 9- to 14-membered bicyclic carbocyclyl; Ring A¹ is optionally substituted with 1 to 3 R^{A}; and R^{A} may be selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo. In some embodiments, Ring A¹ may be a 6- to 14-membered aryl.

In Formula II, Ring E is a 6- to 14-membered aryl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl; Ring E is optionally substituted with 1 to 3 R^{E}; and R^{E} is selected from the group consisting of halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, halosulfonyl, sulfoxy, C₁₋₆ alkylsulfonyl, cyano, nitro, and oxo.

In some embodiments, the compound represented by Formula 1 may be selected from compounds represented by Formula IA, Formula IB, Formula IC, Formula ID, Formula IE, or Formula IIA below.

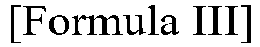

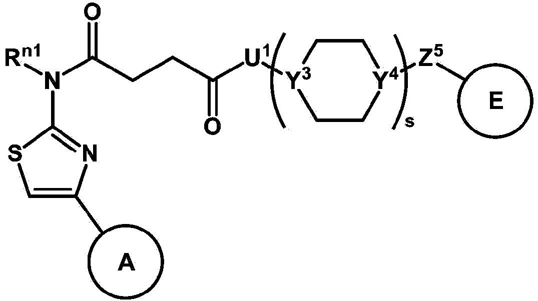

In Formula III, U¹ is a direct bond or NRⁿ²; at least one of Y³ and Y⁴ is N, and the other is CH; Z⁵ is a direct bond, C₁₋₆ alkylene, -NRⁿ³CO-, or a 5- to 7-membered heteroaryl containing 1 to 3 heteroatoms selected from N, O, and S; Rⁿ¹, Rⁿ², and Rⁿ³ are each independently H or C₁₋₆ alkyl; and s is 0 or 1.

In Formula III, Ring A may be a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S.

In Formula III, Ring A is optionally substituted with 1 to 3 R^{A}; and R^{A} may be selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo.

In Formula III, Ring E may be a 6- to 14-membered aryl, a partially unsaturated 9- to 14-membered bicyclic carbocyclyl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl.

In Formula III, Ring E is optionally substituted with 1 to 3 R^{E}; and R^{E} may be selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano.

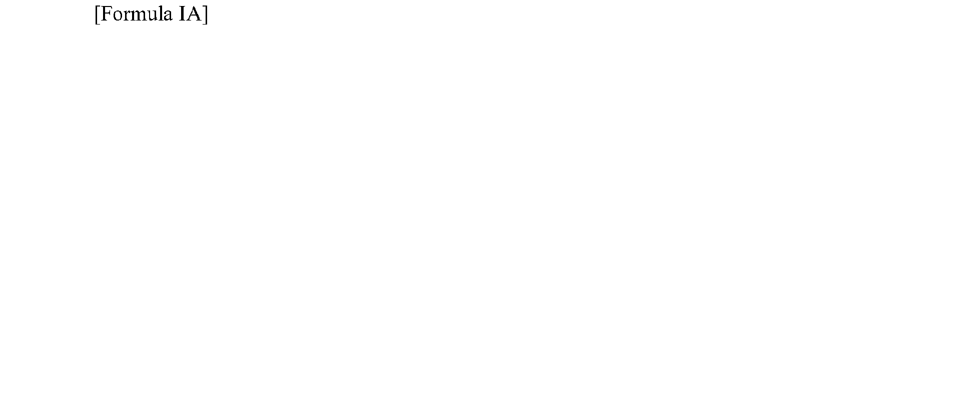

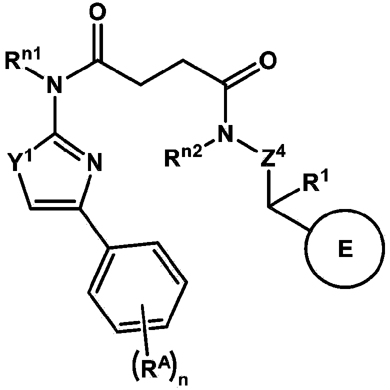

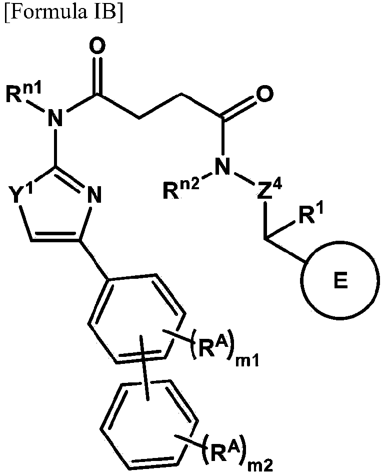

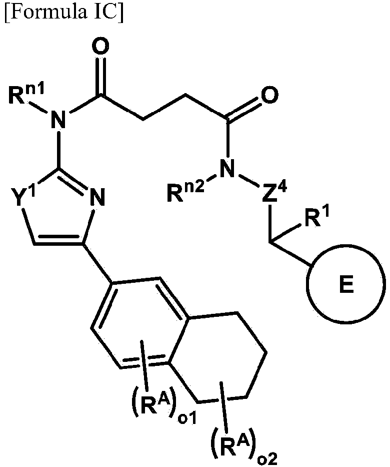

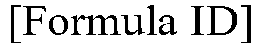

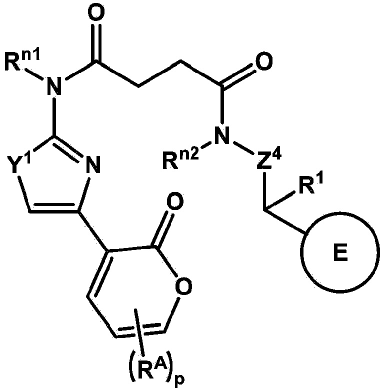

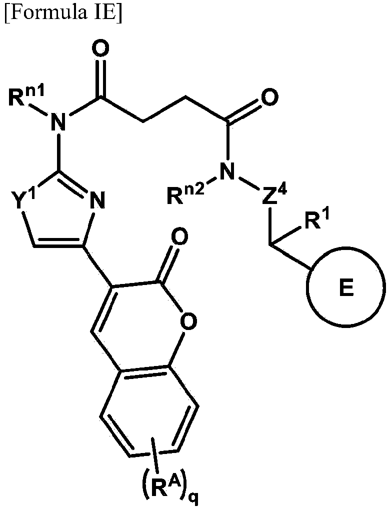

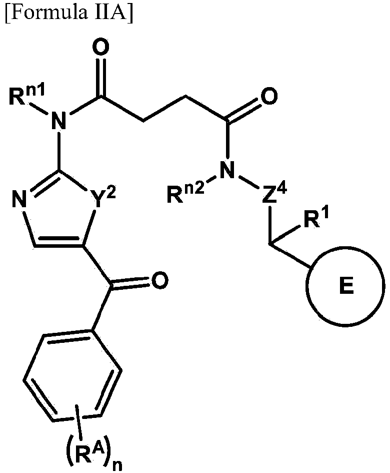

In Formulae IA, IB, IC, ID, IE, and IIA, R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo; n, m1, m2, o1, o2, p, and q are each independently an integer of 0 to 3; the sum of m1 and m2 and the sum of o1 and o2 are each 3 or less; and Y¹, Y², Rⁿ¹, Rⁿ², Z⁴, R¹, and Ring E are as described in Formula I and Formula II above.

In some embodiments, the compound represented by Formula 1 may be selected from the compounds represented by the following chemical structures:

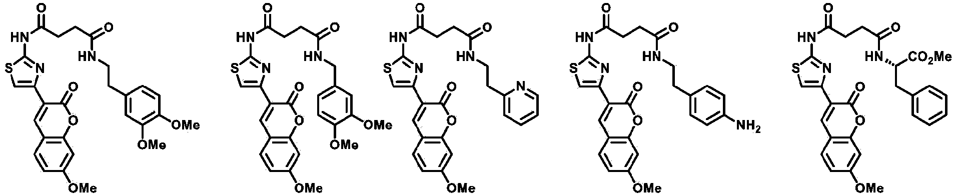

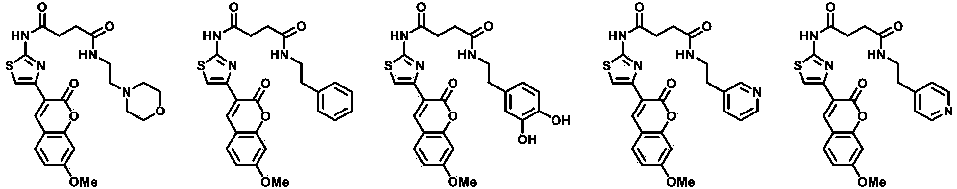

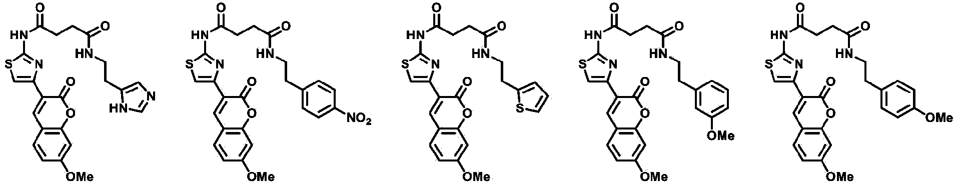

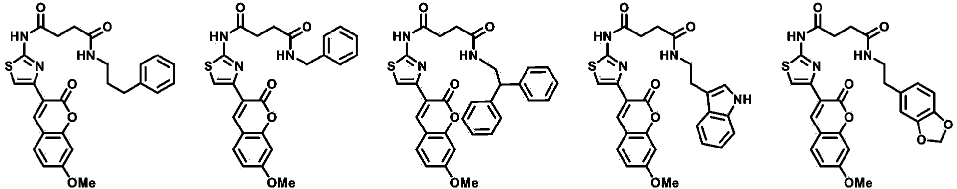

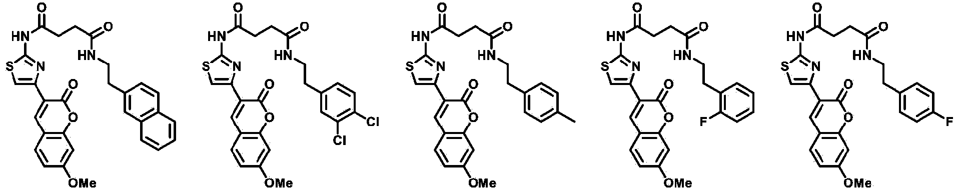

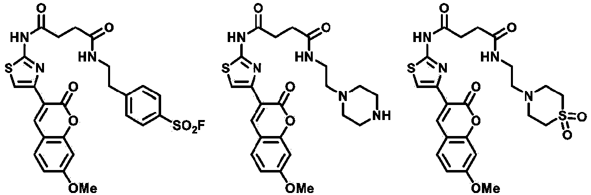

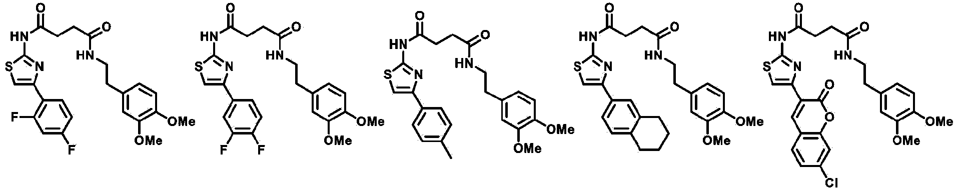

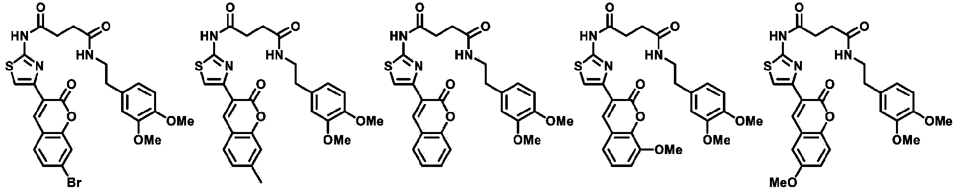

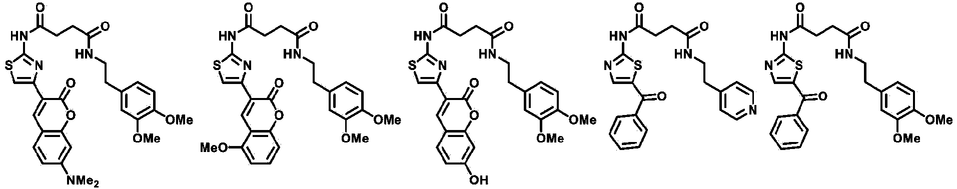

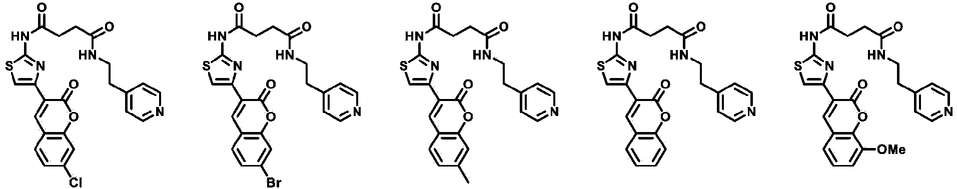

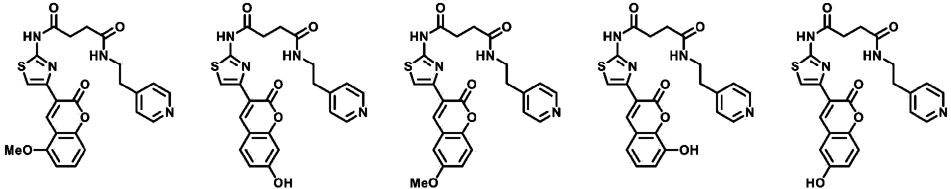

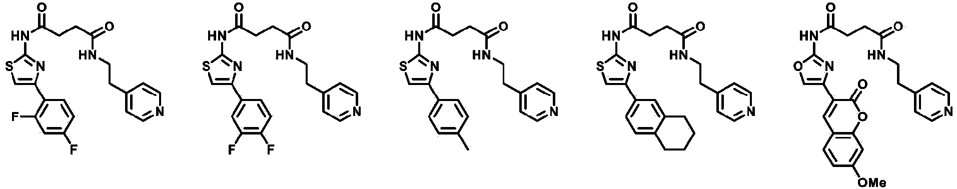

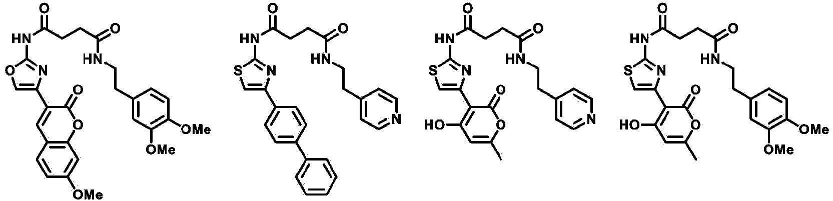

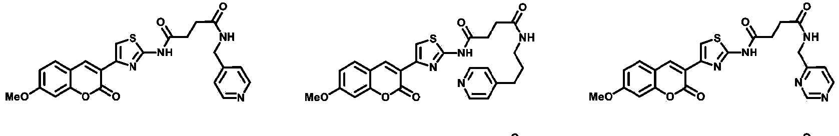

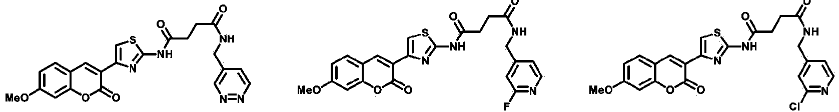

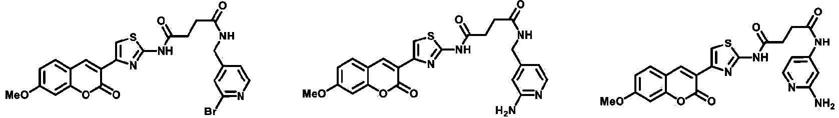

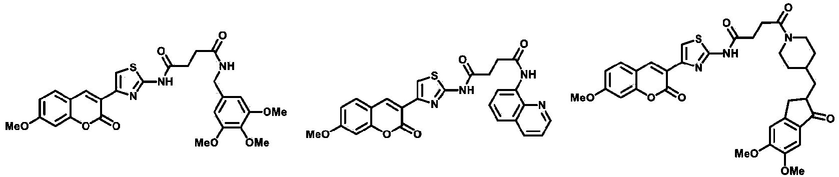

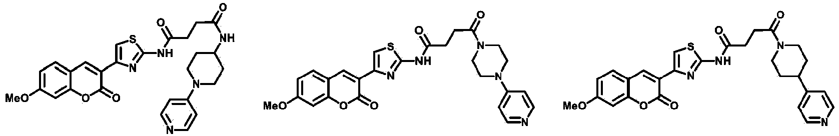

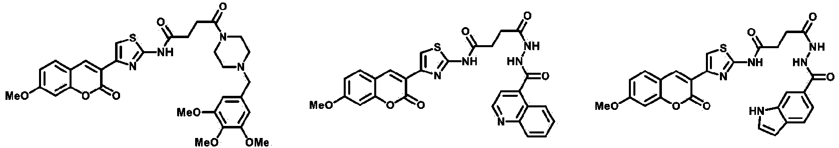

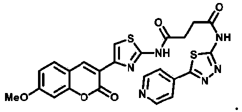

In some embodiments, the compound represented by Formula 1 may be synthesized according to Reaction Scheme 1 below.

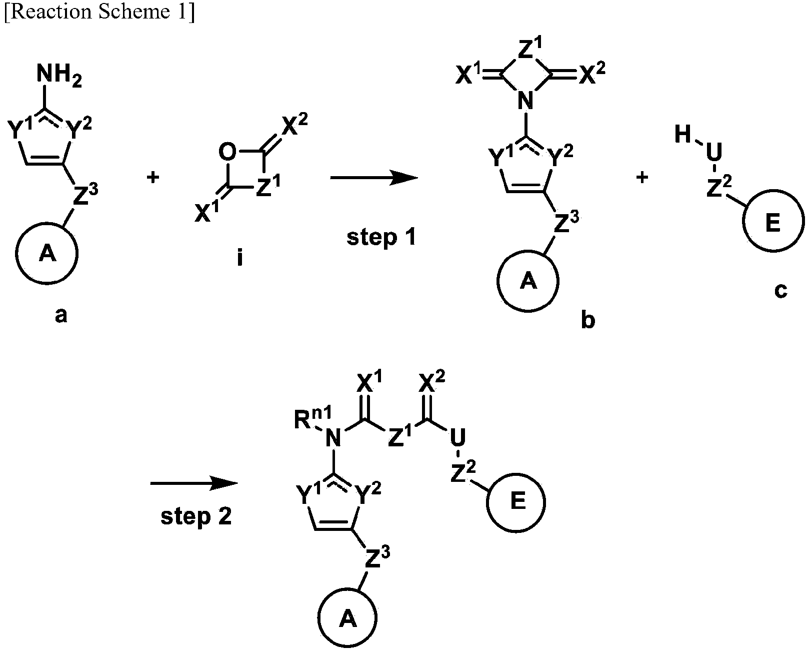

In Reaction Scheme 1, Y¹, Y², Z³, Ring A, X¹, X², Z¹, Z², R¹ⁿ, U, and Ring E are as described in Formula 1 above.

Specifically, in Step 1 of Reaction Scheme 1, Intermediate a, which contains a 5-membered heteroarene ring substituted with amino and optionally connected to Ring A through Z3, may be reacted with cyclic Intermediate 1 to synthesize Intermediate b. The reaction of Step 1 may be carried out in the presence of an appropriate organic solvent capable of dissolving Intermediate a or Intermediate i, or it may be carried out in the molten state, without using an organic solvent, at a temperature equal to or higher than the melting point of Intermediate i (120°C). For example, the reaction of Step 1 may be performed at about 120°C to 180°C for 1 to 5 hours.

In Step 2 of Reaction Scheme 1, Intermediate b may be reacted with amine Intermediate c containing Ring E to prepare the compound represented by Formula 1. Step 2 may be carried out under anhydrous conditions, and it may be performed using an organic solvent, such as dioxane, at a temperature condition of about 80°C to 140°C for 5 to 24 hours.

Intermediate a may be synthesized according to Reaction Scheme 2 below.

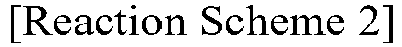

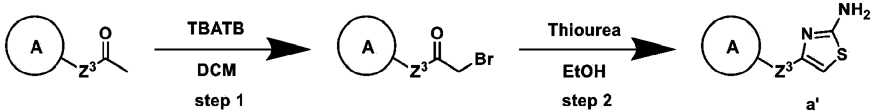

In Reaction Scheme 2, Ring A and Z³ are as described in Formula 1 above.

Specifically, in Step 1 of Reaction Scheme 2, an acetyl compound containing Ring A may be reacted with an excess of tetrabutylammonium tribromide (CAS: 38932-80-8) in an organic solvent to synthesize a bromoacetyl compound. The organic solvent in this reaction may be, but is not limited to, CH₂Cl₂, and the reaction temperature may be about 10°C to 50°C, for example, room temperature.

In Step 2 of Reaction Scheme 2, the product of Step 1 and thiourea may be heated in ethanol to prepare Intermediate a'. Intermediate a' may serve as Intermediate a in Reaction Scheme 1 above.

As used herein, the term "halogen" or "halogen atom" refers to an atom belonging to Group 17 of the periodic table. Halogen atoms include F, Cl, Br, I, and the like. The term "halo" refers to a halogen substituent.

The term "alkyl" refers to a fully saturated branched or unbranched (or straight-chain or linear) hydrocarbon. The alkyl may be substituted or unsubstituted. The C₁₋₂₀ alkyl may be, for example, C₁₋₁₅, C_{1- 10}, or C₁₋₆ alkyl. The C₁₋₆ alkyl may be C₁₋₅, C₁₋₄, C₁₋₃, or C₁₋₂ alkyl. Examples of the alkyl include methyl, ethyl, n-propyl, isopropyl, n-butyl, isobutyl, sec-butyl, n-pentyl, isopentyl, neopentyl, iso-amyl, and n-hexyl.

The term "haloalkyl" refers to an alkyl substituted with one or more halo or halogen, and it includes dihaloalkyl, trihaloalkyl, and the like. "Haloalkyl" may include perhaloalkyl in which all hydrogens of an alkyl are substituted by halogens.

The term "hydroxy" refers to the -OH functional group (hydroxyl group).

The term "hydroxyalkyl" refers to an alkyl substituted with a hydroxy group.

The term "carbonyl" refers to -C(=O)-.

The term "alkoxy" refers to an alkyl bonded to an oxygen atom. The C₁₋₂₀ alkoxy may be, for example, C₁₋₁₅, C₁₋₁₀, or C₁₋₆. The C₁₋₆ alkoxy may be C₁₋₅, C₁₄, C₁₋₃, or C₁₋₂ alkoxy. Examples of the alkoxy include methoxy, ethoxy, propoxy, and butoxy.

The term "alkoxyalkyl" refers to an alkoxy bonded to an alkyl. The C₂₋₂₀ alkoxyalkyl may be, for example, C₂₋₁₅, C₂₋₁₀, or C₂₋₆ alkoxyalkyl. For example, the C₂₋₂₀ alkoxyalkyl may be (C₁₋₁₀ alkoxy)-(C₁₋₁₀ alkyl) or (C₁₋₆ alkoxy)-(C₁₋₆ alkyl). The carbon numbers of the alkoxy and alkyl groups may be the same or different. Examples of the alkoxy include methoxymethyl, methoxyethyl, ethoxymethyl, ethoxyethyl, methoxypropyl, and ethoxypropyl.

The term "amino" refers to -NH₂.

The term "amine group" refers to a substituent in which one, two, or all three hydrogens of ammonia are substituted with an organic functional group. It includes primary amines, secondary amines, and tertiary amines, and it is a term that covers the amino group.

The term "alkylamine" refers to an amine in which one H of amino (-NH₂) is substituted with an alkyl.

The term "di(alkyl)amine" refers to an amine in which both Hs of amino (-NH₂) are substituted with an alkyl. The two alkyls in di(alkyl)amine may be the same or different.

The term "nitro" refers to -NO₂.

The term "cyano" refers to -CN, a functional group consisting of a triple bond between a carbon atom and a nitrogen atom.

The term "carboxy" refers to -COOH. A salt of carboxy refers to the conjugate base of a carboxylic acid.

The term "alkoxycarbonyl" refers to a monovalent substituent in which the -OH of carboxy is substituted with an alkoxy. For example, C₁₋₆ alkoxycarbonyl refers to -C(=O)- substituted with C₁₋₆ alkyl.

The term "carbamoyl" refers to -CONH₂.

The term "alkylcarbamoyl" refers to a substituent in which one hydrogen atom of -NH₂ in carbamoyl is substituted with an alkyl.

The term "dialkylcarbamoyl" refers to a substituent in which two hydrogen atoms of -NH₂ in carbamoyl are each substituted with an alkyl. In dialkylcarbamoyl, the two alkyls may be the same or different.

The term "sulfonyl" refers to a -SO₂- group.

The term "halosulfonyl" refers to a sulfonyl substituted with halogen, and it is a monovalent substituent.

The term "carbocyclyl" refers to a monovalent non-aromatic hydrocarbon ring substituent. The carbocyclyl may be fully saturated or partially unsaturated, and it may have a monocyclic structure, or a fused, bridged, or spiro bicyclic or tricyclic structure. For example, the carbocyclyl includes phenyl fused with a saturated heterohexane ring, such as tetrahydronaphthyl. The carbocyclyl may be a substituent in which 9 to 14 carbons constitute the ring.

The term "cycloalkyl" refers to a saturated non-aromatic monocyclic, bicyclic, or tricyclic hydrocarbon group. The cycloalkyl may contain 3 to 20, for example 5 to 10, 3 to 8, or 3 to 6, carbon atoms. Examples of the monocyclic cycloalkyl include cyclopropyl, cyclobutyl, cyclopentyl, and cyclohexyl. Examples of the bicyclic cycloalkyl include bornyl, decahydronaphthyl, bicyclo[2.1.1]hexyl, bicyclo[2.2.1]heptyl, and bicyclo[2.2.2]octyl. Examples of the tricyclic cycloalkyl include adamantyl.

The term "cycloalkane ring" refers to a saturated non-aromatic monocyclic, bicyclic, or tricyclic hydrocarbon ring. The cycloalkane ring may be the complete (non-radical) form of the cycloalkyl. It may contain 3 to 20, for example 5 to 10, 3 to 8, or 3 to 6, carbon atoms. Examples of the monocyclic cycloalkane ring include a cyclopropane ring, a cyclobutane ring, a cyclopentane ring, and a cyclohexane ring.

The term "aryl" refers to an aromatic hydrocarbon ring group. Aryl includes hydrocarbon ring groups in which a plurality of aryls are connected to provide aromaticity. The C₆₋₃₀ aryl may be, for example, C₆₋₁₄, C₆₋₁₂ or C₆₋₁₀ aryl. The aryl may have a monocyclic structure, or a fused, bridged, or spiro bicyclic or tricyclic structure. The aryl may be phenyl, naphthyl, or biphenyl.

The term "arylalkyl" refers to an alkyl substituted with an aryl.

The term "aryloxy" refers to an aryl bonded to an oxygen atom.

The term "heteroaryl" or "heteroarene" refers to a monocyclic or bicyclic aromatic compound or substituent that contains one or more heteroatoms, with the remaining ring atoms being carbon. The heteroaryl, even if the ring itself is partially unsaturated, may include a ring group having aromaticity as a whole through substitution of the unsaturated ring atoms with oxo (=O), thioxo (=S), or the like. The heteroaryl may include, for example, 1 to 5, 1 to 3, or 1 or 2 heteroatoms, and it may include 5 to 12 ring members. Examples of the "heteroaryl" include pyrrolyl, furanyl, thiophenyl, pyrazolyl, imidazolyl, isoxazolyl, oxazolyl, thiazolyl, isothiazolyl, pyridinyl, pyrimidinyl, pyrazinyl, pyridazinyl, indolyl, benzofuranyl, benzothiophenyl, benzopyrazolyl, benzimidazolyl, benzoxazolyl, benzoisoxazolyl, benzothiazolyl, benzoisothiazolyl, oxopyranyl (pyranonyl), benzopyronyl, and coumarinyl.

The term "heterocycloalkyl" or "heterocyclyl" refers to a saturated or partially unsaturated cyclic hydrocarbon containing at least one heteroatom. The heterocyclyl ring group may be a single ring group, two ring groups, or three ring groups. The two-ring group may be a spiro-ring group, a bridged-ring group, or a fused-ring group. The heterocyclyl ring group may contain 3 to 20, 3 to 10, 3 to 8, 3 to 7, 5 to 7, 4 to 6, or 5 to 6 ring atoms. The heteroatom may be one or more selected from the group consisting of N, O, and S, for example, 1, 2, or 3 heteroatoms. Examples of the heterocyclyl include pyranyl, aziridinyl, oxiranyl, oxetanyl, azetidinyl, pyrrolidinyl, tetrahydrofuranyl, tetrahydrothiophenyl, piperidinyl, tetrahydropyranyl, dihydropyranyl, morpholinyl, thiomorpholinyl, piperazinyl, and oxazolidinyl.

The term "fused benzoheterocyclyl" refers to a substituent in which a benzene ring is fused to a heterocyclyl. The fused benzoheterocyclyl may be a fused heterobicyclic ring group in which two adjacent carbon atoms of the heterocyclyl ring are shared with the benzene ring. The fused benzoheterocyclyl may be a 9- to 11-membered fused heterobicyclic ring group in which a benzene ring is fused to a 5- to 7-membered heterocyclyl containing 1 to 3 heteroatoms selected from O, N, or S. For example, the fused benzoheterocyclyl may include benzopyranyl in which a benzene ring is fused to pyranyl.

The term "fused heterocycloaryl" refers to a substituent in which a heterocyclic ring is fused to an aryl. The fused heterocycloaryl may be a fused heterobicyclic ring group in which two adjacent carbon atoms of the heterocyclic ring are shared with the aryl. The fused heterocycloaryl may be a 9- to 7-membered fused heterobicyclic ring group in which a 5- to 7-membered heterocyclic ring containing 1 to 3 heteroatoms selected from O, N, or S is fused to aryl. For example, the fused heterocycloaryl may include benzodioxolyl in which a dioxolane ring is fused to phenyl.

The heteroatom may be one or more selected from the group consisting of N, O, P, and S. The heteroatom may be 1, 2, or 3 heteroatoms selected from the group consisting of N, O, and S.

In the term "substituted or unsubstituted," "substitution" refers to introduction of an atomic group in place of a hydrogen atom when one or more hydrogen atoms of an organic compound are substituted with another atomic group to form a derivative. A "substituent" refers to the atomic group that has been introduced. As used herein, "substitution" without qualification of the substituent may mean substitution with, for example, a halogen atom, a C₁-C₂₀ alkyl substituted with a halogen atom (e.g., CCF₃, CHCF₂, CH₂F, CCl₃, etc.), a C₁-C₂₀ alkoxy, a C₂-C₂₀ alkoxyalkyl, a hydroxy group, -NH₂, =NH, nitro, cyano, amidino, hydrazine, hydrazone, carboxy or a salt thereof, sulfonyl, sulfamoyl, sulfonic acid or a salt thereof, phosphoric acid or a salt thereof, C₁-C₂₀ alkyl, C₂-C₂₀ alkenyl, C₂-C₂₀ alkynyl, C₆-C₂₀ aryl, C₆-C₂₀ arylalkyl, C₆-C₂₀ heteroaryl, C₇-C₂₀ heteroarylalkyl, C₆-C₂₀ heteroaryloxy, C₆-C₂₀ heteroaryloxyalkyl, or C₆-C₂₀ heteroarylalkyl.

The term "stereoisomer" or "isomer" refers to compounds having the same molecular formula but different connectivity or spatial arrangement of the constituent atoms in the molecule. Isomers include, for example, structural isomers and stereoisomers. The stereoisomers may be diastereomers or enantiomers. Enantiomers refer to isomers whose mirror images cannot be superimposed, like the relationship between the left and right hands, and they are also called optical isomers. Enantiomers are designated as R (Rectus: clockwise) and S (Sinister: counterclockwise) when four or more substituents on a chiral central carbon are different from one another. Diastereomers are stereoisomers that are not in a mirror-image relationship, and they arise from differences in the spatial arrangement of atoms. Diastereomers may be classified as cis-trans isomers and conformational isomers (conformers).

The term "solvate" refers to a compound solvated with an organic or inorganic solvent. The solvate is, for example, a hydrate.

The term "salt" refers to inorganic and organic acid addition salts of a compound. The pharmaceutically acceptable salt may be a salt that does not cause severe irritation in an organism to which the compound is administered and that does not impair the biological activities and physical properties of the compound. The inorganic acid salt may be a hydrochloride, hydrobromide, phosphate, sulfate, or bisulfate. The organic acid salt may be a formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, embonate, glutamate, ethanesulfonate, benzenesulfonate, p-toluenesulfonate, or aspartate. The metal salt may be a calcium salt, sodium salt, magnesium salt, strontium salt, or potassium salt.

The compound of Formula 1 may be an agonist of SOX9 (SRY-Box Transcription Factor 9). SOX9, together with other members of the HMG-box family of DNA-binding proteins, is known to recognize the CCTTGAG sequence and to be expressed by proliferation, not by hypertrophic chondrocytes, which is essential for the differentiation of progenitor cells into chondrocytes. The compound of Formula 1 may increase the expression of SOX9 by inducing condensation of the SOX9 transcription factor. The compound of Formula 1 may be an SOX9 transcription-factor condensation inducer, an SOX9 activator, or an activating agent.

Another aspect of the present invention provides a pharmaceutical composition comprising the compound described above, or a stereoisomer, a solvate, or a pharmaceutically acceptable salt thereof.

Another aspect of the present invention provides a pharmaceutical composition for preventing or treating diseases associated with reduced expression of SOX9 (SRY-Box Transcription Factor 9), comprising the compound described above, or a stereoisomer, a solvate, or a pharmaceutically acceptable salt thereof.

The compound, stereoisomer, solvate, pharmaceutically acceptable salt, and SOX9 are as described above.

The disease associated with reduced expression of SOX9 is attributable to inhibition of SOX9.

In some embodiments, the disease associated with reduced expression of SOX9 may be osteoarthritis and osteoarthritis-related diseases.

In some embodiments, the osteoarthritis-related disease may be chondropathia, osteonecrosis, or chronic pain.

The term "prevention" refers to any action of inhibiting the onset or delaying the development of an SOX9-related disease through administration of the pharmaceutical composition. The term "treatment" refers to any action of improving or beneficially altering the symptoms of an SOX9-related disease through administration of the pharmaceutical composition.

The pharmaceutical composition may comprise a pharmaceutically acceptable carrier. The carrier is used to include excipients, diluents, or adjuvants. The carrier may be selected from the group consisting of, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinyl pyrrolidone, water, physiological saline, buffers such as PBS, methylhydroxy benzoate, propylhydroxy benzoate, talc, magnesium stearate, and mineral oil. The composition may comprise a filler, an anti-aggregating agent, a lubricant, a wetting agent, a flavoring agent, an emulsifier, a preservative, or combinations thereof.

The pharmaceutical composition may be prepared in any formulation according to conventional methods. The composition may be formulated, for example, into oral dosage forms (such as powders, tablets, capsules, syrups, pills, or granules) or parenteral dosage forms (such as injections). In addition, the composition may be prepared as a systemic formulation or as a local formulation.

In the pharmaceutical composition, solid preparations for oral administration may be tablets, pills, powders, granules, or capsules. The solid preparation may further comprise an excipient. Examples of the excipients include starch, calcium carbonate, sucrose, lactose, and gelatin. In addition, the solid preparation may further comprise a lubricant such as magnesium stearate or talc. In the pharmaceutical composition, liquid preparations for oral administration may be suspensions, oral liquids, emulsions, or syrups. The liquid preparation may comprise water or liquid paraffin. The liquid preparation may comprise excipients, such as wetting agents, sweeteners, fragrances, or preservatives. In the pharmaceutical composition, preparations for parenteral administration may be sterilized aqueous solutions, non-aqueous solutions, suspensions, emulsions, lyophilizates, or suppositories. The non-aqueous solution or suspension may comprise vegetable oils or esters. Examples of the vegetable oil include propylene glycol, polyethylene glycol, and olive oil. An example of the ester is ethyl oleate. The base for suppositories may be witepsol, macrogol, tween 61, cocoa butter, lauric butter, or glycerogelatin.

The pharmaceutical composition comprises the compound according to one aspect, or a stereoisomer, a solvate, or a pharmaceutically acceptable salt thereof, as an active ingredient of the pharmaceutical composition. The "active ingredient" refers to a physiologically active substance used to achieve a pharmacological activity (e.g., treatment of diseases associated with reduced expression of SOX9).

The pharmaceutical composition may comprise the compound according to one aspect, or a stereoisomer, a solvate, or a pharmaceutically acceptable salt thereof in an effective amount. The term "effective amount" refers to an amount sufficient to exhibit a preventive or therapeutic effect on a disease when administered to an individual in need of prevention or treatment. The effective amount may be appropriately selected by a person of ordinary skill in the art depending on the cell or individual selected. The preferred dosage of the pharmaceutical composition will vary depending on the condition and weight of the individual, the severity of the disease, the form of the drug, and the route and duration of administration, but it may be appropriately selected by a person of ordinary skill in the art. The effective amount may be from about 0.5 µg to about 2 g, from about 1 µg to about 1 g, from about 10 µg to about 500 mg, from about 100 µg to about 100 mg, or from about 1 mg to about 50 mg, per the pharmaceutical composition. However, the compound, stereoisomer, solvate, or pharmaceutically acceptable salt thereof may be administered, for example, in an amount of from about 0.0001 mg/kg to about 100 mg/kg, or from about 0.001 mg/kg to about 100 mg/kg, divided into 1 to 24 times a day, 1 to 7 times in 2 days to 1 week, or 1 to 24 times in 1 month to 12 months. In the pharmaceutical composition, the compound, stereoisomer, solvate, or pharmaceutically acceptable salt thereof may be contained in an amount of about 0.0001 wt% to about 10 wt%, or about 0.001 wt% to about 1 wt%, based on the total weight of the entire composition.

The administration method may be oral or parenteral administration. The administration method may be, for example, oral, transdermal, subcutaneous, rectal, intravenous, intra-arterial, intraperitoneal, intramuscular, intrasternal, topical, intranasal, intratracheal, or intradermal route. The composition may be administered systemically or locally, and it may be administered alone or in combination with other pharmaceutically active compounds.

Another aspect of the present invention provides a method of preventing or treating a disease associated with reduced expression of SOX9, comprising administering to an individual the compound described above, or a stereoisomer, a solvate, or a pharmaceutically acceptable salt thereof.

The compound, stereoisomer, solvate, pharmaceutically acceptable salt, SOX9, the disease associated with reduced expression of SOX9, prevention, and treatment are as described above.

The individual may be a mammal, for example, a human, mouse, rat, cow, horse, pig, dog, monkey, sheep, goat, ape, or cat. The individual may be one who suffers from, or is at high risk of suffering from, symptoms associated with diseases associated with reduced expression of SOX9.

The method may further comprise administering to the individual a known active ingredient that is effective in preventing or treating SOX9-related diseases. The known active ingredient may be administered to the individual simultaneously, separately, or sequentially with the compound, stereoisomer, solvate, or pharmaceutically acceptable salt according to one aspect.

The administration method may be oral or parenteral administration. The administration method may be, for example, oral, transdermal, subcutaneous, rectal, intravenous, intra-arterial, intraperitoneal, intramuscular, intrasternal, topical, intranasal, intratracheal, or intradermal route. The pharmaceutical composition may be administered systemically or locally, and it may be administered alone or in combination with other pharmaceutically active compounds.

The preferred dosage of the pharmaceutical composition will vary depending on the condition and weight of the patient, the severity of the disease, the form of the drug, and the route and duration of administration, but it may be appropriately selected by a person of ordinary skill in the art. The dosage may be, for example, in the range of about 0.001 mg/kg to about 100 mg/kg, about 0.01 mg/kg to about 10 mg/kg, or about 0.1 mg/kg to about 1 mg/kg for adults. Administration may be, for example, once a day, 2 to 24 times a day, 1 to 2 times every 3 days, 1 to 6 times a week, 1 to 10 times every 2 weeks, 1 to 15 times every 3 weeks, 1 to 3 times every 4 weeks, or 1 to 12 times a year.

Another aspect of the present invention provides a compound, or a stereoisomer, a solvate, or a pharmaceutically acceptable salt thereof, for use in the prevention or treatment of a disease associated with reduced expression of SOX9.

The compound, stereoisomer, solvate, pharmaceutically acceptable salt, SOX9, the disease associated with reduced expression of SOX9, prevention, and treatment are as described above.

Another aspect of the present invention provides the use of a compound, or a solvate, a stereoisomer, or a pharmaceutically acceptable salt thereof, for use in the prevention or treatment of a disease associated with reduced expression of SOX9.

The compound, stereoisomer, solvate, pharmaceutically acceptable salt, SOX9, the disease associated with reduced expression of SOX9, prevention, and treatment are as described above.

Another aspect of the present invention provides the use of the compound according to one aspect, or a stereoisomer, a solvate, or a pharmaceutically acceptable salt thereof, for use in the manufacture of a medicament for preventing or treating a disease associated with reduced expression of SOX9.

The compound, stereoisomer, solvate, pharmaceutically acceptable salt, SOX9, the disease associated with reduced expression of SOX9, prevention, and treatment are as described above.

### Effects of the Invention

The compound of the present invention and the pharmaceutical composition for preventing or treating diseases associated with reduced expression of SOX9 comprising the same can effectively prevent or treat diseases associated with reduced expression of SOX9.

### Brief Description of the Drawings

FIG. 1 shows the data for evaluating the SOX9 transcriptional effect of the Example compounds through luciferase assay.
FIG. 2a is a histogram showing the distribution of the SOX9 transcriptional activity effect of the Example compounds.
FIG. 2b is a graph showing, as a box plot, the transcriptional activity effect according to whether SOX9 is condensed by the Example compounds.
FIG. 3a is a fluorescence image obtained by treating SOX9-overexpressing cell lysates with Compound B8 and Compound C9.
FIG. 3b is a graph showing the SOX9 transcriptional activity effect measured by using the luciferase technique.
FIG. 4a is a fluorescence image showing the degree of SOX9 condensate formation according to drug structure.
FIG. 4b is a graph showing the change in the SOX9 transcriptional activity effect according to the drug structure, measured using the luciferase technique.
FIG. 5a is a graph showing the degree of disorder according to the structure and position of SOX9.
FIG. 5b is a graph showing the degree of SOX9 condensation by the drug according to SOX9 deletion variants.
FIG. 5c is a graph showing the degree of SOX9 condensation by the drug according to deletion variants of the disordered region at the C-terminus of SOX9.
FIG. 6 is a graph showing the degree of SOX9 condensation by the drug according to substitution of aromatic amino acids at the C-terminus of SOX9.
FIG. 7a is a schematic flow diagram showing a method for identifying proteins of drug-induced condensates using SOX9-turboID.
FIG. 7b is a volcano plot showing proteins identified in larger amounts upon drug treatment compared to the solvent that dissolves the drug.
FIG. 8a is a histogram showing the degree of disorder of proteins frequently observed upon treatment with the drug compared to the solvent that dissolves the drug.
FIG. 8b is a histogram showing the pattern similarity of proteins frequently observed upon treatment with the drug, with respect to the aromatic amino acids at the C-terminus of SOX9, compared to the solvent that dissolves the drug.
FIG. 8c is a graph showing the relationship between the pattern similarity of proteins frequently observed upon drug treatment, with respect to the aromatic amino acids at the C-terminus of SOX9, and the conditional probability, compared to the solvent that dissolves the drug.
FIG. 9 shows images of SOX9 observed in cell nuclei using a conventional fluorescence microscope and a super-resolution microscope (dSTORM).
FIG. 10 shows the qPCR results measuring the mRNA levels of SOX9 target genes upon drug treatment.
FIG. 11 shows fluorescence images of mRNA of SOX9 target genes (Acan, Col9a1, and Sox9) measured by FISH together with SOX9 protein.
FIG. 12a is a histogram showing the size distribution of SOX9 condensates near the mRNA of SOX9 target genes upon drug treatment.
FIG. 12b is a graph showing the change in size of SOX9 condensates near the mRNA of SOX9 target genes upon drug treatment.
FIG. 13 is a graph showing the degree of SOX9 binding to gene regulatory regions on DNA upon drug treatment, compared to the solvent that dissolves the drug.
FIG. 14 is a graph showing the degree of SOX9 binding to regulatory regions of individual genes on DNA upon drug treatment, compared to the solvent that dissolves the drug.
FIG. 15a is a schematic flow diagram for the experiment of administering the drug to osteoarthritis model mice.
FIG. 15b shows images (red: cartilage; blue: bone) showing the degree of recovery of cartilage tissue in the joint area of osteoarthritis model mice upon drug treatment.
FIG. 15c is a graph showing the degree of tissue recovery upon drug treatment as evaluated using OARSI grade.
FIG. 16 is a schematic flow diagram and a result graph of an experiment evaluating the degree of weight bearing on the leg of osteoarthritis model mice upon drug treatment, in comparison with the non-operated leg.
FIG. 17 is a schematic flow diagram and a result graph of an experiment quantitatively measuring the degree to which the soles of osteoarthritis model mice can withstand a needle stimulus upon drug treatment.

### Embodiments for Carrying out the Invention

Hereinafter, the present invention will be described in more detail through Examples. However, these Examples are intended to be illustrative, and the scope of the present invention is not limited to these Examples.

### Preparation Example A1: Synthesis of 3-(2-aminothiazol-4-yl)-7-methoxycoumarin

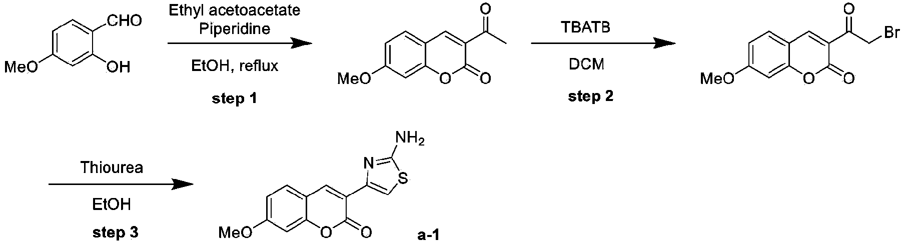

### Step 1: 3-Acetyl-7-methoxycoumarin

2-Hydroxy-4-methoxybenzaldehyde (2 mmol, CAS: 673-22-3), ethyl acetoacetate (2.4 mmol), and piperidine (0.2 mmol) were stirred in ethanol (4 mL). After completion of the reaction, the temperature of the reaction mixture was lowered using an ice-water bath, and the precipitate was filtered to obtain 3-acetyl-7-methoxycoumarin in a yield of 88%.

¹H NMR (400 MHz, DMSO) δ 8.64 (s, 1H), 7.87 (d, J = 8.7 Hz, 1H), 7.06 (d, J = 2.3 Hz, 1H), 7.02 (dd, J = 8.7, 2.4 Hz, 1H), 3.90 (s, 3H), 2.56 (s, 3H).

### Step 2: 3-Bromoacetyl-7-methoxycoumarin

The product of Step 1 (1 mmol) and tetrabutylammonium tribromide (TBATB, 2 mmol) were dissolved in CH₂Cl₂ (10 mL) and stirred at room temperature. After completion of the reaction, the resulting precipitate was separated by filtration to obtain 3-bromoacetyl-7-methoxycoumarin in a yield of 59%.

¹H NMR (400 MHz, DMSO) δ 8.82 (s, 1H), 7.93 (d, J = 8.7 Hz, 1H), 7.12 (d, J = 2.4 Hz, 1H), 7.06 (dd, J = 8.7, 2.5 Hz, 1H), 4.87 (s, 2H), 3.92 (s, 3H).

### Step 3: 3-(2-Aminothiazol-4-yl)-7-methoxycoumarin

The product of Step 2 (1 mmol) and thiourea (2 mmol) were stirred and heated in ethanol (10 mL). After completion of the reaction, the resulting precipitate was filtered to obtain Intermediate a-1 in a yield of 99%.

¹H NMR (400 MHz, DMSO) δ 8.47 (s, 1H), 7.75 (d, J = 8.7 Hz, 1H), 7.42 (s, 1H), 7.07 (d, J = 2.4 Hz, 1H), 7.00 (d, J = 8.6 Hz, 1H), 3.88 (s, 3H).

### Preparation Examples A2 to A13

Intermediates a-2 to a-12 were each obtained in the same manner as in Preparation Example A1, except that the hydroxybenzaldehyde compounds shown in Table 1 below were used instead of 2-hydroxy-4-methoxybenzaldehyde in Step 1 of Preparation Example A1.

**[Table 1]**

| Intermediate | Name / ¹H NMR | Hydroxybenzaldehyde compound |
|---|---|---|
| Intermediate a-2 | 3-(2-aminothiazol-4-yl)-7-hydroxycoumarin (23%, 523 mg) | 2,4-dihydroxybenzaldehyde (CAS: 95-01-2) |
| 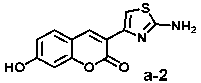 | ¹H NMR (400 MHz, DMSO) δ 10.69 (s, 1H), 8.42 (s, 1H), 7.64 (d, J = 8.5 Hz, 1H), 7.37 (s, 1H), 6.83 (dd, J = 8.5, 2.2 Hz, 1H), 6.77 (s, 1H). | |
| Intermediate a-3 | 3-(2-aminothiazol-4-yl)-7-methylcoumarin (79%, 939 mg) | 2-hydroxy-4-methylbenzaldehyde (CAS: 698-27-1) |
| 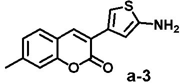 | ¹H NMR (400 MHz, DMSO) δ 8.47 (s, 1H), 7.71 (d, J = 7.9 Hz, 1H), 7.47 (s, 1H), 7.28 (s, 1H), 7.21 (d, J = 7.9 Hz, 1H). | |
| Intermediate a-4 | 3-(2-aminothiazol-4-yl)-7-chlorocoumarin (75%, 1061 mg) | 4-chloro-2-hydroxybenzaldehyde (CAS: 2420-26-0) |
| 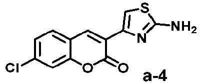 | ¹H NMR (400 MHz, DMSO) δ 8.49 (s, 1H), 7.86 (d, J = 8.3 Hz, 1H), 7.65 (t, J = 1.7 Hz, 1H), 7.52 (s, 1H), 7.50 - 7.37 (m, 1H). | |
| Intermediate a-5 | 3-(2-aminothiazol-4-yl)-7-bromocoumarin (64%, 552 mg) | 4-bromo-2-hydroxybenzaldehyde (CAS: 22532-62-3) |
| 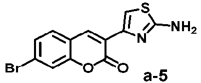 | ¹H NMR (400 MHz, DMSO) δ 8.47 (s, 1H), 7.83 - 7.73 (m, 2H), 7.57 (dd, J = 8.3, 1.9 Hz, 1H), 7.52 (s, 1H). | |
| Intermediate a-6 | 3-(2-aminothiazol-4-yl)-7-N,N-dimethylaminocoumarin (8%, 69 mg) | 4-(dimethylamino)-2-hydroxybenzaldehyde (CAS: 41602-56-6) |
| 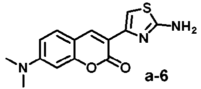 | ¹H NMR (400 MHz, DMSO) δ 8.36 (s, 1H), 7.56 (d, J = 8.9 Hz, 1H), 7.28 (s, 1H), 7.05 (s, 2H), 6.76 (dd, J = 8.9, 2.5 Hz, 1H), 6.60 (d, J = 2.4 Hz, 1H), 3.04 (s, 6H). | |
| Intermediate a-7 | 3-(2-aminothiazol-4-yl)-coumarin (61%, 232 mg) | 2-hydroxybenzaldehyde (CAS: 90-02-8) |
| 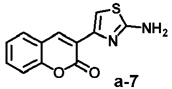 | ¹H NMR (400 MHz, DMSO) δ 8.51 (s, 1H), 7.83 (d, J = 7.9 Hz, 1H), 7.62 (dd, J = 11.0, 4.4 Hz, 1H), 7.53 (d, J = 2.5 Hz, 1H), 7.48 - 7.25 (m, 2H). | |
| Intermediate a-8 | 3-(2-aminothiazol-4-yl)-8-methoxycoumarin (84%, 664 mg) | 2-hydroxy-3-methoxybenzaldehyde (CAS: 148-53-8) |
| 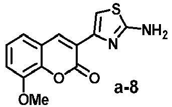 | ¹H NMR (400 MHz, DMSO) δ 8.48 (d, J = 1.6 Hz, 1H), 7.53 (d, J = 3.8 Hz, 1H), 7.41 - 7.21 (m, 3H), 3.93 (d, J = 0.9 Hz, 3H). | |
| Intermediate a-9 | 3-(2-aminothiazol-4-yl)-6-methoxycoumarin (10%, 49 mg) | 2-hydroxy-5-methoxybenzaldehyde (CAS: 672-13-9) |
| 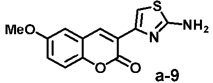 | ¹H NMR (400 MHz, DMSO) δ 8.49 (s, 1H), 7.53 (s, 1H), 7.45 - 7.37 (m, 2H), 7.22 - 7.16 (m, 1H),3.83 (s, 3H). | |
| Intermediate a-10 | 3-(2-aminothiazol-4-yl)-5-methoxycoumarin (8%, 58 mg) | 2-hydroxy-6-methoxybenzaldehyde (CAS: 700-44-7) |
| 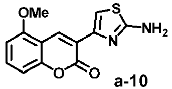 | ¹H NMR (400 MHz, DMSO) δ 8.72 (s, 1H), 7.56 (t, J = 8.4 Hz, 1H), 7.47 (s, 1H), 7.01 (d, J = 8.4 Hz, 2H), 3.96 (s, 3H). | |
| Intermediate a-11 | 3-(2-aminothiazol-4-yl)-8-hydroxycoumarin (72%, 1859 mg) | 2,3-dihydroxybenzaldehyde (CAS: 24677-78-9) |
| 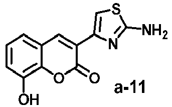 | ¹H NMR (400 MHz, DMSO) δ 10.30 (s, 1H), 8.45 (s, 1H), 7.51 (s, 1H), 7.28 - 6.88 (m, 3H). | |
| Intermediate a-12 | 3-(2-aminothiazol-4-yl)-6-hydroxycoumarin (67%, 571 mg) | 2,5-dihydroxybenzaldehyde (CAS: 1194-98-5) |
| 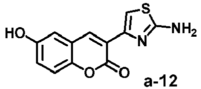 | ¹H NMR (400 MHz, DMSO) δ 9.80 (s, 1H), 8.41 (s, 1H), 7.52 (s, 1H), 7.30 (d, J = 8.8 Hz, 1H), 7.14 - 6.88 (m, 2H). | |

### Preparation Example A13: Synthesis of 3-(2-aminooxazol-4-yl)-7-methoxycoumarin

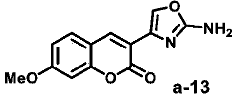

Intermediate a-13 (52%, 387 mg) was obtained in the same manner as in Preparation Example A1, except that urea (CAS: 57-13-6) was used instead of thiourea in Step 3 of Preparation Example A1.

¹H NMR (300 MHz, DMSO) δ 8.24 (s, 1H), 7.84 (s, 1H), 7.77 (d, J = 8.7 Hz, 1H), 7.05 (d, J = 2.6 Hz, 1H), 6.98 (dd, J = 8.7, 2.4 Hz, 1H), 6.83 (s, 2H), 3.87 (s, 3H).

The compounds shown in Table 2 below were used as Intermediates a-14 to a-17.

**[Table 2]**

| | |
|---|---|
| Intermediate a-14 | 1-(2-aminothiazol-4-yl)-2,4-difluorobenzene |
| 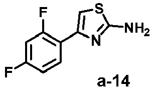 | (CAS: 175135-32-7, Aldrich) |
| Intermediate a-15 | 1-(2-aminothiazol-4-yl)-3,4-difluorobenzene |
| 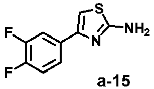 | (CAS: 175135-32-7, Aldrich) |
| Intermediate a-16 | 1-(2-aminothiazol-4-yl)-3,4-difluorobenzene |
| 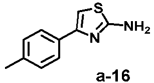 | (CAS: 2103-91-5, Aldrich) |
| Intermediate a-17 | 2-(2-aminothiazol-4-yl)-5,6,7,8-tetrahydronaphthalene |
| 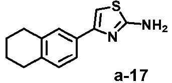 | (CAS: 87999-04-0, Aldrich) |

### Preparation Example A18: Synthesis of 4-(2-aminothiazol-4-yl)biphenyl

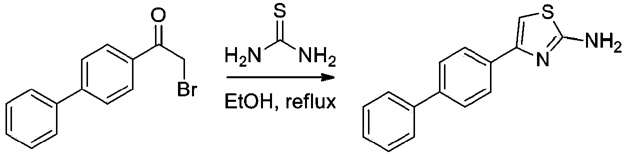

Intermediate a-18 (94%, 476 mg) was obtained in the same manner as in Step 3 of Preparation Example A1, except that 2-bromo-4'-phenylacetophenone (CAS: 135-73-9) was used instead of the product of Step 2 of Preparation Example A1.

¹H NMR (400 MHz, DMSO) δ 8.85 (s, 2H), 7.88 - 7.78 (m, 4H), 7.78 - 7.69 (m, 2H), 7.50 (dd, J = 8.5, 6.9 Hz, 2H), 7.44 - 7.36 (m, 1H), 7.32 (s, 1H).

### Preparation Example A19: Synthesis of 3-(2-aminothiazol-4-yl)-6-methyl-2H-pyran-2,3(3H)-dione

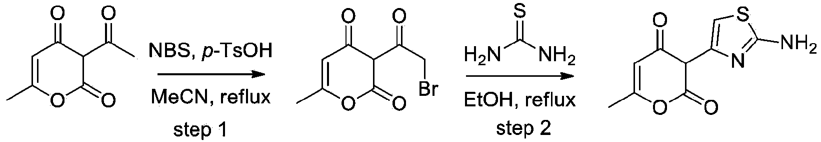

### Step 1: 3-(2-Bromoacetyl)-6-methyl-2H-pyran-2,4(3H)-dione

Dehydroacetic acid (5.0 mmol, CAS: 520-45-6) and p-TsOH (5.5 mmol, CAS: 6192-52-5) were stirred in acetonitrile (10 mL). Thereafter, NBS (5.5 mmol, CAS: 128-08-5) was gradually added thereto, and the mixture was heated. After 9 hours, water was added to the reaction mixture, the product was extracted with DCM, which was then concentrated to obtain the product (see Synthetic Communications, 2012, 42(18), 2739-2747).

### Step 2: 3-(2-Aminothiazol-4-yl)-6-methyl-2H-pyran-2,3(3H)-dione

The product of Step 1 was used to obtain Intermediate a-19 (8%, 94 mg) in the same manner as in Step 3 of Preparation Example A1.

¹H NMR (400 MHz, DMSO) δ 15.12 (s, 1H), 8.02 (s, 2H), 7.10 (s, 1H), 6.12 (d, J = 1.0 Hz, 1H), 2.20 (d, J = 0.9 Hz, 3H).

### Preparation Example A20: Synthesis of (2-aminothiazol-5-yl)(phenyl)methanone

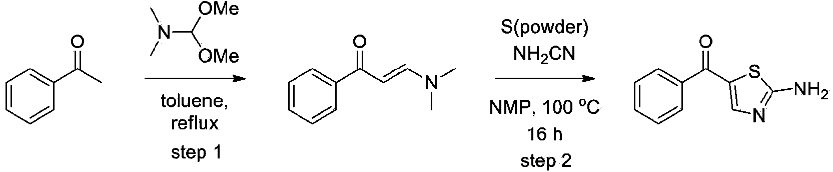

### Step 1: (E)-3-(Dimethylamino)-1-phenylprop-2-en-1-one

Acetophenone (2.0 mmol) and N,N-dimethylformamide dimethyl acetal (4.0 mmol) were stirred and heated in toluene (2 mL) for 18 hours. The solvent was removed, and (E)-3-(dimethylamino)-1-phenylprop-2-en-1-one (124 mg, 35%) was isolated by column chromatography (DCM/MeOH = 5:1).

### Step 2: (2-Aminothiazol-5-yl)(phenyl)methanone

The product of Step 1 (0.4 mmol), S powder (1.7 mmol), cyanamide solution (1.7 mmol), and N-methylmorpholine (NMM, 0.1 mmol) were stirred in N-methylpyrrolidone (NMP, 1 mL). Thereafter, they were reacted at 100°C for 16 hours under an argon atmosphere. Thereafter, the reaction mixture was cooled and diluted with EA. Then, water was added thereto for extraction. The organic layer containing the product was dried over Na₂SO₄ to remove residual moisture, then concentrated, and purified by column chromatography (Hex/EA = 1:1) to obtain Intermediate a-20 (31%, 78 mg) (see J. Org. Chem. 2019, 84(18), 12237-12245).

¹H NMR (600 MHz, CDCl3) δ 7.78 (dd, J = 8.3, 1.4 Hz, 2H), 7.62 (s, 1H), 7.60 - 7.56 (m, 1H), 7.53 - 7.46 (m, 2H), 6.22 (br s, 2H).

### Preparation Example B1: Synthesis of 3-(2-succinimidylthiazol-4-yl)-7-methoxycoumarin

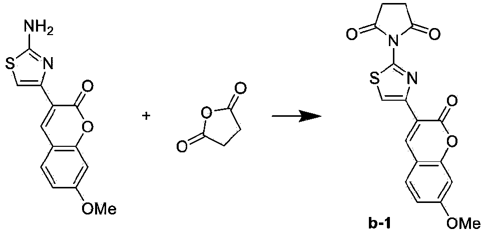

Succinic anhydride (10 mmol, 1.00 g) and Intermediate a-1 (1 mmol) were placed in a mortar, mixed thoroughly, and transferred to a reactor. The reaction mixture was stirred at 150°C for 5 hours. After completion of the reaction, the obtained product was purified by silica gel chromatography (CH₂Cl₂: CH₃C(O)CH₃ = 20:1) to obtain Intermediate b-1 (yield: 84%, 300 mg).

¹H NMR (400 MHz, DMSO) δ 8.65 (s, 1H), 8.39 (s, 1H), 7.84 (d, J = 8.7 Hz, 1H), 7.09 (d, J = 2.4 Hz, 1H), 7.00 (dd, J = 8.7, 2.4 Hz, 1H), 3.88 (s, 3H), 2.87 (s, 4H).

### Preparation Examples B2 to B21

Intermediates b-2 to b-20 were each synthesized by changing Intermediate a-1 of Preparation Example B1 to Intermediates a-2 to a-20.

**[Table 3]**

| | |
|---|---|
| Intermediate b-2 | 3-(2-succinimidylthiazol-4-yl)-7-hydroxycoumarin (46%, 316 mg) |
| 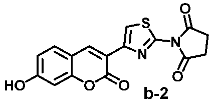 | ¹H NMR (600 MHz, DMSO) δ 10.75 (s, 1H), 8.60 (s, 1H), 8.35 (s, 1H), 7.74 (d, J = 8.6 Hz, 1H), 6.84 (dd, J = 8.5, 2.3 Hz, 1H), 6.79 (d, J = 2.2 Hz, 1H), 2.87 (s, 4H). |
| Intermediate b-3 | 3-(2-succinimidylthiazol-4-yl)-7-methylcoumarin (88%, 60 mg) |
| 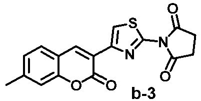 | ¹H NMR (400 MHz, DMSO) δ 8.65 (s, 1H), 8.44 (s, 1H), 7.79 (d, J = 8.0 Hz, 1H), 7.30 (s, 1H), 7.23 (dd, J = 8.2, 1.6 Hz, 1H), 2.88 (s, 4H), 2.44 (s, 3H). |
| Intermediate b-4 | 3-(2-succinimidylthiazol-4-yl)-7-chlorocoumarin (73%, 52 mg) |
| 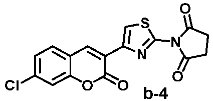 | ¹H NMR (400 MHz, DMSO) δ 8.69 (s, 1H), 8.48 (s, 1H), 7.97 (d, J = 8.4 Hz, 1H), 7.67 (d, J = 2.0 Hz, 1H), 7.47 (dd, J = 8.4, 2.0 Hz, 1H), 2.88 (s, 4H). |
| Intermediate b-5 | 3-(2-succinimidylthiazol-4-yl)-7-bromocoumarin (44%, 36 mg) |
| 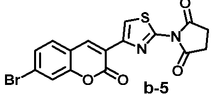 | ¹H NMR (400 MHz, DMSO) δ 8.65 (s, 1H), 8.48 (s, 1H), 7.90 - 7.87 (s, 1H), 7.79 (br s, 1H), 7.64 - 7.57 (m, 1H), 2.90 (s, 3H). |
| Intermediate b-6 | 3-(2-succinimidylthiazol-4-yl)-7-N,N-dimethylaminocoumarin (78%, 69 mg) |
| 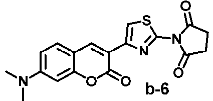 | ¹H NMR (400 MHz, DMSO) δ 8.54 (s, 1H), 8.27 (s, 1H), 7.66 (d, J = 8.9 Hz, 1H), 6.79 (dd, J = 8.9, 2.5 Hz, 1H), 6.63 (d, J = 2.4 Hz, 1H), 3.06 (s, 6H), 2.87 (s, 4H). |
| Intermediate b-7 | 3-(2-succinimidylthiazol-4-yl)-coumarin (99%, 65 mg) |
| 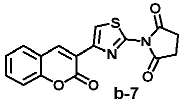 | ¹H NMR (400 MHz, DMSO) δ 8.68 (s, 1H), 8.48 (s, 1H), 7.91 (dd, J = 7.8, 1.6 Hz, 1H), 7.70 - 7.63 (m, 1H), 7.50 - 7.35 (m, 2H), 2.89 (s, 4H). |
| Intermediate b-8 | 3-(2-succinimidylthiazol-4-yl)-8-methoxycoumarin (99%, 71 mg) |
| 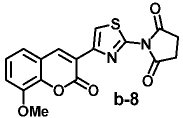 | ¹H NMR (400 MHz, DMSO) δ 8.62 (s, 1H), 8.47 (s, 1H), 7.41 (br s, 1H), 7.29 (br s, 1H), 7.23 - 7.04 (m, 1H), 3.93 (s, 3H), 2.89 (s, 4H). |
| Intermediate b-9 | 3-(2-succinimidylthiazol-4-yl)-6-methoxycoumarin (58%, 41 mg) |
| 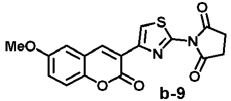 | ¹H NMR (400 MHz, DMSO) δ 8.69 (s, 1H), 8.49 (s, 1H), 7.52 (s, 1H), 7.39 - 7.32 (m, 1H), 7.19 (dd, J = 9.1, 3.0 Hz, 1H), 3.82 (s, 3H), 2.89 (s, 4H). |
| Intermediate b-10 | 3-(2-succinimidylthiazol-4-yl)-5-methoxycoumarin (62%, 20 mg) |
| 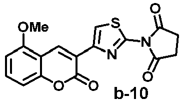 | ¹H NMR (400 MHz, DMSO) δ 8.84 (s, 1H), 8.44 (s, 1H), 7.61 (t, J = 8.4 Hz, 1H), 7.14 - 6.96 (m, 2H), 3.98 (s, 3H), 2.87 (s, 4H). |
| Intermediate b-11 | 3-(2-succinimidylthiazol-4-yl)-8-hydroxycoumarin (22%, 150 mg) |
| 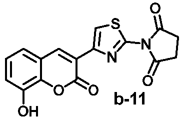 | ¹H NMR (600 MHz, DMSO) δ 10.30 (s, 1H), 8.62 (s, 1H), 8.47 (s, 1H), 7.31 (dd, J = 7.8, 1.5 Hz, 1H), 7.18 (t, J = 7.8 Hz, 1H), 7.13 (dd, J = 8.0, 1.5 Hz, 1H), 2.87 (s, 4H). |
| Intermediate b-12 | 3-(2-succinimidylthiazol-4-yl)-6-hydroxycoumarin (23%, 159 mg) |
| 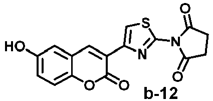 | ¹H NMR (600 MHz, DMSO) δ 9.80 (s, 1H), 8.57 (s, 1H), 8.46 (s, 1H), 7.31 (d, J = 9.0 Hz, 1H), 7.18 (d, J = 2.8 Hz, 1H), 7.08 (dd, J = 8.8, 2.8 Hz, 1H), 2.88 (s, 4H). |
| Intermediate b-13 | 3-(2-succinimidyloxazol-4-yl)-7-methoxycoumarin (69%, 236 mg) |
| 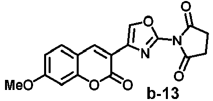 | ¹H NMR (400 MHz, DMSO) δ 8.68 (s, 1H), 8.49 (s, 1H), 7.83 (d, J = 8.7 Hz, 1H), 7.08 (d, J = 2.7 Hz, 1H), 7.01 (dd, J = 8.9, 2.4 Hz, 1H), 3.88 (s, 3H), 2.93 (s, 4H). |
| Intermediate b-14 | 1-(2-succinimidylthiazol-4-yl)-2,4-difluorobenzene (30%, 176 mg) |
| 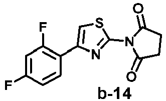 | ¹H NMR (400 MHz, DMSO) δ 8.09 (td, J = 8.9, 6.7 Hz, 1H), 8.00 (d, J = 2.6 Hz, 1H), 7.41 (ddd, J = 11.8, 9.2, 2.6 Hz, 1H), 7.28 - 7.20 (m, 1H), 2.87 (s, 4H). |
| Intermediate b-15 | 1-(2-succinimidylthiazol-4-yl)-3,4-difluorobenzene (31%, 182 mg) |
| 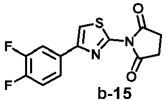 | ¹H NMR (400 MHz, DMSO) δ 8.23 (s, 1H), 8.03 - 7.91 (m, 1H), 7.85 - 7.78 (m, 1H), 7.61 - 7.45 (m, 1H), 2.87 (s, 4H). |
| Intermediate b-16 | 1-(2-succinimidylthiazol-4-yl)-4-methylbenzene (26%, 140 mg) |
| 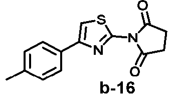 | ¹H NMR (400 MHz, DMSO) δ 8.09 (s, 1H), 7.84 (d, J = 8.2 Hz, 2H), 7.26 (d, J = 8.0 Hz, 2H), 2.86 (s, 4H), 2.33 (s, 3H). |
| Intermediate b-17 | 2-(2-succinimidylthiazol-4-yl)-5,6,7,8-tetrahydronaphthalene (27%, 170 mg) |
| 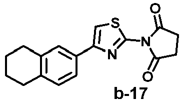 | ¹H NMR (400 MHz, DMSO) δ 8.07 (s, 1H), 7.63 (dd, J = 4.2, 2.3 Hz, 3H), 7.12 (d, J = 8.5 Hz, 1H), 2.85 (s, 4H), 2.82 - 2.69 (m, 4H), 1.75 (p, J = 3.2 Hz, 4H). |
| Intermediate b-18 | 4-(2-succinimidylthiazol-4-yl)biphenyl (58%, 193 mg) |
| 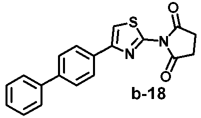 | ¹H NMR (400 MHz, DMSO) δ 8.25 (s, 1H), 8.05 (d, J = 8.4 Hz, 2H), 7.83 - 7.71 (m, 3H), 7.50 (t, J = 7.6 Hz, 2H), 7.39 (t, J = 7.4 Hz, 1H), 2.87 (s, 4H). |
| Intermediate b-19 | 1-(4-(4-hydroxy-6-methyl-2-oxo-2H-pyran-3-yl)thiazol-2-yl)pyrrolidine-2,5-dione (78%, 100 mg) |
| 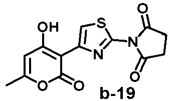 | ¹H NMR (400 MHz, DMSO) δ 14.30 (s, 1H), 8.03 (s, 1H), 6.29 (d, J = 1.0 Hz, 1H), 2.88 (s, 4H), 2.26 (d, J = 0.9 Hz, 3H). |
| Intermediate b-20 | 1-(5-benzoylthiazol-2-yl)pyrrolidine-2,5-dione (46%, 62 mg) |
| 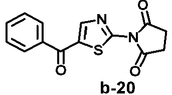 | ¹H NMR (600 MHz, DMSO) δ 7.84 (s, 1H), 7.14 - 6.68 (m, 5H), 2.54 (s, 4H). |

### Example 1: Synthesis of N¹-(3,4-dimethoxyphenethyl)-N⁴-(4-(7-methoxycoumarin-3-yl)thiazol-2-yl)succinamide

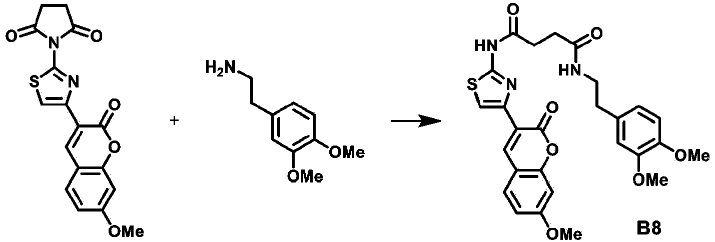

In a reactor equipped with a condenser, Intermediate b-1 (0.20 mmol) was dissolved in anhydrous dioxane (2 mL) under argon gas, and 2-(3,4-dimethoxyphenyl)ethan-1-amine (0.22 mmol, CAS: 120-20-7, TCI/D0678) was added thereto. The reaction mixture was stirred at 110°C for 5 hours. After completion of the reaction, the reaction mixture was concentrated under vacuum. Water (20 mL) was added to the obtained residue to precipitate the product. The precipitated product was separated by centrifugation or filtration and freeze-dried to obtain the compound of Example 1 (Compound B8, yield: 89%, 96 mg).

¹H NMR (400 MHz, DMSO) δ 12.30 (s, 1H), 8.54 (s, 1H), 7.99 (t, J = 5.6 Hz, 1H), 7.89 (s, 1H), 7.77 (d, J = 8.7 Hz, 1H), 7.09 (d, J = 2.4 Hz, 1H), 7.00 (dd, J = 8.6, 2.5 Hz, 1H), 6.84 (d, J = 8.2 Hz, 1H), 6.80 (d, J = 2.0 Hz, 1H), 6.70 (dd, J = 8.1, 2.0 Hz, 1H), 3.88 (s, 3H), 3.74 (s, 3H), 3.70 (s, 3H), 3.23 (dt, J = 6.7, 6.7 Hz, 2H), 2.68 (t, J = 6.9 Hz, 2H), 2.62 (t, J = 7.4 Hz, 2H), 2.44 (t, J = 7.0 Hz, 2H).

### Examples 2 to 81

The compounds of Examples 2 to 81 were each obtained in the same manner as in Example 1, except that Intermediate b-1 and 2-(3,4-dimethoxyphenyl)ethan-1-amine of Example 1 were changed as follows.

**[Table 4]**

| Example No. | Intermediate b | Amine compound |
|---|---|---|
| Example 2 | Intermediate b-1 | 3,4-dimethoxybenzylamine (CAS: 5763-61-1, Alfa Aesar/B25033) |
| Example 3 | Intermediate b-1 | 2-(2-aminoethyl)pyridine (CAS: 2706-56-1, TCI/A1999) |
| Example 4 | Intermediate b-1 | 2-(4-aminophenyl)ethylamine (CAS: 13472-00-9, Alfa Aesar/L15782) |
| Example 5 | Intermediate b-1 | L-phenylalanine methyl ester hydrochloride (CAS: 7524-50-7, TCI/P1278) |
| Example 6 | Intermediate b-1 | 4-(2-aminoethyl)morpholine (CAS: 2038-03-1, Sigma/A55004) |
| Example 7 | Intermediate b-1 | 2-phenylethylamine (CAS: 64-04-0, TCI/P0085) |
| Example 8 | Intermediate b-1 | dopamine hydrochloride (CAS: 62-31-7, Alfa/A11136) |
| Example 9 | Intermediate b-1 | 3-(2-aminoethyl)pyridine (CAS: 20173-24-4, Alfa/L16311) |
| Example 10 | Intermediate b-1 | 4-(2-aminoethyl)pyridine (CAS: 13258-63-4, TCI/A1264) |
| Example 11 | Intermediate b-1 | histamine (CAS: 51-45-6, Alfa/J61727) |
| Example 12 | Intermediate b-1 | 2-(4-nitrophenyl)ethylamine hydrochloride (CAS: 29968-78-3, Alfa/H64894) |
| Example 13 | Intermediate b-1 | 2-(2-aminoethyl)thiophene (CAS: 30433-91-1, TCI/AI637) |
| Example 14 | Intermediate b-1 | 2-(3-methoxyphenyl)ethylamine (CAS: 2039-67-0, TCI/M1891) |
| Example 15 | Intermediate b-1 | 2-(4-methoxyphenyl)ethylamine (CAS: 55-81-2, TCI/M0795) |
| Example 16 | Intermediate b-1 | 3-phenylpropylamine (CAS: 2038-57-5, TCI/P0664) |
| Example 17 | Intermediate b-1 | benzylamine (CAS: 100-46-9, TCI/B0406) |
| Example 18 | Intermediate b-1 | 2,2-diphenylethylamine (CAS: 3963-62-0, TCI/D2018) |
| Example 19 | Intermediate b-1 | tryptamine (CAS: 61-54-1, Alfa Aesar/A11116.09) |
| Example 20 | Intermediate b-1 | 2-(3,4-(methylenedioxy)phenyl)ethylamine hydrochloride (CAS: 1653-64-1, Alfa Aesar/H60208) |
| Example 21 | Intermediate b-1 | 2-naphthaleneethanamine (CAS: 2017-68-7, Sigma/667167) |
| Example 22 | Intermediate b-1 | 2-(3,4-dichlorophenyl)ethylamine (CAS: 21581-45-3, TCI/D2927) |
| Example 23 | Intermediate b-1 | 2-(p-tolyl)ethylamine (CAS: 3261-62-9, Alfa Aesar/15788) |
| Example 24 | Intermediate b-1 | 2-(2-fluorophenyl)ethylamine (CAS: 52721-69-4, TCI/F0933) |
| Example 25 | Intermediate b-1 | 2-(4-fluorophenyl)ethylamine (CAS: 1583-88-6, TCI/F0829) |
| Example 26 | Intermediate b-1 | 4-(2-aminoethyl)benzenesulfonyl fluoride hydrochloride (CAS: 30827-99-7, Alfa Aesar/H26473) |
| Example 27 | Intermediate b-1 | N-(2-aminoethyl)piperazine (CAS: 140-31-6, TCI/A0304) |
| Example 28 | Intermediate b-1 | 4-(2-aminoethyl)thiomorpholine 1,1-dioxide (CAS: 89937-52-0, TCI/A1803) |
| Example 29 | Intermediate b-1 | 4-picolylamine (CAS: 3731-53-1, TCI/P0779) |
| Example 30 | Intermediate b-1 | 3-(pyridin-4-yl)propan-1-amine (CAS: 30532-36-6, Ambeed/A139358) |
| Example 31 | Intermediate b-1 | pyrimidin-4-ylmethanamine dihydrochloride (CAS: 618446-08-5, Ambeed/A283503) |
| Example 32 | Intermediate b-1 | pyridazin-4-ylmethanamine dihydrochloride (CAS: 1028615-75-9, Ambeed/A524320) |
| Example 33 | Intermediate b-1 | (2-fluoropyridin-4-yl)methanamine dihydrochloride (CAS: 667906-60-7, Ambeed/A210034) |
| Example 34 | Intermediate b-1 | (2-chloropyridin-4-yl)methanamine hydrochloride (CAS: 916210-98-5, Ambeed/A166155) |
| Example 35 | Intermediate b-1 | (2-bromopyridin-4-yl)methanamine (CAS: 858362-82-0, Ambeed/A150324) |
| Example 36 | Intermediate b-1 | 4-(aminomethyl)pyridin-2-amine (CAS: 199296-51-0, Ambeed/A548509) |
| Example 37 | Intermediate b-1 | pyridine-2,4-diamine (CAS: 461-88-1, Ambeed/A202971) |
| Example 38 | Intermediate b-1 | 3,4,5-trimethoxybenzylamine (CAS: 18638-99-8, TCI/T3087) |
| Example 39 | Intermediate b-1 | 8-aminoquinoline (CAS: 578-66-5, Alfa/C12371) |
| Example 40 | Intermediate b-14 | 2-(3,4-dimethoxyphenyl)ethylamine (CAS: 120-20-7, TCI/D0678) |
| Example 41 | Intermediate b-15 | 2-(3,4-dimethoxyphenyl)ethylamine (CAS: 120-20-7, TCI/D0678) |
| Example 42 | Intermediate b-16 | 2-(3,4-dimethoxyphenyl)ethylamine (CAS: 120-20-7, TCI/D0678) |
| Example 43 | Intermediate b-17 | 2-(3,4-dimethoxyphenyl)ethylamine (CAS: 120-20-7, TCI/D0678) |
| Example 44 | Intermediate b-4 | 2-(3,4-dimethoxyphenyl)ethylamine (CAS: 120-20-7, TCI/D0678) |
| Example 45 | Intermediate b-5 | 2-(3,4-dimethoxyphenyl)ethylamine (CAS: 120-20-7, TCI/D0678) |
| Example 46 | Intermediate b-3 | 2-(3,4-dimethoxyphenyl)ethylamine (CAS: 120-20-7, TCI/D0678) |
| Example 47 | Intermediate b-7 | 2-(3,4-dimethoxyphenyl)ethylamine (CAS: 120-20-7, TCI/D0678) |
| Example 48 | Intermediate b-8 | 2-(3,4-dimethoxyphenyl)ethylamine (CAS: 120-20-7, TCI/D0678) |
| Example 49 | Intermediate b-9 | 2-(3,4-dimethoxyphenyl)ethylamine (CAS: 120-20-7, TCI/D0678) |
| Example 50 | Intermediate b-6 | 2-(3,4-dimethoxyphenyl)ethylamine (CAS: 120-20-7, TCI/D0678) |
| Example 51 | Intermediate b-10 | 2-(3,4-dimethoxyphenyl)ethylamine (CAS: 120-20-7, TCI/D0678) |
| Example 52 | Intermediate b-2 | 2-(3,4-dimethoxyphenyl)ethylamine (CAS: 120-20-7, TCI/D0678) |
| Example 53 | Intermediate b-20 | 4-(2-aminoethyl)pyridine (CAS: 13258-63-4, TCI/A1264) |
| Example 54 | Intermediate b-20 | 2-(3,4-dimethoxyphenyl)ethylamine (CAS: 120-20-7, TCI/D0678) |
| Example 55 | Intermediate b-4 | 4-(2-aminoethyl)pyridine (CAS: 13258-63-4, TCI/A1264) |
| Example 56 | Intermediate b-5 | 4-(2-aminoethyl)pyridine (CAS: 13258-63-4, TCI/A1264) |
| Example 57 | Intermediate b-3 | 4-(2-aminoethyl)pyridine (CAS: 13258-63-4, TCI/A1264) |
| Example 58 | Intermediate b-7 | 4-(2-aminoethyl)pyridine (CAS: 13258-63-4, TCI/A1264) |
| Example 59 | Intermediate b-6 | 4-(2-aminoethyl)pyridine (CAS: 13258-63-4, TCI/A1264) |
| Example 60 | Intermediate b-10 | 4-(2-aminoethyl)pyridine (CAS: 13258-63-4, TCI/A1264) |
| Example 61 | Intermediate b-2 | 4-(2-aminoethyl)pyridine (CAS: 13258-63-4, TCI/A1264) |
| Example 62 | Intermediate b-9 | 4-(2-aminoethyl)pyridine (CAS: 13258-63-4, TCI/A1264) |
| Example 63 | Intermediate b-11 | 4-(2-aminoethyl)pyridine (CAS: 13258-63-4, TCI/A1264) |
| Example 64 | Intermediate b-12 | 4-(2-aminoethyl)pyridine (CAS: 13258-63-4, TCI/A1264) |
| Example 65 | Intermediate b-14 | 4-(2-aminoethyl)pyridine (CAS: 13258-63-4, TCI/A1264) |
| Example 66 | Intermediate b-15 | 4-(2-aminoethyl)pyridine (CAS: 13258-63-4, TCI/A1264) |
| Example 67 | Intermediate b-16 | 4-(2-aminoethyl)pyridine (CAS: 13258-63-4, TCI/A1264) |
| Example 68 | Intermediate b-17 | 4-(2-aminoethyl)pyridine (CAS: 13258-63-4, TCI/A1264) |
| Example 69 | Intermediate b-13 | 4-(2-aminoethyl)pyridine (CAS: 13258-63-4, TCI/A1264) |
| Example 70 | Intermediate b-13 | 2-(3,4-dimethoxyphenyl)ethylamine (CAS: 120-20-7, TCI/D0678) |
| Example 71 | Intermediate b-18 | 4-(2-aminoethyl)pyridine (CAS: 13258-63-4, TCI/A1264) |
| Example 72 | Intermediate b-20 | 4-(2-aminoethyl)pyridine (CAS: 13258-63-4, TCI/A1264) |
| Example 73 | Intermediate b-20 | 2-(3,4-dimethoxyphenyl)ethylamine (CAS: 120-20-7, TCI/D0678) |
| Example 74 | Intermediate b-1 | 5,6-dimethoxy-2-(4-piperidylmethyl)-1-indanone hydrochloride (CAS: 120013-39-0, TCI/D3997) |
| Example 75 | Intermediate b-1 | 4-(4-aminopiperidino)pyridine dihydrochloride (CAS: 1169396-92-2, Ambeed/A689438) |
| Example 76 | Intermediate b-1 | 1-(4-pyridyl)piperazine (CAS: 1008-91-9, TCI, P1863) |
| Example 77 | Intermediate b-1 | 4-(4-piperidyl)pyridine (CAS: 581-45-3, TCI/P2012) |
| Example 78 | Intermediate b-1 | trimetazidine dihydrochloride (CAS: 13171-25-0, TCI/T2726) |
| Example 79 | Intermediate b-1 | quinoline-4-carbohydrazide (CSA: 29620-62-0, Ambeed/A600347) |
| Example 80 | Intermediate b-1 | 1H-indole-6-carbohydrazide (CAS: 851211-74-0, Ambeed/A208763) |
| Example 81 | Intermediate b-1 | 5-(pyridin-4-yl)-1,3,4-thiadiazol-2-amine (CAS: 2002-04-2, Ambeed/A857207) |

**[Table 5]**

| | |
|---|---|
| Example 2 | N¹-(3,4-dimethoxyphenyl)-N⁴-(4-(7-methoxycoumarin-3-yl)thiazol-2-yl)succinamide (Compound C1, 73%, 115 mg) |
| 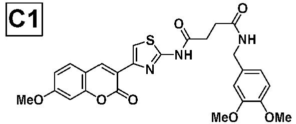 | ¹H NMR (400 MHz, DMSO) δ 11.82 (br s, 1H), 8.52 (s, 1H), 8.35 (s, 1H), 7.88 (s, 1H), 7.75 (d, J = 8.7 Hz, 1H), 7.21 - 6.94 (m, 2H), 6.94 - 6.65 (m, 3H), 4.20 (br s, 2H), 3.88 (s, 3H), 3.74 (s, 3H), 3.71 (s, 3H), 3.32 (br s, 2H), 2.79 - 2.69 (m, 2H). |
| Example 3 | N¹-(2-pyridylethyl)-N⁴-(4-(7-methoxycoumarin-3-yl)thiazol-2-yl)succinamide (Compound C2, 52%, 75 mg) |
| 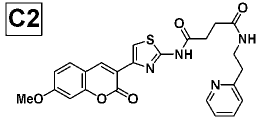 | ¹H NMR (400 MHz, DMSO) δ 12.23 (s, 1H), 8.53 (s, 1H), 8.50 - 8.46 (m, 1H), 8.00 (t, J = 5.6 Hz, 1H), 7.88 (s, 1H), 7.76 (d, J = 8.7 Hz, 1H), 7.69 (td, J = 7.7, 1.9 Hz, 1H), 7.27 - 7.17 (m, 2H), 7.07 (d, J = 2.4 Hz, 1H), 7.00 (dd, J = 8.6, 2.4 Hz, 1H), 3.88 (s, 3H), 3.45 - 3.35 (m, 2H), 2.86 (t, J = 7.3 Hz, 2H), 2.68 (t, J = 6.9 Hz, 2H), 2.43 (t, J = 7.0 Hz, 2H). |
| Example 4 | N¹-(4-aminophenethyl)-N⁴-(4-(7-methoxycoumarin-3-yl)thiazol-2-yl)succinamide (Compound C3, 46%, 68 mg) |
| 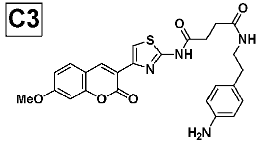 | ¹H NMR (400 MHz, DMSO) δ 12.22 (s, 1H), 8.50 (s, 1H), 7.92 (t, J = 5.6 Hz, 1H), 7.86 (s, 1H), 7.73 (d, J = 8.7 Hz, 1H), 7.04 (d, J = 2.4 Hz, 1H), 6.97 (dd, J = 8.7, 2.4 Hz, 1H), 6.83 (d, J = 8.4 Hz, 2H), 6.47 (d, J = 8.3 Hz, 2H), 4.83 (s, 2H), 3.86 (s, 3H), 3.15 (dt, J = 8.3, 5.9 Hz, 2H), 2.67 (t, J = 7.0 Hz, 2H), 2.53 - 2.48 (m, 2H), 2.43 (t, J = 7.0 Hz, 2H). |
| Example 5 | N¹-((1S)-1-methoxycarbonyl-2-phenylethyl)-N⁴-(4-(7-methoxycoumarin-3-yl)thiazol-2-yl)succinamide (Compound C4, 72%, 115 mg) |
| 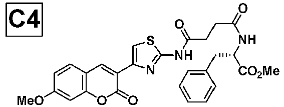 | ¹H MR (400 MHz, DMSO) δ 12.24 (s, 1H), 8.51 (s, 1H), 8.44 (d, J = 7.7 Hz, 1H), 7.87 (s, 1H), 7.74 (d, J = 8.7 Hz, 1H), 7.31 - 7.18 (m, 5H), 7.06 (d, J = 2.4 Hz, 1H), 6.98 (dd, J = 8.7, 2.4 Hz, 1H), 4.45 (td, J = 8.3, 5.8 Hz, 1H), 3.87 (s, 3H), 3.58 (s, 3H), 3.01 (dd, J = 13.7, 5.7 Hz, 1H), 2.95 - 2.87 (m, 1H), 2.63 (t, J = 7.1 Hz, 2H), 2.48 - 2.39 (m, 1H). |
| Example 6 | N¹-(4-morpholinylethyl)-N⁴-(4-(7-methoxycoumarin-3-yl)thiazol-2-yl)succinamide (Compound C5, 26%, 38 mg) |
| 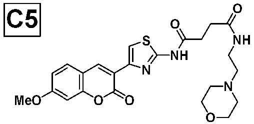 | ¹H NMR (400 MHz, DMSO) δ 12.24 (s, 1H), 8.53 (s, 1H), 7.88 (s, 1H), 7.83 (t, J = 5.6 Hz, 1H), 7.76 (d, J = 8.6 Hz, 1H), 7.07 (d, J = 2.4 Hz, 1H), 7.00 (dd, J = 8.7, 2.4 Hz, 1H), 3.88 (s, 3H), 3.55 (t, J = 4.7 Hz, 4H), 3.16 (q, J = 6.5 Hz, 2H), 2.69 (t, J = 7.0 Hz, 2H), 2.45 (t, J = 7.0 Hz, 2H), 2.38 - 2.28 (m, 4H). |
| Example 7 | N¹-(2-phenethyl)-N⁴-(4-(7-methoxycoumarin-3-yl)thiazol-2-yl)succinamide (Compound C6, 21%, 51 mg) |
| 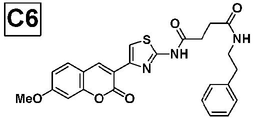 | ¹H NMR (400 MHz, DMSO) δ 11.84 (s, 1H), 8.54 (s, 1H), 8.04 (t, J = 5.6 Hz, 1H), 7.89 (s, 1H), 7.77 (d, J = 8.7 Hz, 1H), 7.33 - 7.15 (m, 5H), 7.09 (d, J = 2.4 Hz, 1H), 7.00 (dd, J = 8.7, 2.5 Hz, 1H), 3.89 (s, 3H), 3.31 - 3.21 (m, 2H), 2.75 - 2.63 (m, 4H), 2.45 (t, J = 7.0 Hz, 2H). |
| Example 8 | N¹-(3,4-dihydroxyphenethyl)-N⁴-(4-(7-methoxycoumarin-3-yl)thiazol-2-yl)succinamide (Compound C7, 56%, 85 mg) |
| 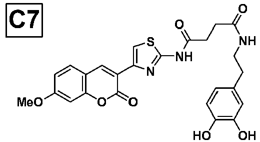 | ¹H NMR (600 MHz, DMSO) δ 12.28 (s, 1H), 8.86 (s, 1H), 8.75 (s, 1H), 8.53 (d, J = 15.3 Hz, 1H), 8.00 (td, J = 5.6, 1.7 Hz, 1H), 7.86 (d, J = 18.8 Hz, 1H), 7.69 (dd, J = 46.6, 8.7 Hz, 1H), 6.65 (d, J = 8.0 Hz, 1H), 6.60 (d, J = 2.0 Hz, 1H), 6.46 (dd, J = 8.0, 2.1 Hz, 1H), 3.88 (s, 3H), 3.19 (dt, J = 8.2, 6.1 Hz, 2H), 2.70 (td, J = 7.1, 2.8 Hz, 2H), 2.56 - 2.48 (m, 2H), 2.46 (td, J = 7.1, 1.9 Hz, 2H). |
| Example 9 | N¹-(3-pyridylethyl)-N⁴-(4-(7-methoxycoumarin-3-yl)thiazol-2-yl)succinamide (Compound C8, 99%, 71 mg) |
| 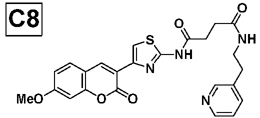 | ¹H NMR (400 MHz, DMSO) δ 10.24 (s, 1H), 8.54 (s, 1H), 8.44 (s, 1H), 8.04 (t, J = 5.6 Hz, 1H), 7.89 (s, 1H), 7.77 (d, J = 8.7 Hz, 1H), 7.67 - 7.61 (m, 1H), 7.31 (dd, J = 7.7, 4.8 Hz, 1H), 7.08 (d, J = 2.3 Hz, 1H), 7.00 (dd, J = 8.7, 2.4 Hz, 1H), 3.89 (s, 3H), 3.30 (q, J = 6.7 Hz, 2H), 2.71 (dt, J = 18.2, 7.0 Hz, 4H), 2.44 (t, J = 7.0 Hz, 2H). |
| Example 10 | N'-(4-pyridylethyl)-N'-(4-(7-methoxycoumarin-3-yl)thiazol-2-yl)succinamide (Compound C9, 82%, 78 mg) |
| 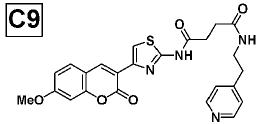 | ¹H NMR (400 MHz, DMSO) δ 12.30 (s, 1H), 8.54 (s, 1H), 8.47 - 8.41 (m, 2H), 8.04 (t, J = 5.6 Hz, 1H), 7.89 (s, 1H), 7.77 (d, J = 8.7 Hz, 1H), 7.25 - 7.21 (m, 2H), 7.09 (d, J = 2.4 Hz, 1H), 7.00 (dd, J = 8.7, 2.4 Hz, 1H), 3.88 (s, 3H), 3.33 - 3.26 (m, 2H), 2.70 (dt, J = 17.9, 7.0 Hz, 4H), 2.42 (t, J = 7.0 Hz, 2H). |
| Example 11 | N¹-(2-(1H-imidazol-5-yl)ethyl)-N⁴-(4-(7-methoxycoumarin-3-yl)thiazol-2-yl)succinamide (Compound C10, 96%, 67 mg) |
| 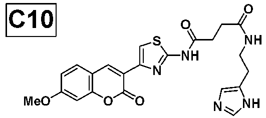 | ¹H NMR (400 MHz, DMSO) δ 12.21 (s, 1H), 11.80 (s, 1H), 8.52 (s, 1H), 7.99 (t, J = 5.7 Hz, 2H), 7.88 (d, J = 1.9 Hz, 1H), 7.81 - 7.69 (m, 1H), 7.52 (s, 1H), 7.07 (s, 1H), 6.99 (d, J = 8.7 Hz, 1H), 6.79 (s, 1H), 3.88 (s, 3H), 3.32 - 3.22 (m, 2H), 2.70 (t, J = 7.0 Hz, 2H), 2.63 (t, J = 7.5 Hz, 2H), 2.46 (t, J = 7.1 Hz, 2H). |
| Example 12 | N¹-(4-nitrophenethyl)-N⁴-(4-(7-methoxycoumarin-3-yl)thiazol-2-yl)succinamide (Compound C11, 70%, 55 mg) |
| 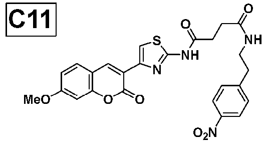 | ¹H NMR (400 MHz, DMSO) δ 12.26 (s, 1H), 8.54 (s, 1H), 8.15 (d, J = 8.8 Hz, 2H), 8.03 (t, J = 5.6 Hz, 1H), 7.89 (s, 1H), 7.77 (d, J = 8.8 Hz, 1H), 7.49 (d, J = 8.7 Hz, 2H), 7.09 (d, J = 2.4 Hz, 1H), 7.00 (dd, J = 8.7, 2.5 Hz, 1H), 3.88 (s, 3H), 3.42 - 3.24 (m, 2H), 2.85 (t, J = 6.9 Hz, 2H), 2.67 (t, J = 7.0 Hz, 2H), 2.42 (t, J = 6.9 Hz, 2H). |
| Example 13 | N¹-(2-thiophen-2-yl-ethyl)-N⁴-(4-(7-methoxycoumarin-3-yl)thiazol-2-yl)succinamide (Compound C12, 91%, 66 mg) |
| 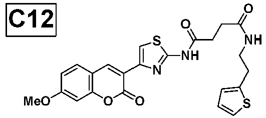 | ¹H NMR (400 MHz, DMSO) δ 12.29 (s, 1H), 8.54 (s, 1H), 8.09 (t, J = 5.6 Hz, 1H), 7.89 (s, 1H), 7.77 (d, J = 8.7 Hz, 1H), 7.33 (dd, J = 5.1, 1.3 Hz, 1H), 7.08 (d, J = 2.5 Hz, 1H), 7.00 (dd, J = 8.7, 2.5 Hz, 1H), 6.95 (dd, J = 5.1, 3.4 Hz, 1H), 6.89 (d, J = 3.4 Hz, 1H), 3.89 (s, 3H), 3.29 (q, J = 7.6 Hz, 2H), 2.93 (t, J = 7.2 Hz, 2H), 2.70 (t, J = 6.9 Hz, 2H), 2.47 (t, J = 6.9 Hz, 2H). |
| Example 14 | N¹-(3-methoxyphenethyl)-N⁴-(4-(7-methoxycoumarin-3-yl)thiazol-2-yl)succinamide (Compound C13, 68%, 52 mg) |
| 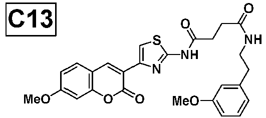 | ¹H NMR (400 MHz, DMSO) δ 12.28 (s, 1H), 8.53 (s, 1H), 8.01 (t, J = 5.6 Hz, 1H), 7.88 (s, 1H), 7.76 (d, J = 8.7 Hz, 1H), 7.20 (t, J = 7.9 Hz, 1H), 7.07 (d, J = 2.5 Hz, 1H), 7.00 (dd, J = 8.7, 2.4 Hz, 1H), 6.78 (dd, J = 4.4, 2.5 Hz, 3H), 3.89 (s, 3H), 3.74 (s, 3H), 3.27 (dt, J = 7.8, 6.2 Hz, 2H), 2.76 - 2.64 (m, 4H), 2.45 (t, J = 7.0 Hz, 2H). |
| Example 15 | N¹-(4-methoxyphenethyl)-N⁴-(4-(7-methoxycoumarin-3-yl)thiazol-2-yl)succinamide (Compound C14, 54%, 41 mg) |
| 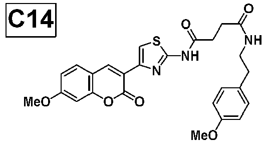 | ¹H NMR (400 MHz, DMSO) δ 12.26 (br s, 1H), 8.54 (s, 1H), 7.96 (br s, 1H), 7.89 (s, 1H), 7.75 (s, 1H), 7.66 (s, 1H), 7.10 (br s, 2H), 7.00 (s, 1H), 6.84 (d, J = 8.1 Hz, 2H), 3.88 (s, 3H), 3.71 (s, 3H), 3.21 (br s, 2H), 2.65 (d, J = 19.8 Hz, 4H), 2.44 (br s, 2H). |
| Example 16 | N¹-(phenylpropyl)-N⁴-(4-(7-methoxycoumarin-3-yl)thiazol-2-yl)succinamide (Compound C15, 43%, 32 mg) |
| 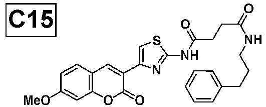 | ¹H NMR (400 MHz, DMSO) δ 12.28 (s, 1H), 8.53 (s, 1H), 7.94 (t, J = 5.6 Hz, 1H), 7.88 (s, 1H), 7.81 - 7.71 (m, 1H), 7.27 (t, J = 7.5 Hz, 2H), 7.23 - 7.14 (m, 3H), 7.07 (t, J = 2.5 Hz, 1H), 7.00 (dt, J = 8.6, 2.0 Hz, 1H), 3.88 (d, J = 1.2 Hz, 4H), 3.06 (q, J = 6.6 Hz, 2H), 2.71 (t, J = 7.0 Hz, 3H), 2.62 - 2.54 (m, 2H), 2.47 (t, J = 6.9 Hz, 2H), 1.77 - 1.62 (m, 2H). |
| Example 17 | N¹-(benzyl)-N⁴-(4-(7-methoxycoumarin-3-yl)thiazol-2-yl)succinamide (Compound C16, 56%, 39 mg) |
| 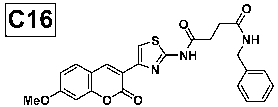 | ¹H NMR (400 MHz, DMSO) δ 12.30 (s, 1H), 8.53 (s, 1H), 8.44 (t, J = 6.0 Hz, 1H), 7.89 (s, 1H), 7.76 (d, J = 8.5 Hz, 1H), 7.35 - 7.19 (m, 5H), 7.08 (d, J = 2.4 Hz, 1H), 7.00 (dd, J = 8.7, 2.5 Hz, 1H), 4.28 (d, J = 5.9 Hz, 2H), 3.89 (s, 3H), 2.75 (t, J = 6.9 Hz, 2H), 2.55 (t, J = 6.9 Hz, 2H). |
| Example 18 | N'-(2,2-diphenylethyl)-N'-(4-(7-methoxycoumarin-3-yl)thiazol-2-yl)succinamide (Compound C17, 45%, 37 mg) |
| 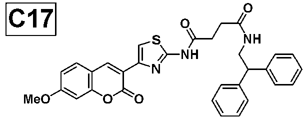 | ¹H NMR (400 MHz, DMSO) δ 11.98 (s, 1H), 8.54 (s, 1H), 7.99 (t, J = 5.6 Hz, 1H), 7.89 (s, 1H), 7.77 (dd, J = 8.8, 1.4 Hz, 1H), 7.36 - 7.24 (m, 10H), 7.08 (d, J = 2.3 Hz, 1H), 7.01 (dd, J = 8.7, 2.3 Hz, 1H), 4.18 (t, J = 7.8 Hz, 1H), 3.89 (s, 3H), 3.70 (dd, J = 7.8, 5.6 Hz, 2H), 2.64 (t, J = 7.1 Hz, 2H), 2.37 (t, J = 7.1 Hz, 2H). |
| Example 19 | N¹-(2-indol-3-ylethyl)-N⁴-(4-(7-methoxycoumarin-3-yl)thiazol-2-yl)succinamide (Compound C18, 65%, 50 mg) |
| 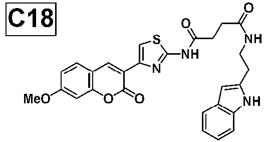 | ¹H NMR (400 MHz, DMSO) δ 12.28 (s, 1H), 10.80 (s, 1H), 8.54 (s, 1H), 8.04 (t, J = 5.7 Hz, 1H), 7.89 (s, 1H), 7.76 (dd, J = 8.8, 1.5 Hz, 1H), 7.53 (d, J = 7.8 Hz, 1H), 7.40 - 7.31 (m, 1H), 7.16 (d, J = 2.3 Hz, 1H), 7.11 - 7.03 (m, 2H), 7.04 - 6.92 (m, 2H), 3.89 (s, 3H), 3.37 - 3.28 (m, 2H), 2.82 (t, J = 7.5 Hz, 2H), 2.72 (t, J = 7.0 Hz, 2H), 2.48 (t, J = 7.3 Hz, 2H). |
| Example 20 | N¹-(5-benzo-1,3-dioxolylethyl)-N⁴-(4-(7-methoxycoumarin-3-yl)thiazol-2-yl)succinamide (Compound C19, 54%, 42 mg) |
| 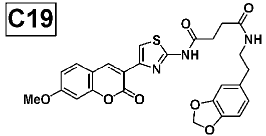 | ¹H NMR (400 MHz, DMSO) δ 12.28 (s, 1H), 8.55 (s, 1H), 7.97 (t, J = 5.6 Hz, 1H), 7.89 (d, J = 1.6 Hz, 1H), 7.77 (d, J = 8.7 Hz, 1H), 7.09 (d, J = 2.5 Hz, 1H), 7.01 (dd, J = 8.7, 2.4 Hz, 1H), 6.91 - 6.75 (m, 2H), 6.66 (dd, J = 7.9, 1.8 Hz, 1H), 5.96 (s, 2H), 3.89 (s, 3H), 3.22 (q, J = 6.8 Hz, 2H), 2.69 (t, J = 7.1 Hz, 2H), 2.62 (t, J = 7.4 Hz, 1H), 2.44 (t, J = 6.9 Hz, 1H). |
| Example 21 | N¹-(2-naphthalen-2-ylethyl)-N⁴-(4-(7-methoxycoumarin-3-yl)thiazol-2-yl)succinamide (Compound C20, 88%, 70 mg) |
| 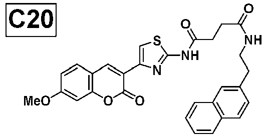 | ¹H NMR (400 MHz, DMSO) δ 12.28 (s, 1H), 8.55 (s, 1H), 8.05 (s, 1H), 7.96 - 7.81 (m, 5H), 7.77 (d, J = 8.6 Hz, 1H), 7.72 (s, 1H), 7.52 - 7.42 (m, 2H), 7.40 (d, J = 8.3 Hz, 1H), 7.09 (s, 1H), 7.05 - 6.99 (m, 1H), 3.89 (s, 3H), 2.88 (t, J = 7.3 Hz, 2H), 2.69 (d, J = 7.2 Hz, 2H), 2.48 - 2.34 (m, 4H). |
| Example 22 | N¹-(3,4-dichlorophenethyl)-N⁴-(4-(7-methoxycoumarin-3-yl)thiazol-2-yl)succinamide (Compound C21, 83%, 68 mg) |
| 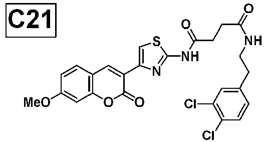 | ¹H NMR (400 MHz, DMSO) δ 12.28 (s, 1H), 8.55 (s, 1H), 7.99 (t, J = 5.7 Hz, 1H), 7.89 (s, 1H), 7.77 (d, J = 8.7 Hz, 1H), 7.57 - 7.49 (m, 2H), 7.21 (dd, J = 8.3, 2.1 Hz, 1H), 7.09 (d, J = 2.4 Hz, 1H), 7.01 (dd, J = 8.7, 2.4 Hz, 1H), 3.89 (s, 3H), 3.28 (q, J = 6.6 Hz, 2H), 2.70 (dt, J = 13.3, 6.9 Hz, 4H), 2.43 (t, J = 7.0 Hz, 2H). |
| Example 23 | N¹-(4-methylphenethyl)-N⁴-(4-(7-methoxycoumarin-3-yl)thiazol-2-yl)succinamide (Compound C22, 76%, 35 mg) |
| 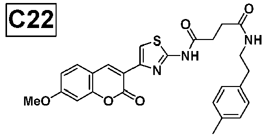 | ¹H NMR (300 MHz, DMSO) δ 12.28 (br s, 1H), 8.55 (s, 1H), 7.98 (d, J = 6.0 Hz, 1H), 7.90 (s, 1H), 7.78 (d, J = 8.7 Hz, 1H), 7.18 - 7.02 (m, 5H), 7.01 (dd, J = 8.7, 2.4 Hz, 1H), 3.89 (s, 3H), 3.28 - 3.12 (m, 2H), 2.76 - 2.60 (m, 4H), 2.44 (t, J = 6.9 Hz, 2H), 2.26 (s, 3H). |
| Example 24 | N¹-(2-fluorophenethyl)-N⁴-(4-(7-methoxycoumarin-3-yl)thiazol-2-yl)succinamide (Compound C23, 28%, 14 mg) |
| 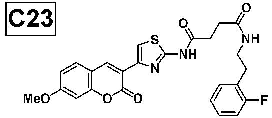 | ¹H NMR (400 MHz, DMSO) δ 12.30 (s, 1H), 8.54 (s, 1H), 8.07 (t, J = 5.7 Hz, 1H), 7.89 (s, 1H), 7.77 (d, J = 8.7 Hz, 1H), 7.37 - 7.22 (m, 2H), 7.21 - 7.10 (m, 2H), 7.09 (d, J = 2.4 Hz, 1H), 7.00 (dd, J = 8.6, 2.5 Hz, 1H), 3.89 (s, 3H), 3.32 - 3.17 (m, 2H), 2.75 (t, J = 7.3 Hz, 2H), 2.69 (t, J = 7.0 Hz, 2H), 2.44 (t, J = 7.0 Hz, 2H). |
| Example 25 | N¹-(4-fluorophenethyl)-N⁴-(4-(7-methoxycoumarin-3-yl)thiazol-2-yl)succinamide (Compound C24, 81%, 12 mg) |
| 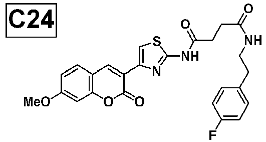 | ¹H NMR (400 MHz, DMSO) δ 12.23 (s, 1H), 8.54 (s, 1H), 8.01 (t, J = 5.7 Hz, 1H), 7.89 (s, 1H), 7.77 (d, J = 8.7 Hz, 1H), 7.31 - 7.21 (m, 2H), 7.16 - 7.05 (m, 3H), 7.00 (dd, J = 8.6, 2.5 Hz, 1H), 3.89 (s, 3H), 3.25 (q, J = 6.8 Hz, 2H), 2.69 (td, J = 7.1, 3.3 Hz, 4H), 2.44 (t, J = 7.0 Hz, 2H). |
| Example 26 | N¹-(4-fluorosulfonylphenethyl)-N⁴-(4-(7-methoxycoumarin-3-yl)thiazol-2-yl)succinamide (Compound C25, 89%, 15 mg) |
| 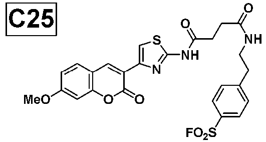 | ¹H NMR (400 MHz, DMSO) δ 12.31 (s, 1H), 8.53 (s, 1H), 8.13 - 7.97 (m, 3H), 7.89 (s, 1H), 7.76 (d, J = 8.7 Hz, 1H), 7.63 (d, J = 8.2 Hz, 2H), 7.08 (d, J = 2.5 Hz, 1H), 7.00 (dd, J = 8.7, 2.5 Hz, 1H), 3.88 (s, 3H), 3.39 - 3.25 (m, 2H), 2.94 - 2.85 (m, 2H), 2.69 (t, J = 6.9 Hz, 2H), 2.43 (t, J = 6.9 Hz, 2H). |
| Example 27 | N¹-(N-piperazinylethyl)-N⁴-(4-(7-methoxycoumarin-3-yl)thiazol-2-yl)succinamide (Compound C26, 21%, 5 mg) |
| 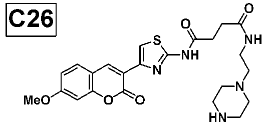 | ¹H NMR (400 MHz, DMSO) δ 12.28 (s, 1H), 8.54 (s, 1H), 7.88 (d, J = 5.7 Hz, 1H), 7.77 (dd, J = 8.7, 2.8 Hz, 1H), 7.08 (dd, J = 6.4, 2.4 Hz, 1H), 7.05 - 6.80 (m, 1H), 3.89 (s, 3H), 3.59 - 3.39 (m, 4H), 3.20 (br s, 2H), 2.80 - 2.60 (m, 6H), 2.48 - 2.21 (m, 4H). |
| Example 28 | N¹-(2-(1,1-dioxidothiomorpholino)ethyl)-N⁴-(4-(7-methoxycoumarin-3-yl)thiazol-2-yl)succinamide (Compound C27, 75%, 20 mg) |
| 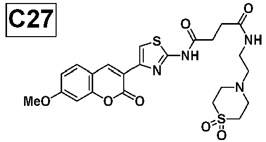 | ¹H NMR (400 MHz, DMSO) δ 11.85 (s, 1H), 8.52 (s, 1H), 7.87 (s, 2H), 7.75 (d, J = 8.7 Hz, 1H), 7.07 (d, J = 2.4 Hz, 1H), 6.99 (dd, J = 8.7, 2.4 Hz, 1H), 3.88 (s, 3H), 3.17 (q, J = 6.3 Hz, 2H), 3.12 - 3.03 (m, 4H), 2.97 - 2.84 (m, 4H), 2.70 (t, J = 7.0 Hz, 2H), 2.54 (d, J = 6.5 Hz, 2H), 2.46 (t, J = 7.0 Hz, 2H). |
| Example 29 | N¹-(4-(7-methoxycoumarin-3-yl)thiazol-2-yl)-N⁴-(pyridin-4-ylmethyl)succinamide (Compound C28,75%,35 mg) |
| 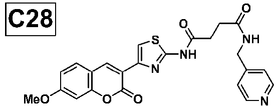 | ¹H NMR (600 MHz, DMSO) δ 12.19 (s, 1H), 8.45 (s, 1H), 8.41 (s, 1H), 8.35 (s, 2H), 7.86 - 7.74 (m, 1H), 7.68 - 7.57 (m, 1H), 7.14 (s, 2H), 7.01 - 6.90 (m, 1H), 6.90 - 6.73 (m, 1H), 4.19 (s, 2H), 3.84 - 3.66 (m, 3H), 2.70 - 2.60 (m, 2H), 2.56 - 2.42 (m, 2H). |
| Example 30 | N¹-(4-(7-methoxycoumarin-3-yl)thiazol-2-yl)-N⁴-(3-(pyridin-4-yl)propyl)succinamide (Compound C29,97%,48 mg) |
| 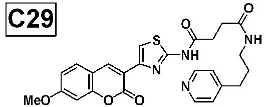 | ¹H NMR (600 MHz, DMSO) δ 12.17 (s, 1H), 8.39 (s, 1H), 8.35 - 8.26 (m, 2H), 7.85 (br s, 2H), 7.75 (s, 1H), 7 7.68 - 7.50 (m, 1H), 7.18 - 7.07 (m, 2H), 6.94 (br s, 1H), 6.90 - 6.75 (m, 1H), 3.76 (s, 3H), 2.98 - 2.85 (m, 2H), 2 2.62 - 2.54 (m, 2H), 2.54 - 2.45 (m, 2H), 2.35 (t, J = 7.1 Hz, 2H), 1.68 - 1.36 (m, 2H). |
| Example 31 | N¹-(4-(7-methoxycoumarin-3-yl)thiazol-2-yl)-N⁴-(pyrimidin-4-ylmethyl)succinamide (Compound C30, 84%,39 mg) |
| 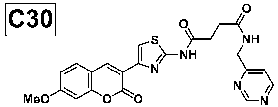 | ¹H NMR (600 MHz, DMSO) δ 12.20 (s, 1H), 8.97 (d, J = 1.4 Hz, 1H), 8.59 (d, J = 5.2 Hz, 1H), 8.54 (t, J = 5.8 Hz, 1H), 8.42 (s, 1H), 7.79 (s, 1H), 7.66 (d, J = 8.8 Hz, 1H), 7.31 (dd, J = 5.2, 1.4 Hz, 1H), 6.98 (d, J = 2.4 Hz, 1H), 6.89 (dd, J = 8.6, 2.4 Hz, 1H), 4.23 (d, J = 6.0 Hz, 2H), 3.77 (s, 3H), 2.64 (t, J = 6.8 Hz, 2H), 2.49 (t, J = 6.7 Hz, 2H). |
| Example 32 | N¹-(4-(7-methoxycoumarin-3-yl)thiazol-2-yl)-N⁴-(pyridazin-4-ylmethyl)succinamide (Compound C31, 56%, 26 mg) |
| 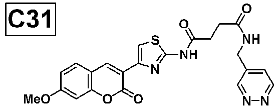 | ¹H NMR (600 MHz, DMSO) δ 12.20 (s, 1H), 9.09 - 9.02 (m, 1H), 9.00 (dd, J = 5.3, 1.3 Hz, 1H), 8.50 (t, J = 6.0 Hz, 1H), 8.42 (s, 1H), 7.79 (s, 1H), 7.65 (d, J = 8.7 Hz, 1H), 7.43 - 7.39 (m, 1H), 6.97 (d, J = 2.3 Hz, 1H), 6.88 (dd, J = 8.6, 2.5 Hz, 1H), 4.23 (d, J = 5.9 Hz, 2H), 3.77 (s, 3H), 2.64 (t, J = 6.8 Hz, 2H), 2.47 (t, J = 6.8 Hz, 2H). |
| Example 33 | N¹-((2-fluoropyridin-4-yl)methyl)-N⁴-((7-methoxycoumarin-3-yl)thiazol-2-yl)succinamide (Compound C32, 41%, 20 mg) |
| 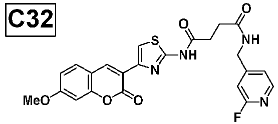 | ¹H NMR (600 MHz, DMSO) δ 12.20 (s, 1H), 8.49 (t, J = 6.1 Hz, 1H), 8.42 (s, 1H), 8.02 (d, J = 5.1 Hz, 1H), 7.78 (s, 1H), 7.65 (d, J = 8.8 Hz, 1H), 7.12 (dt, J = 5.3, 1.7 Hz, 1H), 6.97 (d, J = 2.4 Hz, 1H), 6.92 (s, 1H), 6.89 (dd, J = 8.6, 2.4 Hz, 1H), 4.24 (d, J = 6.0 Hz, 2H), 3.77 (s, 3H), 2.65 (t, J = 6.8 Hz, 2H), 2.47 (t, J = 6.8 Hz, 2H). |
| Example 34 | N¹-((2-chloropyridin-4-yl)methyl)-N⁴-((7-methoxycoumarin-3-yl)thiazol-2-yl)succinamide (Compound C33, 92%, 46 mg) |
| 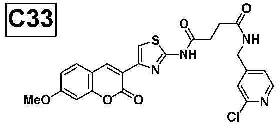 | ¹H NMR (600 MHz, DMSO) δ 12.19 (s, 1H), 8.47 (t, J = 6.0 Hz, 1H), 8.42 (d, J = 2.4 Hz, 1H), 8.19 (d, J = 5.1 Hz, 1H), 7.78 (d, J = 1.3 Hz, 1H), 7.68 - 7.55 (m, 1H), 7.27 (d, J = 1.3 Hz, 1H), 7.17 (dd, J = 5.1, 1.5 Hz, 1H), 6.97 (t, J = 2.8 Hz, 1H), 6.88 (dt, J = 8.7, 2.4 Hz, 1H), 4.21 (d, J = 6.0 Hz, 2H), 3.77 (d, J = 1.1 Hz, 3H), 2.65 (t, J = 6.8 Hz, 2H), 2.46 (t, J = 6.8 Hz, 2H). |
| Example 35 | N¹-((2-bromopyridin-4-yl)methyl)-N⁴-((7-methoxycoumarin-3-yl)thiazol-2-yl)succinamide (Compound C34, 33%, 16 mg) |
| 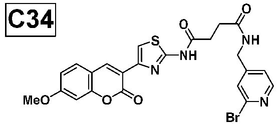 | ¹H NMR (600 MHz, DMSO) δ 12.19 (s, 1H), 8.46 (t, J = 6.1 Hz, 1H), 8.42 (s, 1H), 8.17 (d, J = 5.1 Hz, 1H), 7.78 (s, 1H), 7.65 (d, J = 8.6 Hz, 1H), 7.40 (d, J = 1.4 Hz, 1H), 7.20 (dd, J = 5.1, 1.4 Hz, 1H), 6.97 (d, J = 2.4 Hz, 1H), 6.89 (dd, J = 8.7, 2.4 Hz, 1H), 4.19 (d, J = 6.0 Hz, 2H), 3.77 (s, 3H), 2.64 (t, J = 6.8 Hz, 2H), 2.46 (t, J = 6.8 Hz, 2H). |
| Example 36 | N¹-((2-aminopyridin-4-yl)methyl)-N⁴-((7-methoxycoumarin-3-yl)thiazol-2-yl)succinamide (Compound C35, 33%, 16 mg) |
| 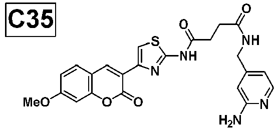 | ¹H NMR (600 MHz, DMSO) δ 12.19 (s, 1H), 8.46 (t, J = 6.1 Hz, 1H), 8.42 (s, 1H), 8.25 (d, J = 5.1 Hz, 1H), 7.78 (s, 1H), 7.65 (d, J = 8.7 Hz, 1H), 7.24 (s, 1H), 7.00 (dd, J = 5.1, 1.7 Hz, 1H), 6.97 (d, J = 2.4 Hz, 1H), 6.89 (dd, J = 8.6, 2.5 Hz, 1H), 4.43 (d, J = 3.8 Hz, 2H), 4.19 (d, J = 6.0 Hz, 2H), 3.77 (d, J = 2.5 Hz, 3H), 2.63 (t, J = 7.0 Hz, 2H), 2.46 (t, J = 6.9 Hz, 2H). |
| Example 37 | N¹-(2-aminopyridin-4-yl)-N⁴-((7-methoxycoumarin-3-yl)thiazol-2-yl)succinamide (Compound C36, 33 %, 16 mg) |
| 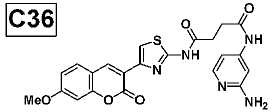 | ¹H NMR (600 MHz, DMSO) δ 12.19 (s, 1H), 8.42 (d, J = 2.0 Hz, 1H), 8.31 (t, J = 6.0 Hz, 1H), 7.78 (d, J = 1.0 Hz, 1H), 7.69 - 7.62 (m, 2H), 6.97 (d, J = 2.4 Hz, 1H), 6.89 (dd, J = 8.7, 2.3 Hz, 1H), 6.25 (dd, J = 5.2, 1.5 Hz, 1H), 6.21 - 6.15 (m, 1H), 5.70 (s, 2H), 3.77 (s, 3H), 2.62 (t, J = 7.0 Hz, 2H), 2.44 (t, J = 7.0 Hz, 2H). |
| Example 38 | N¹-(4-(7-methoxycoumarin-3-yl)thiazol-2-yl)-N⁴-(3,4,5-trimethoxybenzyl)succinamide (Compound C37, 99%, 55 mg) |
| 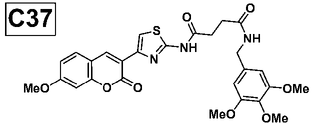 | ¹H NMR (400 MHz, DMSO) δ 12.28 (br s, 1H), 8.54 (s, 1H), 8.41 (br s, 1H), 7.88 (s, 1H), 7.77 (d, J = 9.0 Hz, 1H), 7.08 (br s, 1H), 7.03 - 6.90 (m, 1H), 6.57 (br s, 2H), 4.33 - 4.09 (m, 2H), 3.88 (s, 3H), 3.83 - 3.68 (m, 6H), 3.61 (s, 3H), 2.90 - 2.62 (m, 2H), 2.56 - 2.48 (m, 2H). |
| Example 39 | N¹-(4-(7-methoxycoumarin-3-yl)thiazol-2-yl)-N⁴-(quinolin-8-yl)succinamide (Compound C38, 20%, 10 mg) |
| 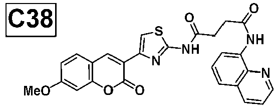 | ¹H NMR (400 MHz, DMSO) δ 12.40 (s, 1H), 10.23 (s, 1H), 8.95 (dd, J = 4.2, 1.7 Hz, 1H), 8.60 (dd, J = 7.7, 1.4 Hz, 1H), 8.55 (s, 1H), 8.41 (dd, J = 8.3, 1.7 Hz, 1H), 7.89 (s, 1H), 7.77 (d, J = 8.7 Hz, 1H), 7.70 - 7.62 (m, 2H), 7.56 (t, J = 8.0 Hz, 1H), 7.09 (d, J = 2.4 Hz, 1H), 7.00 (dd, J = 8.6, 2.4 Hz, 1H), 3.88 (s, 3H), 3.04 - 2.94 (m, 2H), 2.90 - 2.79 (m, 2H). |
| Example 40 | N¹-(3,4-dimethoxyphenethyl)-N⁴-(4-(2,4-difluorophenyl)thiazol-2-yl)succinamide (Compound D1, 68%, 342 mg) |
| 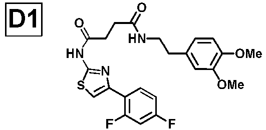 | ¹H NMR (300 MHz, DMSO) δ 12.02 (s, 1H), 8.16 - 7.86 (m, 2H), 7.47 (d, J = 2.6 Hz, 1H), 7.37 (ddd, J = 11.8, 9.3, 2.6 Hz, 1H), 7.21 (td, J = 8.3, 2.6 Hz, 1H), 6.88 - 6.79 (m, 2H), 6.70 (dd, J = 8.1, 2.0 Hz, 1H), 3.75 (s, 3H), 3.71 (s, 3H), 3.30 - 3.18 (m, 2H), 2.66 (dt, J = 17.8, 7.1 Hz, 4H), 2.45 (t, J = 6.9 Hz, 2H). |
| Example 41 | N¹-(3,4-dimethoxyphenethyl)-N⁴-(4-(3,4-difluorophenyl)thiazol-2-yl)succinamide (Compound D2, 68%, 323 mg) |
| 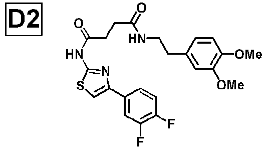 | ¹H NMR (300 MHz, DMSO) δ 12.21 (s, 1H), 8.03 - 7.83 (m, 2H), 7.80 - 7.71 (m, 1H), 7.69 (s, 1H), 7.49 (dt, J = 10.7, 8.6 Hz, 1H), 6.89 - 6.76 (m, 2H), 6.70 (dd, J = 8.1, 2.0 Hz, 1H), 3.31 - 3.19 (m, 2H), 2.66 (dt, J = 18.0, 7.1 Hz, 4H), 2.46 (t, J = 6.9 Hz, 2H). |
| Example 42 | N¹-(3,4-dimethoxyphenethyl)-N⁴-(4-(p-tolyl)thiazol-2-yl)succinamide (Compound D3, 35%, 157 mg) |
| 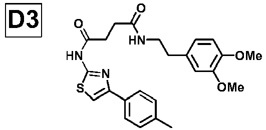 | ¹H NMR (300 MHz, DMSO) δ 9.21 (s, 1H), 7.96 (t, J = 5.6 Hz, 1H), 7.78 (d, J = 8.2 Hz, 2H), 7.51 (s, 1H), 7.30 - 7.21 (m, 2H), 6.90 - 6.79 (m, 2H), 6.71 (dd, J = 8.1, 2.0 Hz, 1H), 3.75 (s, 3H), 3.71 (s, 3H), 3.24 (q, J = 6.7 Hz, 2H), 2.72 - 2.58 (m, 4H), 2.44 (t, J = 7.0 Hz, 2H), 2.33 (s, 3H). |
| Example 43 | N¹-(3,4-dimethoxyphenethyl)-N⁴-(4-(5,6,7,8-tetrahydronaphthalen-2-yl)thiazol-2-yl)succinamide (Compound D4, 85%, 42 mg) |
| 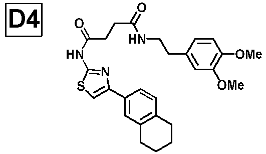 | ¹H NMR (300 MHz, DMSO) δ 11.69 (s, 1H), 7.97 (t, J = 5.6 Hz, 1H), 7.60 (s, 1H), 7.57 (s, 1H), 7.47 (s, 1H), 7.12 - 7.02 (m, 1H), 6.89 - 6.78 (m, 2H), 6.71 (dd, J = 8.1, 2.0 Hz, 1H), 3.75 (s, 3H), 3.71 (s, 3H), 3.32 - 3.18 (m, 2H), 2.82 - 2.57 (m, 4H), 2.45 (t, J = 7.0 Hz, 2H), 1.83 - 1.67 (m, 4H). |
| Example 44 | N¹-(3,4-dimethoxyphenethyl)-N⁴-(4-(7-chlorocoumarin-3-yl)thiazol-2-yl)succinamide (Compound D5, 88%, 61 mg) |
| 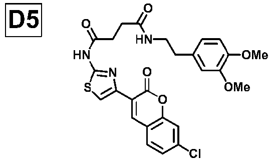 | ¹H NMR (400 MHz, DMSO) δ 12.06 (s, 1H), 8.55 (s, 1H), 7.98 (s, 2H), 7.88 (d, J = 8.4 Hz, 1H), 7.65 (d, J = 2.0 Hz, 1H), 7.46 (dd, J = 8.4, 2.0 Hz, 1H), 6.90 - 6.77 (m, 2H), 6.74 - 6.67 (m, 1H), 3.75 (s, 3H), 3.71 (s, 3H), 3.25 (q, J = 6.8 Hz, 2H), 2.70 (t, J = 6.9 Hz, 2H), 2.64 (t, J = 7.3 Hz, 2H), 2.46 (t, J = 6.9 Hz, 2H). |
| Example 45 | N¹-(3,4-dimethoxyphenethyl)-N⁴-(4-(7-bromocoumarin-3-yl)thiazol-2-yl)succinamide (Compound D6, 70%, 65 mg) |
| 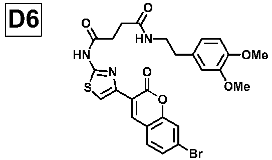 | ¹H NMR (400 MHz, DMSO) δ 12.27 (s, 1H), 8.44 (s, 1H), 7.99 (t, J = 5.6 Hz, 1H), 7.92 (s, 1H), 7.74 - 7.63 (m, 2H), 7.50 (dd, J = 8.3, 1.9 Hz, 1H), 6.87 - 6.77 (m, 2H), 6.69 (dd, J = 8.1, 2.0 Hz, 1H), 3.74 (s, 3H), 3.70 (s, 3H), 3.29 - 3.20 (m, 2H), 2.71 (t, J = 7.0 Hz, 2H), 2.64 (t, J = 7.4 Hz, 2H), 2.47 (t, J = 7.0 Hz, 2H). |
| Example 46 | N¹-(3,4-dimethoxyphenethyl)-N⁴-(4-(7-methylcoumarin-3-yl)thiazol-2-yl)succinamide (Compound D7, 78%, 60 mg) |
| 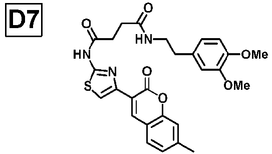 | ¹H NMR (400 MHz, DMSO) δ 12.29 (s, 1H), 8.69 - 8.37 (m, 1H), 7.98 (s, 1H), 7.93 (s, 1H), 7.72 (dd, J = 30.7, 8.1 Hz, 1H), 7.34 - 7.12 (m, 2H), 6.90 - 6.77 (m, 2H), 6.76 - 6.66 (m, 1H), 3.75 (s, 3H), 3.71 (s, 3H), 3.30 - 3.19 (m, 2H), 2.77 - 2.68 (m, 2H), 2.68 - 2.59 (m, 2H), 2.49 - 2.44 (m, 2H), 2.42 (s, 3H). |
| Example 47 | N¹-(3,4-dimethoxyphenethyl)-N⁴-(4-(coumarin-3-yl)thiazol-2-yl)succinamide (Compound D8, 56%, 42 mg) |
| 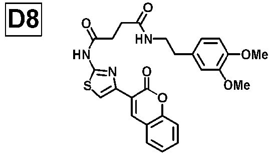 | ¹H NMR (400 MHz, DMSO) δ 12.28 (s, 1H), 8.55 (s, 1H), 7.97 (br s, 2H), 7.82 (d, J = 7.7 Hz, 1H), 7.69 - 7.57 (m, 1H), 7.52 - 7.31 (m, 2H), 6.93 - 6.76 (m, 2H), 6.76 - 6.63 (m, 1H), 3.75 (s, 3H), 3.71 (s, 3H), 3.31 - 3.14 (m, 2H), 2.78 - 2.68 (m, 2H), 2.68 - 2.59 (m, 2H), 2.50 - 2.15 (m, 2H). |
| Example 48 | N¹-(3,4-dimethoxyphenethyl)-N⁴-(4-(8-methoxycoumarin-3-yl)thiazol-2-yl)succinamide (Compound D9, 29%, 23 mg) |
| 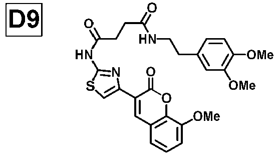 | ¹H NMR (400 MHz, DMSO) δ 12.31 (s, 1H), 8.53 (s, 1H), 7.98 (s, 2H), 7.40 - 7.33 (m, 1H), 7.33 - 7.29 (m, 1H), 7.26 - 7.14 (m, 1H), 6.87 - 6.79 (m, 2H), 6.71 (dd, J = 8.2, 1.9 Hz, 1H), 3.93 (s, 3H), 3.75 (s, 3H), 3.71 (s, 3H), 3.25 (q, J = 6.8 Hz, 2H), 2.70 (t, J = 6.9 Hz, 2H), 2.64 (t, J = 7.3 Hz, 2H), 2.46 (t, J = 6.9 Hz, 2H). |
| Example 49 | N¹-(3,4-dimethoxyphenethyl)-N⁴-(4-(6-methoxycoumarin-3-yl)thiazol-2-yl)succinamide (Compound D10, 55%, 20 mg) |
| 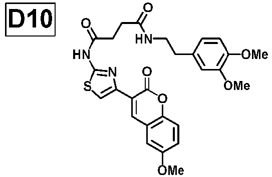 | ¹H NMR (400 MHz, DMSO) δ 12.27 (s, 1H), 8.53 (s, 1H), 7.97 (s, 2H), 7.39 (d, J = 9.0 Hz, 1H), 7.34 (d, J = 3.0 Hz, 1H), 7.22 (dd, J = 9.0, 3.0 Hz, 1H), 6.87 - 6.78 (m, 2H), 6.75 - 6.67 (m, 1H), 3.83 (s, 3H), 3.75 (s, 3H), 3.71 (s, 3H), 3.25 (q, J = 6.8 Hz, 2H), 2.77 - 2.68 (m, 2H), 2.64 (t, J = 7.3 Hz, 2H), 2.49 - 2.42 (m, 2H). |
| Example 50 | N¹-(3,4-dimethoxyphenethyl)-N⁴-(4-(7-N,N-dimethylaminocoumarin-3-yl)thiazol-2-yl)succinamide (Compound D11, 23%, 19 mg) |
| 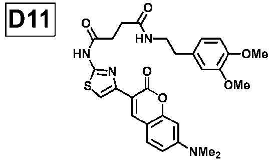 | ¹H NMR (400 MHz, DMSO) δ 12.24 (s, 1H), 8.45 (s, 1H), 7.98 (t, J = 5.7 Hz, 1H), 7.78 (s, 1H), 7.58 (d, J = 8.9 Hz, 1H), 6.90 - 6.74 (m, 3H), 6.71 (dd, J = 8.1, 2.0 Hz, 1H), 6.63 (d, J = 2.4 Hz, 1H), 3.75 (s, 3H), 3.71 (s, 3H), 3.24 (q, J = 6.7 Hz, 2H), 3.06 (s, 6H), 2.69 (t, J = 6.9 Hz, 2H), 2.63 (t, J = 7.3 Hz, 2H), 2.45 (t, J = 7.0 Hz, 2H). |
| Example 51 | N¹-(3,4-dimethoxyphenethyl)-N⁴-(4-(5-methoxycoumarin-3-yl)thiazol-2-yl)succinamide (Compound D12, 60%, 18 mg) |
| 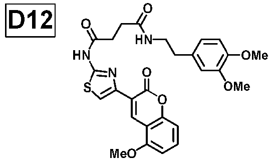 | ¹H NMR (400 MHz, DMSO) δ 12.36 (s, 1H), 8.85 (s, 1H), 8.44 (s, 1H), 8.04 - 7.90 (m, 1H), 7.67 - 7.50 (m, 1H), 7.10 - 6.93 (m, 2H), 6.89 - 6.79 (m, 2H), 6.71 (dd, J = 8.1, 2.0 Hz, 1H), 3.98 (s, 3H), 3.75 (s, 3H), 3.71 (s, 3H), 3.24 (q, J = 6.6 Hz, 1H), 2.69 (t, J = 7.0 Hz, 1H), 2.63 (t, J = 7.3 Hz, 1H), 2.45 (t, J = 7.0 Hz, 1H). |
| Example 52 | N¹-(3,4-dimethoxyphenethyl)-N⁴-(4-(7-hydroxycoumarin-3-yl)thiazol-2-yl)succinamide (Compound D13, 63%, 58 mg) |
| 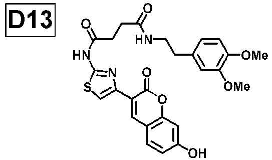 | ¹H NMR (400 MHz, DMSO) δ 12.27 (s, 1H), 10.69 (s, 1H), 8.51 (s, 1H), 7.98 (t, J = 5.5 Hz, 1H), 7.85 (s, 1H), 7.67 (d, J = 8.6 Hz, 1H), 6.85 (dd, J = 8.4, 2.2 Hz, 2H), 6.80 (dd, J = 7.2, 2.1 Hz, 2H), 6.71 (dd, J = 8.1, 2.0 Hz, 1H), 3.75 (s, 3H), 3.71 (s, 3H), 3.28 - 3.20 (m, 2H), 2.69 (t, J = 6.9 Hz, 2H), 2.63 (t, J = 7.3 Hz, 2H), 2.45 (t, J = 7.0 Hz, 2H). |
| Example 53 | N¹-(4-pyridylethyl)-N⁴-(5-benzoylthiazol-2-yl)succinamide (Compound D14, 24%, 10 mg) |
| 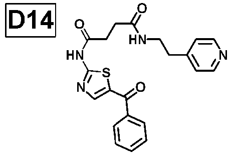 | ¹H NMR (600 MHz, DMSO) δ 12.50 (s, 1H), 8.27 (d, J = 5.5 Hz, 2H), 7.90 (s, 1H), 7.87 (t, J = 5.8 Hz, 1H), 7.69 - 7.62 (m, 2H), 7.49 (t, J = 7.5 Hz, 1H), 7.39 (t, J = 7.6 Hz, 2H), 7.05 (d, J = 5.7 Hz, 2H), 3.13 (q, J = 6.7 Hz, 2H), 2.54 (td, J = 7.1, 3.0 Hz, 4H), 2.28 (t, J = 6.9 Hz, 2H). |
| Example 54 | N¹-(3,4-dimethoxyphenethyl)-N⁴-(5-benzoylthiazol-2-yl)succinamide (Compound D15, 17%, 8 mg) |
| 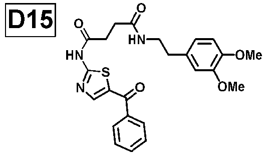 | ¹H NMR (600 MHz, DMSO) δ 12.50 (s, 1H), 7.90 (s, 1H), 7.81 (t, J = 5.6 Hz, 1H), 7.69 - 7.63 (m, 2H), 7.51 - 7.46 (m, 1H), 7.42 - 7.34 (m, 2H), 6.66 (d, J = 8.2 Hz, 1H), 6.62 (d, J = 2.0 Hz, 1H), 6.52 (dd, J = 8.2, 2.0 Hz, 1H), 3.56 (s, 3H), 3.52 (s, 3H), 3.10 - 3.03 (m, 2H), 2.55 (t, J = 7.0 Hz, 2H), 2.45 (t, J = 7.4 Hz, 2H), 2.30 (t, J = 6.9 Hz, 1H). |
| Example 55 | N¹-(4-pyridylethyl)-N⁴-(4-(7-chlorocoumarin-3-yl)thiazol-2-yl)succinamide (Compound D16, 91%, 22 mg) |
| 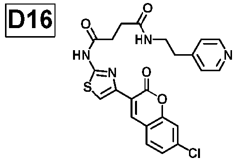 | ¹H NMR (600 MHz, DMSO) δ 12.13 (s, 1H), 8.38 (s, 1H), 8.30 - 8.24 (m, 2H), 7.85 (t, J = 5.7 Hz, 1H), 7.81 (s, 1H), 7.71 (d, J = 8.4 Hz, 1H), 7.48 (d, J = 2.0 Hz, 1H), 7.29 (d, J = 8.3 Hz, 1H), 7.06 (d, J = 6.0 Hz, 2H), 3.13 (q, J = 5.2 Hz, 2H), 2.55 (t, J = 7.1 Hz, 2H), 2.51 (t, J = 7.0 Hz, 2H), 2.25 (t, J = 7.0 Hz, 2H). |
| Example 56 | N¹-(4-pyridylethyl)-N⁴-(4-(7-bromocoumarin-3-yl)thiazol-2-yl)succinamide (Compound D17, 83%, 22 mg) |
| 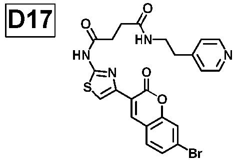 | ¹H NMR (600 MHz, DMSO) δ 12.13 (s, 1H), 8.36 (s, 1H), 8.30 - 8.25 (m, 2H), 7.85 (t, J = 5.7 Hz, 1H), 7.82 (s, 1H), 7.67 - 7.58 (m, 2H), 7.41 (dd, J = 8.3, 1.9 Hz, 1H), 7.06 (d, J = 6.0 Hz, 2H), 3.15 - 3.09 (m, 2H), 2.55 (t, J = 7.1 Hz, 2H), 2.51 (t, J = 7.0 Hz, 2H), 2.25 (t, J = 7.0 Hz, 2H). |
| Example 57 | N'-(4-pyridylethyl)-N'-(4-(7-methylcoumarin-3-yl)thiazol-2-yl)succinamide (Compound D18, 95%, 22 mg) |
| 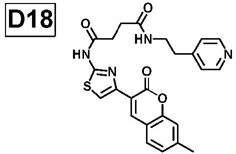 | ¹H NMR (600 MHz, DMSO) δ 12.20 (s, 1H), 8.53 (s, 1H), 8.50 - 8.39 (m, 2H), 8.03 (t, J = 5.7 Hz, 1H), 7.94 (s, 1H), 7.71 (d, J = 7.9 Hz, 1H), 7.34 - 7.27 (m, 1H), 7.27 - 7.15 (m, 3H), 3.33 - 3.22 (m, 2H), 2.73 (t, J = 7.1 Hz, 2H), 2.68 (t, J = 7.0 Hz, 2H), 2.48 - 2.36 (m, 5H). |
| Example 58 | N¹-(4-pyridylethyl)-N⁴-(4-(coumarin-3-yl)thiazol-2-yl)succinamide (D19, 98%, 22 mg) |
| 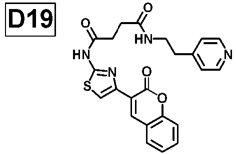 | ¹H NMR (600 MHz, DMSO) δ 11.86 (s, 1H), 8.40 (s, 1H), 8.27 (d, J = 5.0 Hz, 2H), 7.85 (t, J = 5.8 Hz, 1H), 7.81 (s, 1H), 7.67 (d, J = 7.7 Hz, 1H), 7.47 (t, J = 7.8 Hz, 1H), 7.29 (d, J = 8.2 Hz, 1H), 7.22 (t, J = 7.5 Hz, 1H), 7.06 (d, J = 4.9 Hz, 2H), 3.15 - 3.07 (m, 2H), 2.55 (t, J = 7.0 Hz, 2H), 2.51 (t, J = 7.0 Hz, 2H), 2.25 (t, J = 7.1 Hz, 2H). |
| Example 59 | N'-(4-pyridylethyl)-N'-(4-(8-methoxycoumarin-3-yl)thiazol-2-yl)succinamide (Compound D20, 84%, 20 mg) |
| 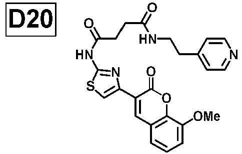 | ¹H NMR (600 MHz, DMSO) δ 12.13 (s, 1H), 8.36 (s, 1H), 8.27 (d, J = 6.0 Hz, 2H), 7.85 (t, J = 5.7 Hz, 1H), 7.81 (d, J = 1.8 Hz, 1H), 7.22 - 7.17 (m, 1H), 7.16 - 7.10 (m, 1H), 7.09 - 7.03 (m, 2H), 7.01 (t, J = 7.8 Hz, 1H), 3.75 (s, 3H), 3.16 - 3.08 (m, 2H), 2.55 (t, J = 7.1 Hz, 2H), 2.51 (t, J = 7.0 Hz, 2H), 2.25 (t, J = 7.0 Hz, 2H). |
| Example 60 | N'-(4-pyridylethyl)-N'-(4-(5-methoxycoumarin-3-yl)thiazol-2-yl)succinamide (Compound D21, 31%, 15 mg) |
| 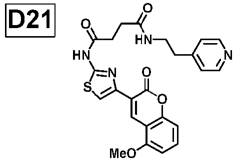 | 1H NMR (400 MHz, DMSO) δ 12.36 (s, 1H), 8.84 (s, 1H), 8.49 (d, J = 5.0 Hz, 2H), 8.05 (t, J = 5.7 Hz, 1H), 7.95 (s, 1H), 7.62 - 7.55 (m, 1H), 7.30 (d, J = 5.0 Hz, 2H), 7.10 - 6.95 (m, 2H), 3.97 (s, 3H), 3.33 (m, 2H), 2.75 (t, J = 7.0 Hz, 2H), 2.68 (t, J = 7.1 Hz, 2H), 2.43 (t, J = 7.0 Hz, 2H). |
| Example 61 | N'-(4-pyridylethyl)-N'-(4-(7-hydroxycoumarin-3-yl)thiazol-2-yl)succinamide (Compound D22, 22%, 5 mg) |
| 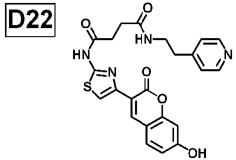 | ¹H NMR (400 MHz, DMSO) δ 12.29 (s, 1H), 10.72 (s, 1H), 8.50 (s, 1H), 8.46 (d, J = 5.1 Hz, 2H), 8.05 (t, J = 5.6 Hz, 1H), 7.85 (s, 1H), 7.67 (d, J = 8.5 Hz, 1H), 7.25 (d, J = 5.0 Hz, 2H), 6.85 (dd, J = 8.7, 2.2 Hz, 1H), 6.79 (d, J = 2.2 Hz, 1H), 3.31 (q, J = 6.6 Hz, 2H), 2.73 (t, J = 7.0 Hz, 2H), 2.68 (t, J = 7.0 Hz, 2H), 2.43 (t, J = 6.9 Hz, 2H). |
| Example 62 | N'-(4-pyridylethyl)-N'-(4-(6-methoxycoumarin-3-yl)thiazol-2-yl)succinamide (Compound D23, 17%, 8 mg) |
| 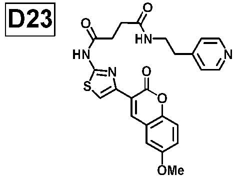 | ¹H NMR (400 MHz, DMSO) δ 12.36 (s, 1H), 8.84 (s, 1H), 8.49 (d, J = 5.0 Hz, 2H), 8.05 (t, J = 5.7 Hz, 1H), 7.95 (s, 1H), 7.62 - 7.55 (m, 1H), 7.30 (d, J = 5.0 Hz, 2H), 7.10 - 6.95 (m, 2H), 3.97 (s, 3H), 3.33 (m, 2H), 2.75 (t, J = 7.0 Hz, 2H), 2.68 (t, J = 7.1 Hz, 2H), 2.43 (t, J = 7.0 Hz, 2H). |
| Example 63 | N'-(4-pyridylethyl)-N'-(4-(8-hydroxycoumarin-3-yl)thiazol-2-yl)succinamide (Compound D24, 15%, 7 mg) |
| 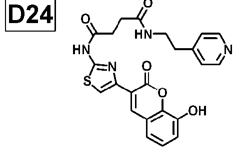 | ¹H NMR (400 MHz, DMSO) δ 12.34 (s, 1H), 10.34 (s, 1H), 8.54 (s, 1H), 8.46 (d, J = 5.0 Hz, 2H), 8.06 (t, J = 5.6 Hz, 1H), 8.00 (s, 1H), 7.28 - 7.17 (m, 4H), 7.13 (dd, J = 7.9, 1.7 Hz, 1H), 3.34 - 3.21 (m, 2H), 2.71 (dt, J = 16.9, 7.0 Hz, 4H), 2.43 (dd, J = 12.3, 5.2 Hz, 2H). |
| Example 64 | N'-(4-pyridylethyl)-N'-(4-(6-hydroxycoumarin-3-yl)thiazol-2-yl)succinamide (Compound D25, 11%, 5 mg) |
| 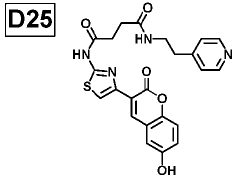 | ¹H NMR (400 MHz, DMSO) δ 12.31 (br s, 1H), 9.81 (br s, 1H), 8.49 (s, 1H), 8.47 - 8.40 (m, 2H), 8.05 (s, 1H), 7.98 (d, J = 1.4 Hz, 1H), 7.31 (d, J = 8.8 Hz, 1H), 7.28 - 7.19 (m, 2H), 7.14 (d, J = 2.9 Hz, 1H), 7.08 (dd, J = 8.9, 2.8 Hz, 1H), 3.34 - 3.11 (m, 2H), 2.71 (dt, J = 17.3, 7.0 Hz, 4H), 2.47 - 2.40 (m, 2H). |
| Example 65 | N¹-(4-pyridylethyl)-N⁴-(4-(2,4-difluorophenyl)thiazol-2-yl)succinamide (Compound D26, 23%, 19 mg) |
| 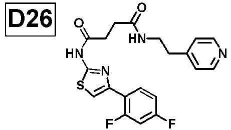 | ¹H NMR (400 MHz, DMSO) δ 12.32 (s, 1H), 8.46 (d, J = 5.3 Hz, 2H), 8.16 - 7.99 (m, 2H), 7.48 (d, J = 2.5 Hz, 1H), 7.45 - 7.32 (m, 1H), 7.28 - 7.14 (m, 3H), 3.31 (q, J = 6.7 Hz, 2H), 2.70 (dt, J = 17.6, 7.0 Hz, 4H), 2.43 (t, J = 6.9 Hz, 2H). |
| Example 66 | N¹-(4-pyridylethyl)-N⁴-(4-(3,4-difluorophenyl)thiazol-2-yl)succinamide (Compound D27, 26%, 22 mg) |
| 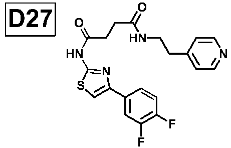 | ¹H NMR (400 MHz, DMSO) δ 12.31 (s, 1H), 8.45 (d, J = 5.5 Hz, 2H), 8.05 (t, J = 5.7 Hz, 1H), 7.94 - 7.86 (m, 1H), 7.79 - 7.72 (m, 1H), 7.70 (s, 1H), 7.59 - 7.44 (m, 2H), 7.24 (d, J = 5.6 Hz, 2H), 3.31 (q, J = 6.7 Hz, 2H), 2.70 (dt, J = 17.8, 7.0 Hz, 4H), 2.43 (t, J = 7.0 Hz, 2H). |
| Example 67 | N¹-(4-pyridylethyl)-N⁴-(4-(p-tolyl)thiazol-2-yl)succinamide (Compound D28, 39%, 31 mg) |
| 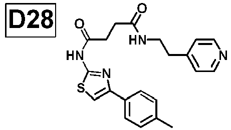 | ¹H NMR (400 MHz, DMSO) δ 12.26 (s, 1H), 8.49 - 8.44 (m, 2H), 8.05 (t, J = 5.6 Hz, 1H), 7.79 (d, J = 7.9 Hz, 2H), 7.52 (s, 1H), 7.30 - 7.12 (m, 4H), 3.31 (q, J = 6.7 Hz, 2H), 2.73 (t, J = 7.0 Hz, 2H), 2.67 (t, J = 7.0 Hz, 2H), 2.43 (t, J = 6.9 Hz, 2H), 2.32 (s, 3H). |
| Example 68 | N¹-(4-pyridylethyl)-N⁴-(4-(5,6,7,8-tetrahydro-2-naphthyl)thiazol-2-yl)succinamide (Compound D29, 69%, 30 mg) |
| 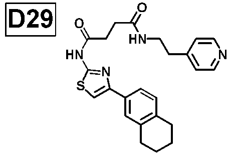 | ¹H NMR (400 MHz, DMSO) δ 12.25 (s, 1H), 8.46 (d, J = 6.0 Hz, 1H), 8.04 (t, J = 5.7 Hz, 1H), 7.62 - 7.55 (m, 2H), 7.48 (s, 1H), 7.27 - 7.20 (m, 2H), 7.09 (d, J = 7.8 Hz, 1H), 3.31 (q, J = 6.7 Hz, 2H), 2.80 - 2.71 (m, 6H), 2.71 - 2.59 (m, 2H), 2.43 (t, J = 6.9 Hz, 2H), 1.80 - 1.69 (m, 4H). |
| Example 69 | N¹-(4-pyridylethyl)-N⁴-(4-(7-methoxycoumarin-3-yl)oxazol-2-yl)succinamide (Compound D30, 84%, 39 mg) |
| 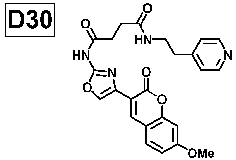 | ¹H NMR (400 MHz, DMSO) δ 11.24 (br s, 1H), 8.46 (d, J = 6.0 Hz, 2H), 8.37 (s, 1H), 8.26 (s, 1H), 8.04 (t, J = 5.6 Hz, 1H), 7.83 (d, J = 8.7 Hz, 1H), 7.24 (d, J = 5.9 Hz, 2H), 7.09 (d, J = 2.4 Hz, 1H), 7.01 (dd, J = 8.7, 2.4 Hz, 1H), 3.88 (s, 3H), 3.31 (q, J = 7.3 Hz, 2H), 2.73 (t, J = 7.1 Hz, 2H), 2.65 (br s, 2H), 2.40 (t, J = 6.9 Hz, 2H). |
| Example 70 | N¹-(3,4-dimethoxyphenethyl)-N⁴-(4-(7-methoxycoumarin-3-yl)oxazol-2-yl)succinamide (Compound D31, 59%, 31 mg) |
| 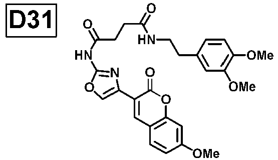 | ¹H NMR (400 MHz, DMSO) δ 11.26 (s, 1H), 8.37 (s, 1H), 8.25 (s, 1H), 7.98 (t, J = 5.6 Hz, 1H), 7.82 (d, J = 8.7 Hz, 1H), 7.09 (d, J = 2.4 Hz, 1H), 7.01 (dd, J = 8.7, 2.4 Hz, 1H), 6.89 - 6.78 (m, 2H), 6.71 (dd, J = 8.1, 2.0 Hz, 1H), 3.88 (s, 3H), 3.74 (s, 3H), 3.71 (s, 3H), 3.30 - 3.16 (m, 2H), 2.70 - 2.59 (m, 4H), 2.41 (t, J = 6.9 Hz, 2H). |
| Example 71 | N¹-(4-pyridylethyl)-N⁴-(4-([1,1'-biphenyl]-4-yl)thiazol-2-yl)succinamide (Compound D32, 66%, 30 mg) |
| 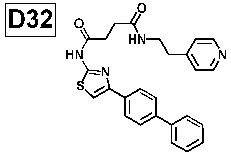 | ¹H NMR (400 MHz, DMSO) δ 12.20 (s, 1H), 8.46 (d, J = 5.9 Hz, 2H), 8.03 (t, J = 5.7 Hz, 1H), 8.02 - 7.97 (m, 2H), 7.74 (dd, J = 10.5, 8.0 Hz, 4H), 7.67 (s, 1H), 7.49 (t, J = 7.6 Hz, 2H), 7.38 (t, J = 7.4 Hz, 1H), 7.27 - 7.20 (m, 2H), 3.34 - 3.28 (m, 2H), 2.73 (t, J = 7.1 Hz, 2H), 2.69 (t, J = 7.0 Hz, 2H), 2.44 (t, J = 7.0 Hz, 2H). |
| Example 72 | N¹-(4-pyridylethyl)-N⁴-(4-(4-hydroxy-6-methyl-2-oxo-2H-pyran-3-yl)thiazol-2-yl)succinamide (Compound D33, 97%, 27 mg) |
| 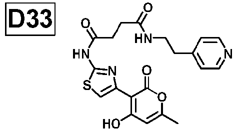 | ¹H NMR (400 MHz, DMSO) δ 14.29 (s, 1H), 12.47 (s, 1H), 8.44 (d, J = 5.0 Hz, 2H), 8.05 (t, J = 5.7 Hz, 1H), 7.69 (s, 1H), 7.33 - 7.11 (m, 2H), 6.24 (s, 1H), 3.33 - 3.24 (m, 2H), 2.72 (t, J = 7.0 Hz, 2H), 2.67 (t, J = 6.8 Hz, 2H), 2.45 (t, J = 6.9 Hz, 2H). |
| Example 73 | N¹-(3,4-dimethoxyphenethyl)-N⁴-(4-(4-hydroxy-6-methyl-2-oxo-2H-pyran-3-yl)thiazol-2-yl)succinamide (Compound D34, 88%, 28 mg) |
| 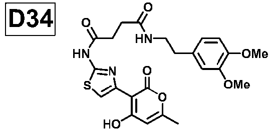 | ¹H NMR (400 MHz, DMSO) δ 14.30 (s, 1H), 12.47 (s, 1H), 8.00 (t, J = 5.6 Hz, 1H), 7.68 (s, 1H), 6.91 - 6.76 (m, 2H), 6.69 (dd, J = 8.1, 2.0 Hz, 1H), 6.24 (s, 1H), 3.74 (s, 3H), 3.70 (s, 3H), 3.23 (q, J = 6.8 Hz, 2H), 2.68 (t, J = 6.8 Hz, 2H), 2.62 (t, J = 7.3 Hz, 2H), 2.50 - 2.36 (m, 2H), 2.24 (s, 3H). |
| Example 74 | N¹-(4-((5,6-dimethoxy-1-oxo-2,3-dihydro-1H-indene-2-yl)methyl)piperidin-1-yl)-N⁴-(4-(7-methoxycoumarin-3-yl)thiazol-2-yl)succinamide (Compound C39, 56%, 36 mg) |
| 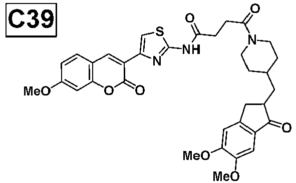 | ¹H NMR (400 MHz, DMSO) δ 12.29 (s, 1H), 8.55 (s, 1H), 7.88 (s, 1H), 7.77 (d, J = 8.7 Hz, 1H), 7.11 - 7.08 (m, 2H), 7.06 (s, 1H), 7.00 (dd, J = 8.7, 2.5 Hz, 1H), 4.41 - 4.29 (m, 1H),3.97 - 3.89 (m, 1H), 3.88 (s, 3H), 3.86 (s, 3H), 3.79 (s, 3H), 3.25 (ddt, J = 12.3, 8.1, 4.6 Hz, 1H), 3.06 - 2.94 (m, 2H), 2.81 (s, 1H), 2.73 - 2.63 (m, 6H), 1.88 - 1.60 (m, 4H), 1.34 - 1.21 (m, 1H), 1.04 - 0.84 (m, 1H). |
| Example 75 | N¹-(4-(7-methoxycoumarin-3-yl)thiazol-2-yl)-N⁴-(1-(pyridin-4-yl)piperidin-4-yl)succinamide (Compound C40, 34%, 18 mg) |
| 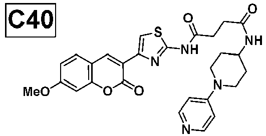 | ¹H NMR (400 MHz, DMSO) δ 12.29 (s, 1H), 8.55 (s, 1H), 8.13 (br s, 2H), 7.89 (br s, 2H), 7.78 (d, J = 8.6 Hz, 1H), 7.10 (d, J = 2.6 Hz, 1H), 7.01 (dd, J = 8.5, 2.5 Hz, 1H), 6.83 (d, J = 6.0 Hz, 2H), 3.87 (s, 3H), 3.86 - 3.64 (m, 4H), 3.07 - 2.85 (m, 2H), 2.82 - 2.63 (m, 3H), 1.84 - 1.71 (m, 2H), 1 1.55 - 1.27 (m, 2H). |
| Example 76 | N¹-(4-(7-methoxycoumarin-3-yl)thiazol-2-yl)-N⁴-(4-(pyridin-4-yl)piperazin-1-yl)succinimide (Compound C41, 98%, 51 mg) |
| 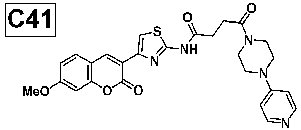 | ¹H NMR (400 MHz, DMSO) δ 8.56 (s, 1H), 8.18 (d, J = 5.9 Hz, 2H), 7.89 (s, 1H), 7.78 (d, J = 8.7 Hz, 1H), 7.10 (d, J = 2.4 Hz, 1H), 7.01 (dd, J = 8.7, 2.4 Hz, 1H), 6.84 (d, J = 6.1 Hz, 2H), 3.89 (s, 3H), 3.73 (d, J = 11.0 Hz, 2H), 3.63 (s, 2H), 3.46 - 3.36 (m, 4H), 2.82 - 2.67 (m, 4H). |
| Example 77 | N¹-(4-(7-methoxycoumarin-3-yl)thiazol-2-yl)-N⁴-(4-(pyridin-4-yl)piperidin-1-yl)succinimide (Compound C42, 96%, 50 mg) |
| 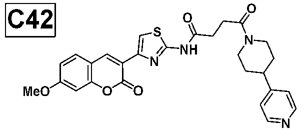 | ¹H NMR (400 MHz, DMSO) δ 8.56 (s, 1H), 8.48 (d, J = 6.0 Hz, 2H), 7.90 (s, 1H), 7.78 (d, J = 8.7 Hz, 1H), 7.36 - 7.24 (m, 2H), 7.10 (d, J = 2.4 Hz, 1H), 7.01 (dd, J = 8.6, 2.4 Hz, 1H), 4.52 (d, J = 13.0 Hz, 1H), 4.05 (d, J = 13.7 Hz, 1H), 3.89 (s, 3H), 3.14 (t, J = 12.6 Hz, 1H), 2.82 (t, J = 12.1 Hz, 1H), 2.79 - 2.58 (m, 5H), 1.89 - 1.73 (m, 2H), 1.72 - 1.57 (m, 1H), 1.54 - 1.40 (m, 1H). |
| Example 78 | N¹-(4-(2,3,4-trimethoxybenzyl)piperazin-1-yl)-N⁴-(4-(7-methoxycoumarin-3-yl)thiazol-2-yl)succinamide (Compound C43, 96%, 60 mg) |
| 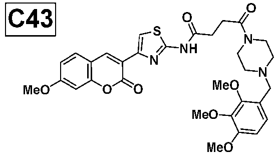 | ¹H NMR (400 MHz, DMSO) δ 12.30 (br s, 1H), 8.54 (s, 1H), 7.88 (s, 1H), 7.77 (d, J = 8.7 Hz, 1H), 7.09 (s, 1H), 7.04 - 6.93 (m, 2H), 6.86 - 6.67 (m, 1H), 3.88 (s, 3H), 3.78 (br s, 6H), 3.74 (s, 3H), 3.49 - 3.36 (m, 6H), 2.67 (s, 4H), 2.38 (s, 2H), 2.34 - 2.23 (m, 2H). |
| Example 79 | N¹-(4-(7-methoxycoumarin-3-yl)thiazol-2-yl)-N⁴-(2-(quinolin-4-carbonyl)hydrazinyl)succinimide (Compound C44, 98%, 53 mg) |
| 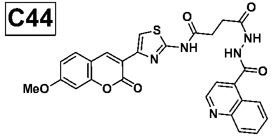 | 1H NMR (400 MHz, DMSO) δ 9.00 (d, J = 4.3 Hz, 1H), 8.55 (s, 1H), 8.32 (d, J = 8.4 Hz, 1H), 8.10 (d, J = 8.3 Hz, 1H), 7.90 (s, 1H), 7.84 (ddd, J = 8.4, 6.9, 1.5 Hz, 1H), 7.78 (d, J = 8.7 Hz, 1H), 7.70 (ddd, J = 8.3, 6.9, 1.3 Hz, 1H), 7.57 (d, J = 4.3 Hz, 1H), 7.09 (d, J = 2.4 Hz, 1H), 7.01 (dd, J = 8.7, 2.4 Hz, 1H), 3.89 (s, 3H), 2.82 (t, J = 6.9 Hz, 2H), 2.66 (t, J = 6.9 Hz, 2H). |
| Example 80 | N¹-(4-(7-methoxycoumarin-3-yl)thiazol-2-yl)-N⁴-(2-(1H-indol-6-carbonyl)hydrazinyl)succinimide (Compound C45, 98%, 52 mg) |
| 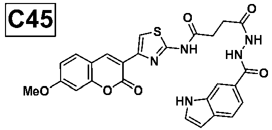 | ¹H NMR (400 MHz, DMSO) δ 11.46 (s, 1H), 11.38 (s, 1H), 9.65 (s, 1H), 8.55 (s, 1H), 7.99 (s, 1H), 7.91 (d, J = 7.5 Hz, 2H), 7.77 (d, J = 8.7 Hz, 1H), 7.62 - 7.44 (m, 3H), 7.09 (s, 1H), 7.01 (d, J = 2.4 Hz, 1H), 6.48 (d, J = 8.2 Hz, 1H), 3.89 (s, 3H), 2.81 - 2.74 (m, 2H), 2.65 - 2.57 (m, 2H). |
| Example 81 | N¹-(4-(7-methoxycoumarin-3-yl)thiazol-2-yl)-N⁴-(5-(pyridin-4-yl)-1,3,4-thiadiazol-2-yl)succinamide (Compound C46, 26%, 14 mg) |
| 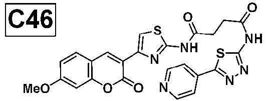 | ¹H NMR (400 MHz, DMSO) δ 12.98 (s, 1H), 12.44 (s, 1H), 8.80 - 8.67 (m, 2H), 8.55 (s, 1H), 8.00 - 7.82 (m, 4H), 7.10 (s, 1H), 7.01 (dd, J = 8.6, 2.5 Hz, 1H), 3.89 (s, 3H), 2.98 - 2.82 (m, 4H). |

### Experimental Example 1: Evaluation of SOX9 Transcriptional Activity

To confirm whether the compounds would enhance the SOX9 transcriptional activity, a luciferase assay capable of measuring SOX9 transcriptional activity was carried out using the following experimental method.

Mouse primary chondrocytes were extracted from mouse cartilage. The extracted cells were stabilized in a low-oxygen-environment incubator (O₂ 3%, CO₂ 5%). Here, the medium used was DMEM (Dulbecco Modified Eagle Medium, LM001-05, Welgene) supplemented with 10% FBS (fetal bovine serum, Gibco).

After cell stabilization, hyaluronidase type I-S (Sigma-Aldrich, Hase) was treated for 4 hours for transfection of the luciferase plasmid.

For transfection, Metafectene pro reagent (Biontex) was used to co-treat cells for 6 hours with the 4X48pCol2al-Luciferase plasmid, the expression of which is promoted by SOX9 transcriptional activity, and a Renilla luciferase plasmid, which is constitutively expressed as a transfection control. The 4X48pCol2a1-Luciferase plasmid and Renilla luciferase plasmid were as described in Kim, S. et al. "Tankyrase inhibition preserves osteoarthritic cartilage by coordinating cartilage matrix anabolism via effects on SOX9 PARylation," Nat Commun 10, 4898 (2019).

After 6 hours, the medium was replaced with a culture medium containing the compound. Here, each compound was dissolved in DMSO at a level of about 5 mM to 10 mM and dissolved in the medium to provide a final concentration of 7.5 mM.

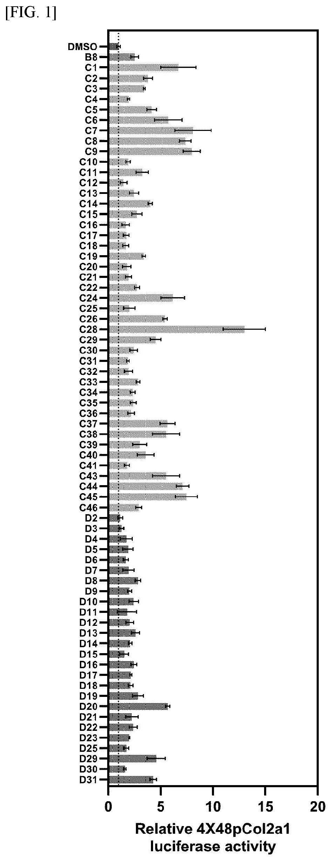

After 48 hours, the cells were lysed and reacted with the luciferase substrate using a Dual Luciferase Assay Kit (Promega), and luminescence from the luciferase (relative to DMSO) was measured using a microplate reader. The results were classified according to the criteria below and shown in Table 6, providing the graph of FIG. 1.

++++: 8 or more / +++: less than 8, 5 or more / ++: less than 5, 2 or more / +: less than 2, greater than 1

**[Table 6]**

| **Example** | **Comp'd No.** | **SOX9 activity** | **Example** | **Comp'd No.** | **SOX9 activity** | **Example** | **Comp'd No.** | **SOX9 activity** |
|---|---|---|---|---|---|---|---|---|
| 1 | B8 | ++ | 27 | C26 | +++ | 53 | D14 | ++ |
| 2 | C1 | ++++ | 28 | C27 | + | 54 | D15 | ++ |
| 3 | C2 | ++ | 29 | C28 | ++++ | 55 | D16 | ++ |
| 4 | C3 | ++ | 30 | C29 | +++ | 56 | D17 | ++ |
| 5 | C4 | ++ | 31 | C30 | ++ | 57 | D18 | ++ |
| 6 | C5 | +++ | 32 | C31 | ++ | 58 | D19 | ++ |
| 7 | C6 | ++++ | 33 | C32 | ++ | 59 | D20 | +++ |
| 8 | C7 | ++++ | 34 | C33 | ++ | 60 | D21 | ++ |
| 9 | C8 | ++++ | 35 | C34 | ++ | 61 | D22 | ++ |
| 10 | C9 | ++++ | 36 | C35 | ++ | 62 | D23 | ++ |
| 11 | C10 | ++ | 37 | C36 | ++ | 63 | D24 | ++ |
| 12 | C11 | ++ | 38 | C37 | +++ | 64 | D25 | ++ |
| 13 | C12 | ++ | 39 | C38 | ++++ | 65 | D26 | + |
| 14 | C13 | ++ | 40 | D1 | + | 68 | D29 | +++ |
| 15 | C14 | ++ | 41 | D2 | + | 69 | D30 | + |
| 16 | C15 | ++ | 42 | D3 | + | 70 | D31 | ++ |
| 17 | C16 | ++ | 43 | D4 | ++ | 71 | D32 | + |
| 18 | C17 | ++ | 44 | D5 | ++ | 74 | C43 | ++ |
| 19 | C18 | ++ | 45 | D6 | ++ | 75 | C39 | +++ |
| 20 | C19 | ++ | 46 | D7 | ++ | 76 | C40 | +++ |
| 21 | C20 | ++ | 47 | D8 | ++ | 77 | C41 | ++ |
| 22 | C21 | ++ | 48 | D9 | ++ | 79 | C44 | ++++ |
| 23 | C22 | ++ | 49 | D10 | ++ | 80 | C45 | ++++ |
| 24 | C23 | + | 50 | D11 | ++ | 81 | C46 | ++ |
| 25 | C24 | ++++ | 51 | D12 | ++ | | | |
| 26 | C25 | ++ | 52 | D13 | ++ | | | |

Referring to Table 6 and FIG. 1, it was confirmed that the compounds of the present invention have an excellent SOX9 transcriptional activity effect.

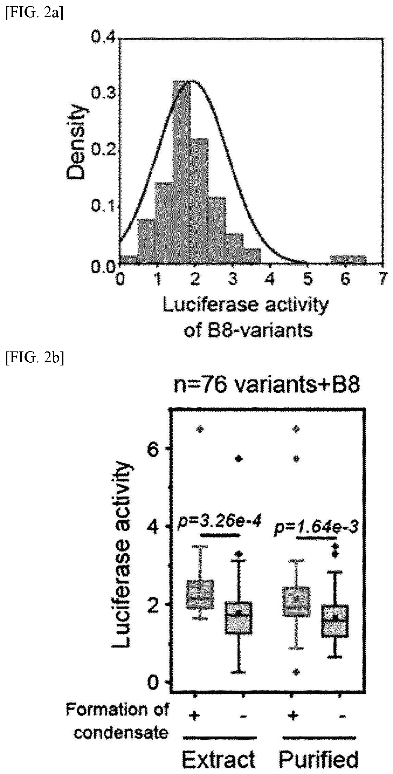

The compounds of the Examples showed different degrees of effect of increasing transcriptional activity (FIG. 2a). When changes in transcriptional activity according to the induction of condensation were examined, a statistically significant correlation was found between the condensation phenomenon and the transcriptional activity effect (FIG. 2b). This means that, at the SOX9 protein level, the condensationinducing capacity of a drug has an impact on, and is associated with, drug efficacy.

### Experimental Example 2: Induction of SOX9 Condensation and Evaluation of Transcriptional Activity

In a 100-mm dish, 9 × 10⁵ cells to be transfected were seeded and grown for two days in a cell incubator. The grown cells were transfected with 7 µg of an SOX9-eGFP plasmid capable of overexpression in cells, using a transfection reagent (Poly-jet). Here, the cells were exposed to an FBS-free DMEM medium for 30 minutes. After 30 minutes, the SOX9-eGFP plasmid and Polyjet were mixed in DMEM and added to the cells after waiting for 10 minutes. After reacting in the incubator for 4 hours, an FBS-containing DMEM medium was added, and the cells were placed in the incubator for two days. Thereafter, the cells were detached using a scraper and centrifuged. Only the cell pellets were frozen in liquid nitrogen and stored in a -80°C freezer.

A cell lysis buffer containing 0.2% Triton X-100, 1 mM EDTA, 50 mM HEPES, 150 mM NaCl, 10% glycerol, 1% protease inhibitor cocktail, 1% phosphatase inhibitor cocktail (tyrosine, serine/threonine), and 2 mM TCEP was added to the cell pellets to disperse the pellets. The dispersed cells were lysed using an ultrasonic homogenizer, and the lysate was centrifuged (15,000×g, 10 minutes) to obtain a supernatant. The total protein concentration of the supernatant obtained was measured using a DC (detergent-compatible) assay, and the SOX9-eGFP concentration was measured using a fluorometer. The cell lysate, with its concentration measured, was used immediately or frozen in liquid nitrogen and stored at -80°C.

Through the Halo-Tag purification method, SOX9, which is relatively difficult to purify in E. coli, was expressed and purified from HEK293T. Even when SOX9 was used (instead of the lysate), the correlation between the induction of condensation and the degree of transcriptional activity was reproduced, thereby confirming the validity of using the lysate (FIG. 2b).

The Example compounds were diluted to 1/100 of the concentration to be reacted, which were then transferred to tubes according to each reaction volume. The prepared cell lysate was added to the tubes containing Compound B8 or Compound C9 and reacted. The lysate reacted with the drug was transferred to a 96-well plate with cover-glass surfaces and reacted at room temperature for 1 hour until the reaction was complete. The 96-well plate, after the reaction was complete, was transferred to a confocal fluorescence microscope, and fluorescence imaging was performed.

The fluorescence images taken were analyzed using an in-house MATLAB code. K-mean clustering was used to discriminate between condensates and non-condensates, and the fluorescence intensity, area, shape, and number of the condensates were analyzed.

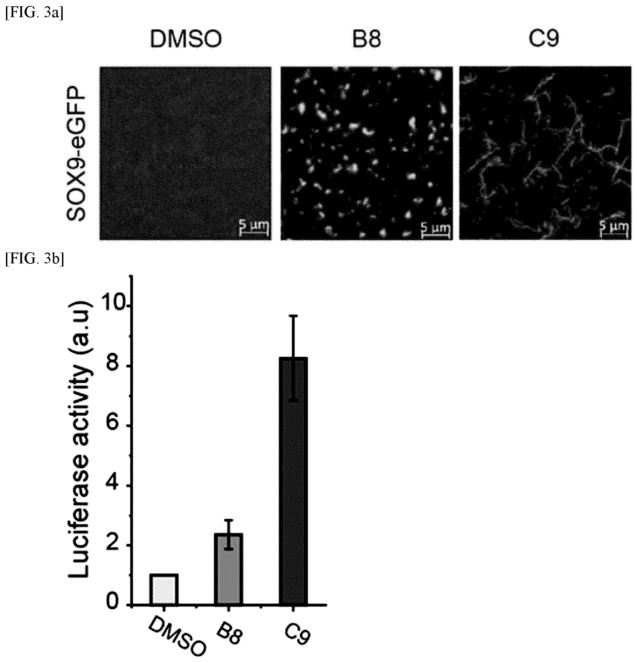

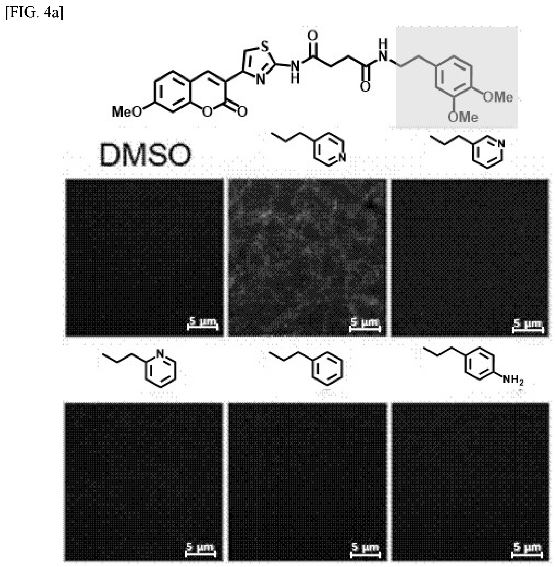

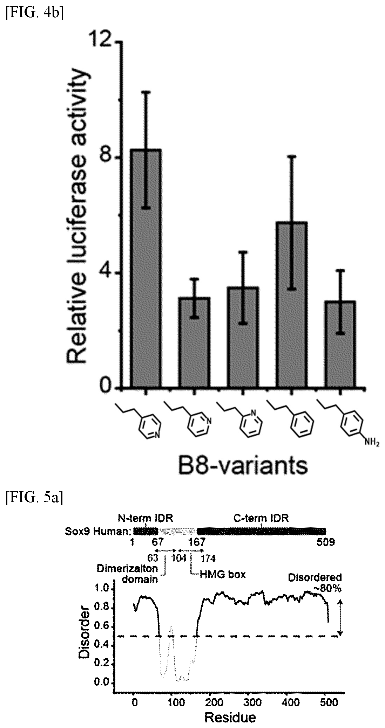

As a result, it was verified that, compared to DMSO, Compound B8 and Compound C9 induced the condensation of SOX9 (FIG. 3a). Together with this, it was confirmed, using a luciferase plasmid that responds specifically to the transcriptional activity of SOX9, that Compound B8 and Compound C9, which induce the condensation formation, increase the activity of the SOX9 transcription factor (FIG. 3b). In particular, Compound B8 increased SOX9 transcriptional activity by approximately 2-fold, whereas Compound C9 markedly enhanced the activity by about 8-fold. Here, due to a structural change in Compound C9, the SOX9 condensation formation was lost upon a single-atom change, such as a change in the position of a nitrogen atom or substitution, and it was confirmed that whether such condensation was induced affected the regulation of transcriptional activity (FIGS. 4a and 4b). This indicates that the induction of condensation and the regulation of transcriptional activity occur based on the chemical structure of the compound.

### Experimental Example 3: Evaluation of the Degree of Condensation According to the Deletion Region of SOX9

It was investigated by what mechanism Compound C9 contributes to enhancing the cartilage-regenerating effect.

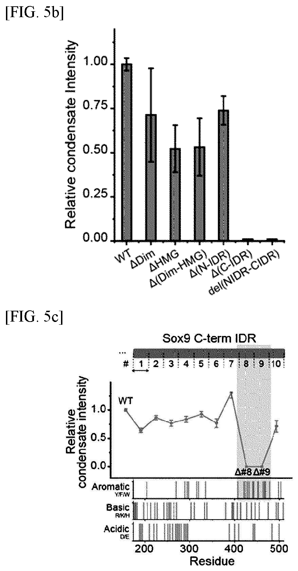

Specifically, based on Compound C9, which had been confirmed to be the most efficacious, the binding site of the drug was investigated. SOX9 is divided into structured and unstructured regions; in particular, IDR as an unstructured region is so large as to constitute about 80% of the protein (FIG. 5a). To investigate which region of interaction induces the condensation effect of Compound C9, SOX9 variants in which each region was deleted were prepared. As in the above Experimental Examples, cell lysates of the SOX9 variants were prepared, and it was confirmed that when C9 was deleted in the IDR region corresponding to the C-terminus of the protein, the condensation phenomenon was lost (FIG. 5b). Furthermore, in order to narrow down the binding site, deletion variants were constructed at 35-amino acid intervals across the C-terminal region, and cell lysates of the variants were prepared and examined. Here, when the regions corresponding to regions 8 and 9, where aromatic amino acids are clustered, were deleted, the condensation phenomenon was confirmed to decrease sharply (FIG. 5c).

### Experimental Example 4: Change in the Degree of SOX9 Condensation According to Mutation of Aromatic Amino Acids in Regions 8 and 9 of the SOX9 C-Terminus

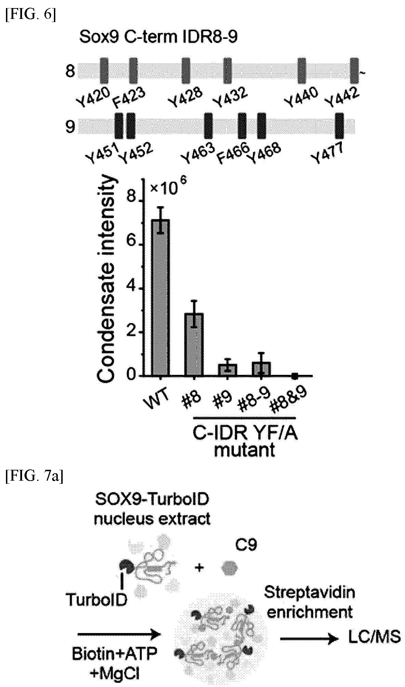

Cell lysates expressing mutants in which aromatic amino acids were substituted with alanine were prepared, and the importance of aromatic amino acids was confirmed. As in the case of the variants in which regions 8 and 9 were deleted, mutations of aromatic amino acids corresponding to regions 8 and 9 reduced the condensation phenomenon induced by Compound C9 (FIG. 6). It was confirmed through this that Compound C9 induces SOX9 condensation through interaction with the region where aromatic amino acids are clustered, in the unstructured region of the C-terminus of SOX9.

### Experimental Example 5: Identification of Proteins within Condensation-Induced SOX9 Condensates

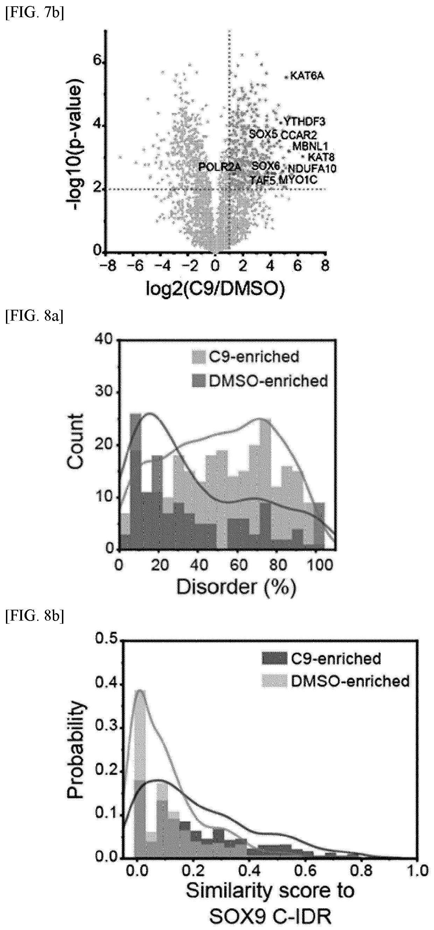

Since condensates are often formed by multiple proteins rather than by a single protein, to identify proteins that co-condense with SOX9 upon treatment with Compound C9, SOX9 was fused to TurboID, and proteins located within 10 nm of SOX9 upon treatment with Compound C9 were biotinylated. Biotinbound proteins were separated using Streptavidin-Bead, and liquid chromatography-mass spectrometry (LC/MS) was performed (FIG. 7a). Each mass spectrometry experiment was performed three times for each condition. Through mass spectrometry, in the C9-treated group compared to DMSO, proteins that were observed statistically significantly more frequently were identified as proteins involved in transcriptional regulation, such as Pol2, YTHDF3, CCAR2, MBNL1, NDUFA10, MYO1C, TAF5, and chromatin remodelers (KAT6A, KAT8). Proteins known to act together with SOX9, such as SOX5 and SOX6, were also detected (FIG. 7b).

### Experimental Example 6: Confirmation of Disorder of Proteins and Evaluation of Similarity to SOX9-C-IDR Through Spatial Frequency Analysis

The IDR regions of the proteins detected significantly in C9- and DMSO-treated samples were specified using sequence information through Metapredict. Here, it was confirmed that the proteins detected upon Compound C9 treatment, compared to DMSO, contained a large amount of IDRs (FIG. 8a). Since IDRs are known to play an important role in condensation, this indirectly suggests that Compound C9 induces SOX9 condensation.

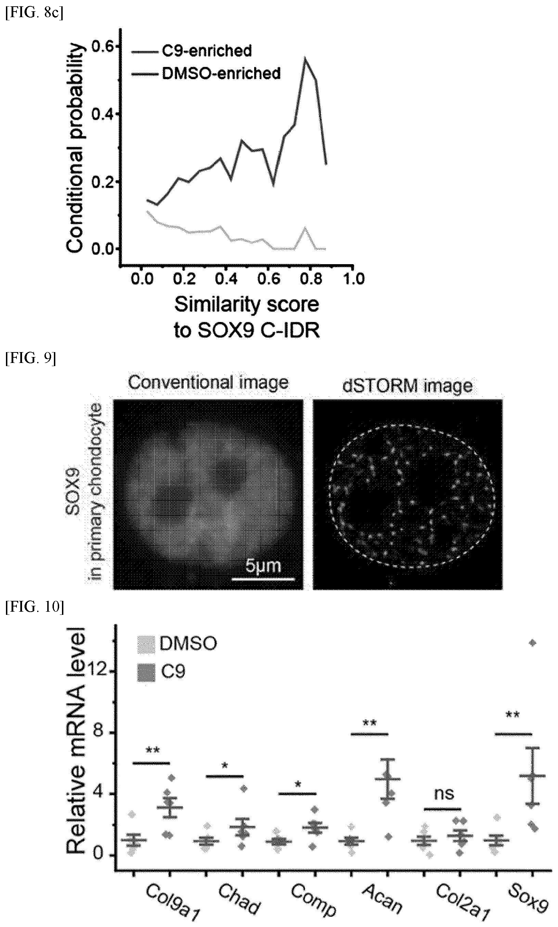

Because Compound C9 induced condensation by targeting the aromatic amino acids of the SOX9 IDR, it was confirmed, through mass spectrometry, whether the IDRs of the detected proteins contained the targets of Compound C9. A one-dimensional binary vector was constructed, with aromatic amino acids in the IDR of the protein assigned a value of 1 and other amino acids assigned a value of 0. It was subjected to a wavelet transform. The transformed information includes information on the density of aromatic amino acids as a function of time (position) and frequency. Cross-correlation analysis between the wavelettransformed data of the detected proteins and the data corresponding to the C-IDR of SOX9 was used to compare the similarity to the C-IDR of SOX9. It was found from this that, in the samples treated with Compound C9, IDRs having patterns similar to that of the SOX9 C-IDR had a higher probability of having a higher similarity score (FIG. 8b). Since proteins with high similarity to the SOX9 C-IDR pattern are originally rare, in order to exclude the possibility that they were detected less frequently for that reason, the conditional probability was calculated by considering the number of proteins corresponding to each similarity score. It was found from this that IDRs with high similarity scores had a higher probability of being found in samples treated with Compound C9 (FIG. 8c). These results support the fact that Compound C9 specifically induces condensate formation according to the specific pattern (spatial frequency) created by the aromatic amino acids in the SOX9 C-IDR.

### Experimental Example 7: Analysis of SOX9 Condensates Using Super-Resolution Fluorescence Microscope dSTORM

To investigate the effect of the drugs on changes in SOX9 condensates within cells, SOX9 was labeled by IF (immunofluorescence staining), and fluorescence imaging was performed. Since transcription-factor condensates such as SOX9 condensates have a size on the order of 100 nm to 1 µm, changes in SOX9 condensates cannot be confirmed at the level of conventional fluorescence microscopy. Therefore, the fluorescence images of FIG. 9 were obtained using dSTORM (direct stochastic optical reconstruction microscopy), one of the super-resolution microscopes.

### Experimental Example 8: Evaluation of mRNA Levels of Genes Involved in Cartilage Regeneration and RNA-FISH Analysis

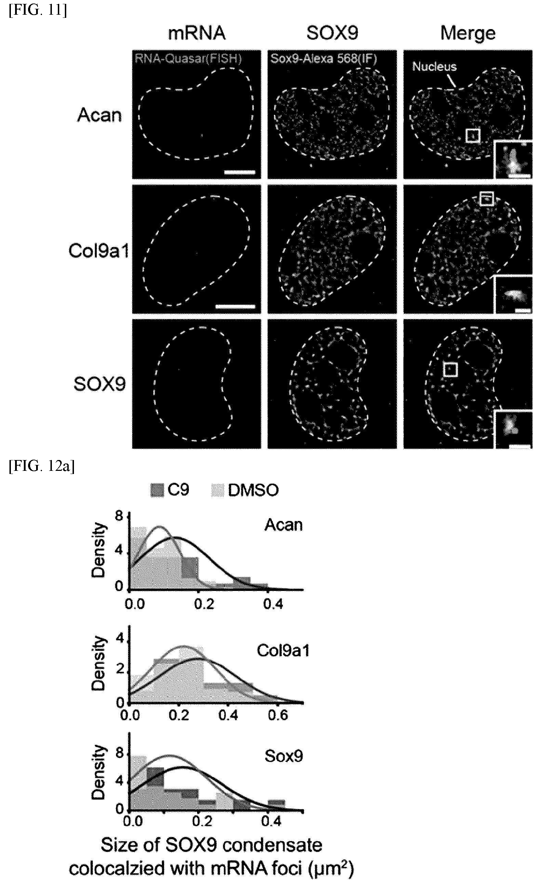

In order to investigate the relationship between the observed changes in SOX9 condensates and transcriptional activity, RNA FISH (Fluorescence In Situ Hybridization) was performed together with dSTORM for Col9a1, Acan, and SOX9, the transcripts whose mRNA levels were highly upregulated in C9-treated samples compared to DMSO, providing the mRNA-level graph of FIG. 10 and the dSTORM fluorescence images of FIG. 11.

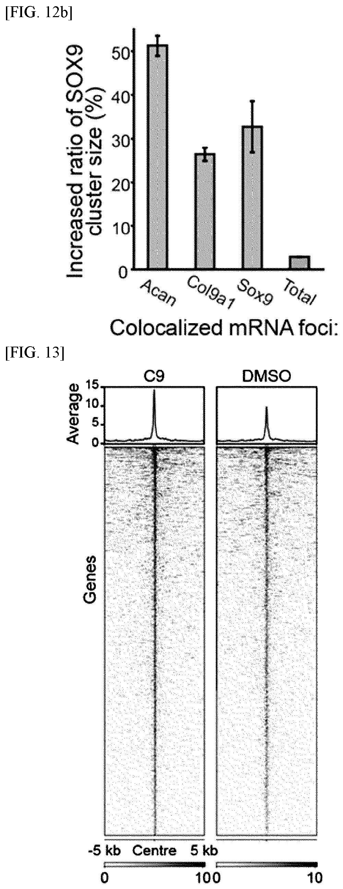

It was confirmed through RNA-FISH that the positions of the RNA foci and those of the SOX9 condensates observed within the nucleus were well colocalized (FIG. 11). Through image analysis, the size changes of SOX9 condensates observed near the RNA foci in C9 compared to DMSO showed that the size of the SOX9 condensates increased by approximately 30% to 50% (FIGS. 12a and 12b). This was a high level compared to the 2% size change of SOX9 condensates in the nucleus. Therefore, it was found that Compound C9 is involved in changing the size of SOX9 condensates near the RNA, specifically with respect to transcriptional activity.

### Experimental Example 9: Confirmation of the Effect of the Compound on the Degree of SOX9-DNA Binding Through Cut&Tag

To investigate whether the SOX9 condensates altered by the drug are actually involved in transcriptional activity, a Cut&Tag experiment was performed to identify genome-wide DNA regions bound by SOX9. It was confirmed that SOX9 binding to DNA was increased upon drug treatment (FIG. 13).

### Experimental Example 10: Confirmation of the Change in the Degree of SOX9 Binding to the DNA of Genes Involved in Cartilage Regeneration Upon Compound Treatment

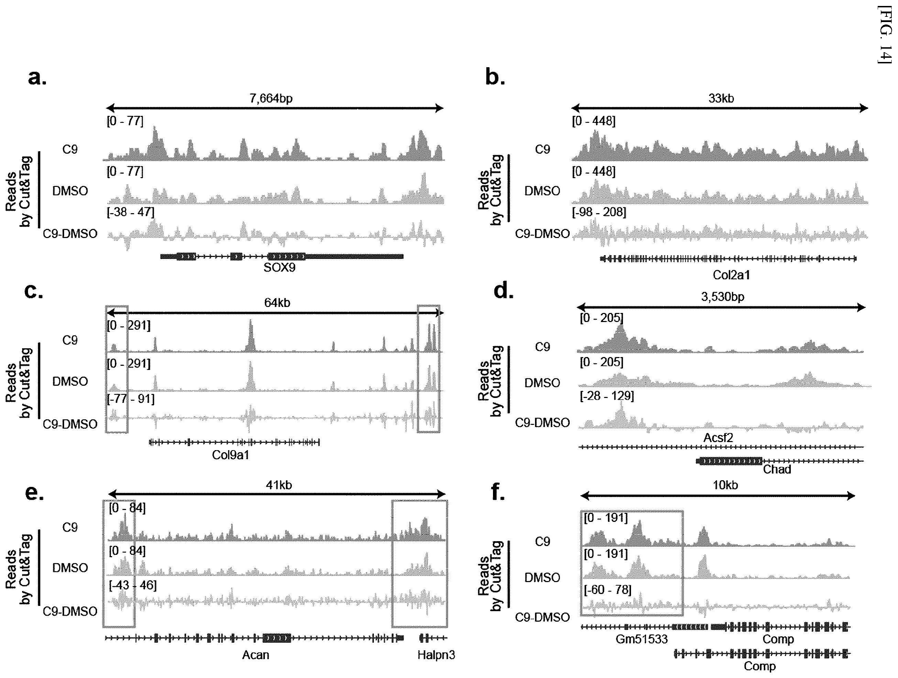

When the information on the degree of SOX9 binding to DNA obtained through Cut&Tag was confirmed for SOX9, Col2a1, Col9a1, Chad, Acan, and Comp, which are genes involved in cartilage formation, treatment with Compound C9 was found to increase SOX9 binding throughout the DNA (SOX9, Col2a1, and Chad), or to increase binding at specific regions of the DNA (red box) (Col9a1, Acan, and Comp) (data with DMSO subtracted from C9 data, in yellow) (FIGS. 14a to 14f). These results show that Compound C9 affected the size change of SOX9 condensates and the degree of DNA binding.

### Experimental Example 11: Evaluation of the Degree of Recovery of Cartilage Tissue in an Osteoarthritis Mouse Model

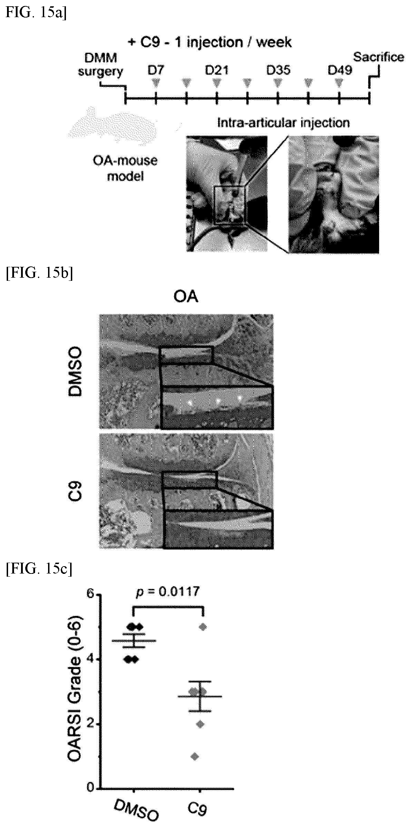

In order to confirm whether Compound C9 helps cartilage formation, an experiment was performed in which Compound C9 was injected intra-articularly a total of 7 times at intervals of 1 week so that the intra-articular concentration was 750 µM, in a mouse model of osteoarthritis (OA) induced by DMM (destabilization of the medial meniscus) surgery (FIG. 15a). In the DMM-operated mouse tissue, when DMSO was treated, recovery did not occur and damage was observed (yellow triangles). In contrast, when the drug was treated, the damaged cartilage tissue was found to have recovered (FIG. 15b). To objectively evaluate the recovery of cartilage tissue, the OARSI Grade (a higher grade indicates greater damage to the cartilage tissue) was assessed through blinded testing by individual researchers. It was confirmed that the OARSI grade was statistically significantly lower when Compound C9 was treated (FIG. 15c). This suggests that Compound C9 can help in the recovery of damaged cartilage tissue.

### Experimental Example 12: Evaluation of the Degree of Osteoarthritis Alleviation Through Behavioral Experiments

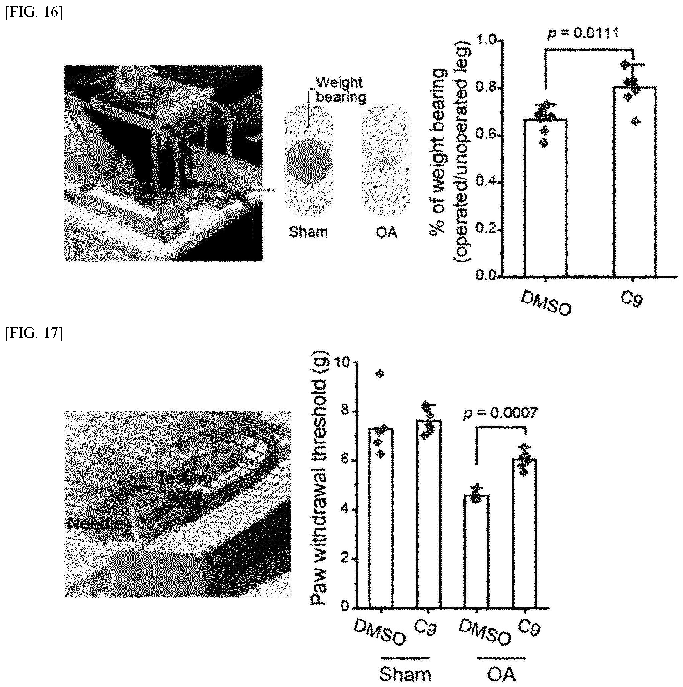

To confirm the recovery of osteoarthritis at the behavioral level, weight-bearing and Von-Frey behavioral experiments were performed. The weight-bearing experiment is an experiment that measures the degree to which the operated leg or non-operated leg bears weight upon drug treatment, to confirm the change in the degree of weight-bearing upon treatment with Compound C9 (FIG. 16). When Compound C9 was administered to DMM-induced osteoarthritis (OA) mice, the weight-bearing levels were comparable to those observed in non-OA-induced mice, and the degree of change was significant relative to the DMSO-treated group(FIG. 16).

The Von-Frey behavioral experiment assesses cartilage recovery in mice by gently stimulating the plantar surface of the paws and recording the stimulus intensity at which the mice withdraw their paws as the stimulation increases (FIG. 17). When cartilage recovery improves, the intensity of the stimulus that the mice can endure increases. Compared to mice not subjected to OA induction, OA-induced mice were found to be able to endure greater stimulus intensity upon treatment with Compound C9 (FIG. 17).

It was confirmed, through these behavioral experimental results, that Compound C9 alleviates osteoarthritis at the behavioral level. Therefore, the present invention has verified that compounds such as Compound C9, which is a cartilage-regenerating substance capable of inducing condensation of SOX9 and thereby exhibiting cartilage-regenerating effects, can be screened.

## Claims

1. A compound represented by Formula 1 below, or a stereoisomer, a solvate, or a pharmaceutically acceptable salt thereof: 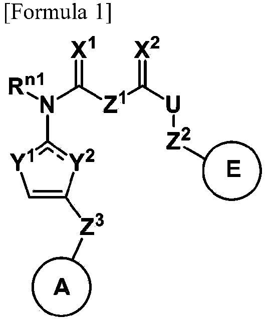 wherein, in Formula 1,
one of Y¹ and Y² is N, and the other is S, O, or NR^{a1};
X¹ and X² are each independently O, S, or NR^{a2};
U is NRⁿ², a 5- to 7-membered heterocyclyl containing 1 to 3 heteroatoms N, or a moiety in which these are linked to each other;
Z¹ is C₁₋₆ alkylene;
Z² is a direct bond, C₁₋₆ alkylene, -NRⁿ³CO-, or a 5- to 7-membered heteroaryl containing 1 to 3 heteroatoms selected from N, O, and S;
R^{a1} and R^{a2} may each independently be H or C₁₋₆ alkyl;
Rⁿ¹, Rⁿ², and Rⁿ³ are each independently H or C₁₋₆ alkyl;
when Z² is C₁₋₆ alkylene, any carbon of the alkylene is optionally substituted with halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl;
Z³ is a direct bond or -C(=O)-;
Ring A is a 6- to 14-membered aryl, a partially unsaturated 9- to 14-membered bicyclic carbocyclyl, a 5- to 12-membered heteroaryl containing 1 to 3 heteroatoms selected from O, N, or S, a 5-to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S;
Ring A is optionally substituted with 1 to 3 R^{A}; and
R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo;
Ring E is a 6- to 14-membered aryl, a partially unsaturated 9- to 14-membered bicyclic carbocyclyl, a 5- to 12-membered heteroaryl containing 1 to 3 heteroatoms selected from O, N, or S, a 5-to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl;
Ring E is optionally substituted with 1 to 3 R^{E}; and
R^{E} is selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano.

2. The compound according to claim 1, or a stereoisomer, a solvate, or a pharmaceutically acceptable salt thereof,
wherein the compound is selected from the compounds represented by Formula 2 or Formula 3 below: 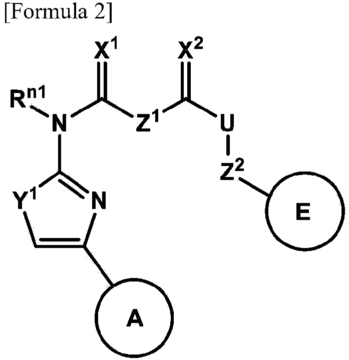 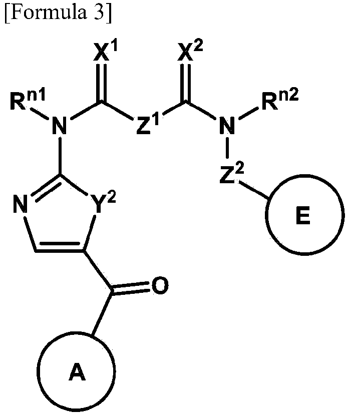
wherein, in Formulae 2 and 3,
Z¹, Z², X¹, X², U, Rⁿ¹, Rⁿ², Ring A, and Ring E are as defined in claim 1.

3. The compound according to claim 2, or a stereoisomer, a solvate, or a pharmaceutically acceptable salt thereof,
wherein Y¹ in Formula 2 and Y² in Formula 3 are each independently S or O.

4. The compound according to claim 1, or a stereoisomer, a solvate, or a pharmaceutically acceptable salt thereof,
wherein U is selected from NRⁿ²; piperidinediyl; piperazinediyl; or a combination of NR²ⁿ with piperidinediyl or piperazinediyl.

5. The compound according to claim 1, or a stereoisomer, a solvate, or a pharmaceutically acceptable salt thereof,
wherein U is selected from the group consisting of NRⁿ² and the structures shown below, in which *¹ is connected to the carbon atom of -C(=X²)-, and *² is connected to Z²:

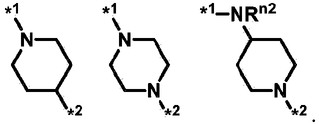

6. The compound according to claim 1, or a stereoisomer, a solvate, or a pharmaceutically acceptable salt thereof,
wherein Ring A is phenyl, biphenylyl, tetrahydronaphthyl, pyranyl, pyranonyl, benzopyranyl, or benzopyronyl.

7. The compound according to claim 1, or a stereoisomer, a solvate, or a pharmaceutically acceptable salt thereof,
wherein Ring A is 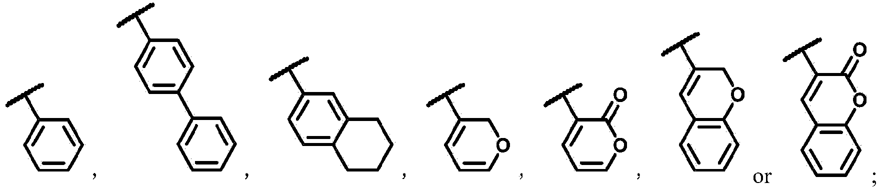 and Ring A is optionally substituted with 1 to 3 R^{A}.

8. The compound according to claim 7, or a stereoisomer, a solvate, or a pharmaceutically acceptable salt thereof,
wherein Z³ is a direct bond, and Ring A is 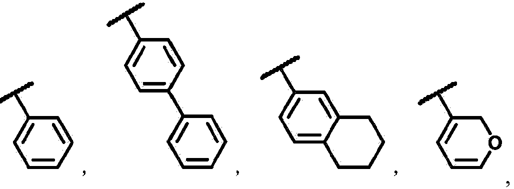 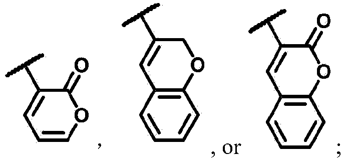 or
Z³ is -C(=O)-, and Ring A is

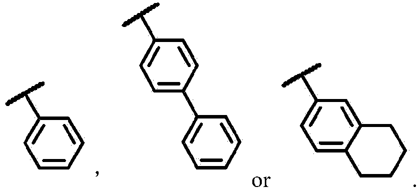

9. The compound according to claim 1, or a stereoisomer, a solvate, or a pharmaceutically acceptable salt thereof,
wherein R^{A} is selected from the group consisting of F, Cl, Br, hydroxy, methoxy, dimethylamino, methyl, and oxo.

10. The compound according to claim 1, or a stereoisomer, a solvate, or a pharmaceutically acceptable salt thereof,
wherein Ring A is selected from the chemical structures shown below:

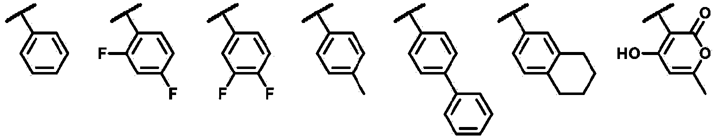

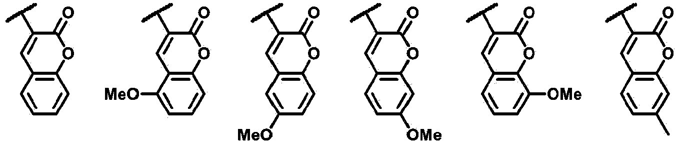

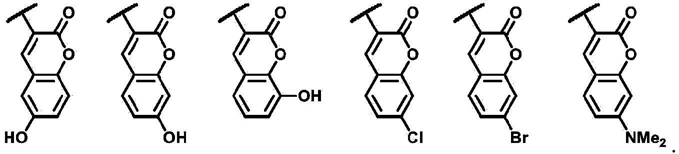

11. The compound according to claim 1, or a stereoisomer, a solvate, or a pharmaceutically acceptable salt thereof,
wherein Ring E is phenyl, naphthalenyl, indanyl, pyridinyl, pyridazinyl, pyrimidinyl, pyrazinyl, imidazolyl, pyrazolyl, triazolyl, thiophenyl, thiazolyl, isothiazolyl, oxazolyl, isoxazolyl, morpholinyl, thiomorpholinyl, piperidinyl, hexahydroxypyridazinyl, hexahydroxypyrimidinyl, piperazinyl, indolyl, indazolyl, benzoimidazolyl, quinolinyl, isoquinolinyl, cinnolinyl, quinazolinyl, quinoxalinyl, phthalazinyl, or benzodioxolyl.

12. The compound according to claim 1, or a stereoisomer, a solvate, or a pharmaceutically acceptable salt thereof,
wherein Ring E is selected from the chemical structures shown below, and Ring E is optionally substituted with 1 to 3 R^{E}:

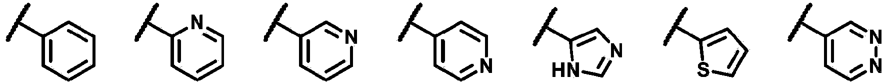

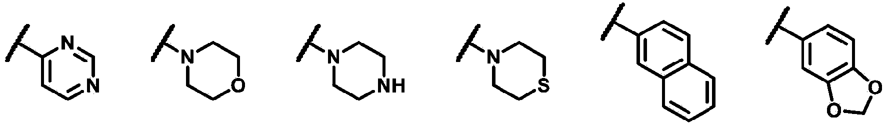

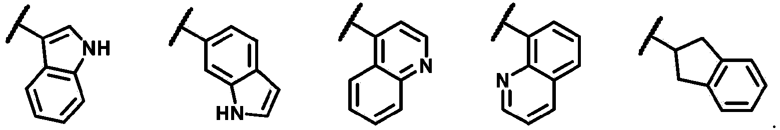

13. The compound according to claim 1, or a stereoisomer, a solvate, or a pharmaceutically acceptable salt thereof,
wherein R^{E} is selected from the group consisting of methoxy, hydroxy, amino, F, Cl, Br, trifluoromethyl, cyano, hydroxymethyl, methyl, fluorosulfonyl, nitro, and oxo.

14. The compound according to claim 1, or a stereoisomer, a solvate, or a pharmaceutically acceptable salt thereof,
wherein Ring E is selected from the chemical structures shown below:

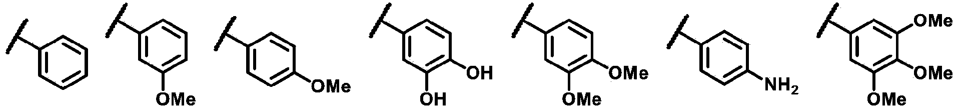

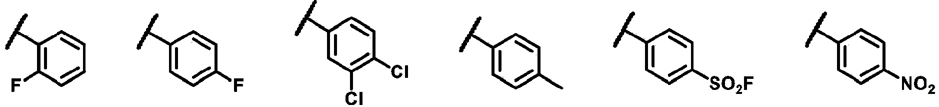

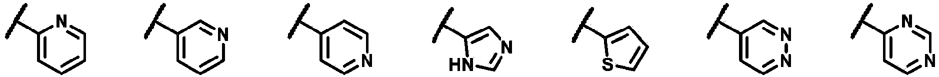

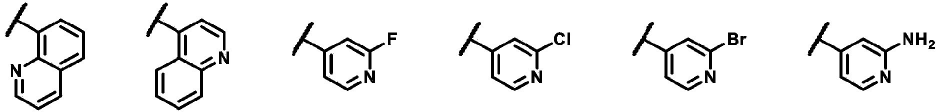

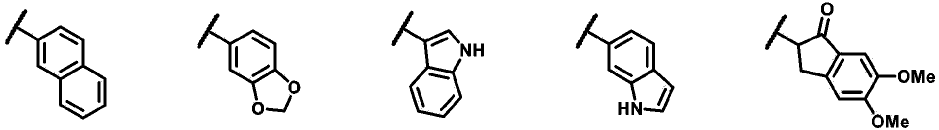

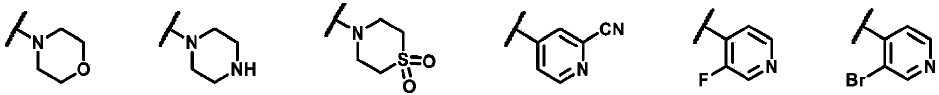

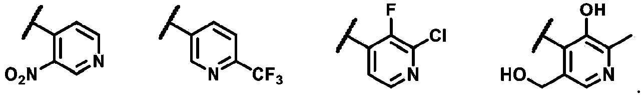

15. The compound according to claim 1, or a stereoisomer, a solvate, or a pharmaceutically acceptable salt thereof,
wherein the compound is represented by Formula I, Formula II, or Formula III below: 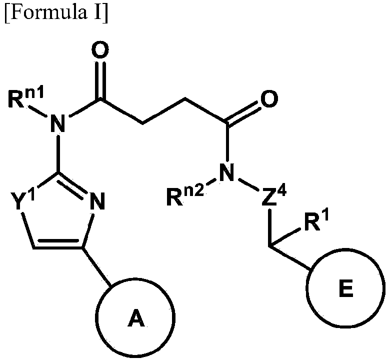
wherein, in Formula I,
Y¹ is S, O, or NR^{a1};
R^{a1} is H or C₁₋₆ alkyl;
Rⁿ¹ and Rⁿ² are each independently H or C₁₋₆ alkyl;
Z⁴ is a direct bond or C₁₋₄ alkylene;
R¹ is H, halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl;
Ring A is a 6- to 14-membered aryl, a partially unsaturated 9- to 14-membered bicyclic carbocyclyl, a 6- to 12-membered heteroaryl containing 1 to 3 heteroatoms selected from O, N, or S, a 5-to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S;
Ring A is optionally substituted with 1 to 3 R^{A}; and
R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo;
Ring E is a 6- to 14-membered aryl, a partially unsaturated 9- to 14-membered bicyclic carbocyclyl, a 5- to 12-membered heteroaryl containing 1 to 3 heteroatoms selected from O, N, or S, a 5-to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl;
Ring E is optionally substituted with 1 to 3 R^{E}; and
R^{E} is selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano; 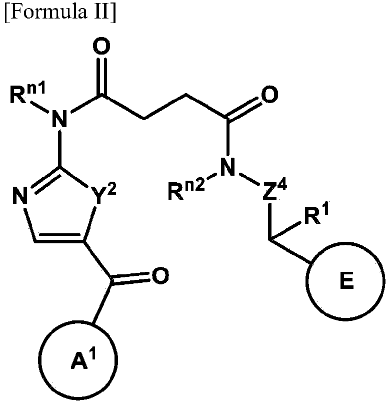
wherein, in Formula II,
Y² is S, O, or NR^{a1};
R^{a1} is H or C₁₋₆ alkyl;
Rⁿ¹ and Rⁿ² are each independently H or C₁₋₆ alkyl;
Z⁴ is a direct bond or C₁₋₄ alkylene;
R¹ is H, halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, cyano, nitro, oxo, or C₆₋₁₂ aryl;
Ring A¹ is a 6- to 14-membered aryl or a partially unsaturated 9- to 14-membered bicyclic carbocyclyl;
Ring A¹ is optionally substituted with 1 to 3 R^{A}; and
R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo;
Ring E is a 6- to 14-membered aryl, a 5- to 12-membered heteroaryl containing 1 to 3 heteroatoms selected from O, N, or S, a 5- to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl;
Ring E is optionally substituted with 1 to 3 R^{E}; and
R^{E} is selected from the group consisting of halo, hydroxy, C₁₋₆ alkoxy, amino, C₁₋₆ alkylamino, di(C₁₋₆ alkyl)amino, C₁₋₆ alkyl, carboxy, C₁₋₆ alkoxycarbonyl, carbamoyl, C₁₋₆ alkylcarbamoyl, di(C₁₋₆ alkyl)carbamoyl, halosulfonyl, sulfoxy, C₁₋₆ alkylsulfonyl, cyano, nitro, and oxo; 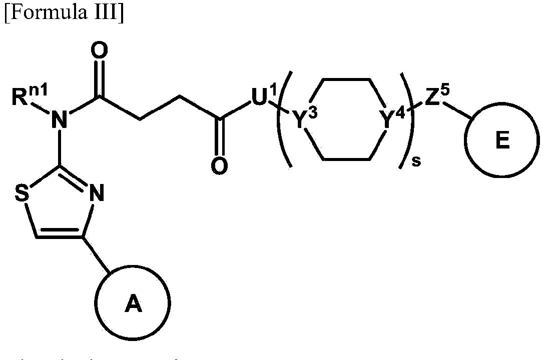
wherein, in Formula III,
U¹ is a direct bond or NRⁿ²;
at least one of Y³ and Y⁴ is N, and the other is CH;
Z⁵ is a direct bond, C₁₋₆ alkylene, -NRⁿ³CO-, or a 5- to 7-membered heteroaryl containing 1 to 3 heteroatoms selected from N, O, and S;
Rⁿ¹, Rⁿ², and Rⁿ³ are each independently H or C₁₋₆ alkyl;
Ring A is a 6- to 14-membered aryl, a partially unsaturated 9- to 14-membered bicyclic carbocyclyl, a 5- to 12-membered heteroaryl containing 1 to 3 heteroatoms selected from O, N, or S, a 5-to 8-membered heterocyclyl containing 1 to 3 heteroatoms selected from O, N, or S, or a fused benzoheterocyclyl in which a benzene ring is fused to a 5- to 7-membered heterocyclyl containing 1 to 3 heteroatoms selected from O, N, or S;
Ring A is optionally substituted with 1 to 3 R^{A}; and
R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo;
Ring E is a 6- to 14-membered aryl, a partially unsaturated 9- to 14-membered bicyclic carbocyclyl, a 5- to 12-membered heteroaryl containing 1 to 3 heteroatoms selected from O, N, or S, a 5-to 12-membered heterocyclyl containing 1 to 3 heteroatoms selected from O, N, or S, or a fused heterocycloaryl in which a 5- to 7-membered heterocyclic ring containing 1 to 3 heteroatoms selected from O, N, or S is fused to phenyl;
Ring E is optionally substituted with 1 to 3 R^{E}; and
R^{E} is selected from the group consisting of halo; hydroxy; C₁₋₆ alkoxy; amino; C₁₋₆ alkylamino; di(C₁₋₆ alkyl)amino; carboxy; C₁₋₆ alkoxycarbonyl; carbamoyl; C₁₋₆ alkylcarbamoyl; di(C₁₋₆ alkyl)carbamoyl; halosulfonyl; sulfoxy; C₁₋₆ alkylsulfonyl; cyano; nitro; oxo; or C₁₋₆ alkyl optionally substituted with one or more selected from halo, hydroxy, C₁₋₆ alkoxy, amino, C₁₋₆ alkylamino, di(C₁₋₆ alkyl)amino, nitro, and cyano; and
s is 0 or 1.

16. The compound according to claim 15, or a stereoisomer, a solvate, or a pharmaceutically acceptable salt thereof,
wherein the compound is represented by Formula IA, Formula IB, Formula IC, Formula ID, Formula IE, or Formula IIA: 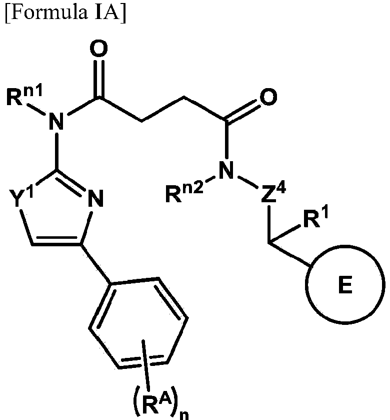 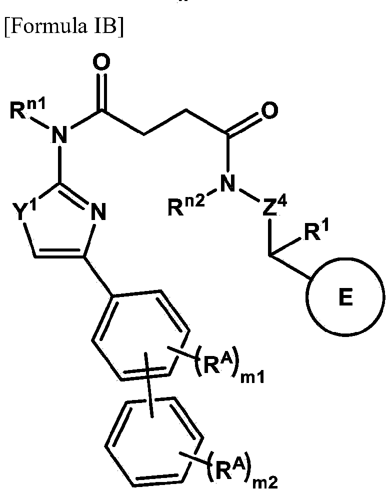 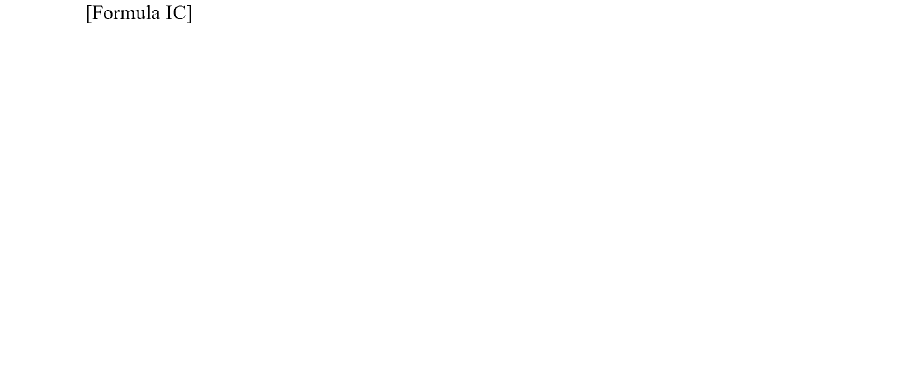 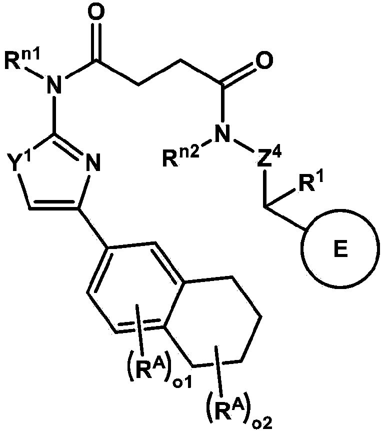 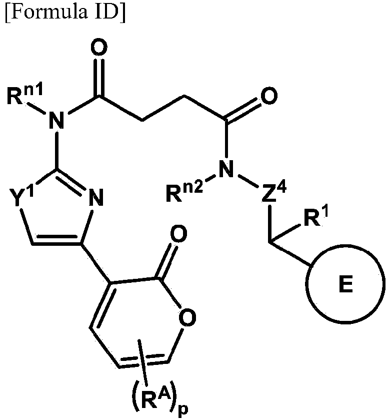 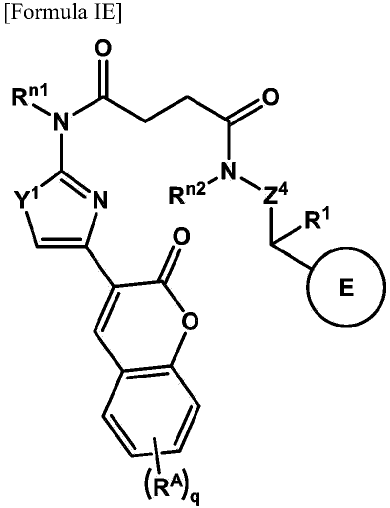 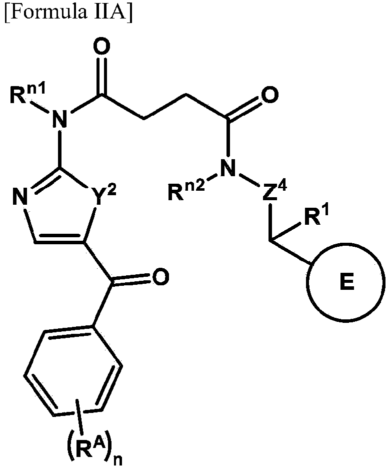
wherein, in Formula IA, Formula IB, Formula IC, Formula ID, Formula IE, or Formula IIA,
R^{A} is selected from the group consisting of halo, hydroxy, C₁₋₆ alkoxy, amino, C₁₋₆ alkylamino, di(C₁₋₆ alkyl)amino, C₁₋₆ alkyl, cyano, nitro, and oxo;
n, m1, m2, o1, o2, p, and q are each independently an integer of 0 to 3;
the sum of m1 and m2 and the sum of o1 and o2 are each 3 or less; and
Y¹, Y², Rⁿ¹, Rⁿ², Z⁴, R¹, and Ring E are as defined in claim 13.

17. The compound according to claim 1, or a stereoisomer, a solvate, or a pharmaceutically acceptable salt thereof,
wherein the compound is selected from the compounds represented by the chemical structures shown below:

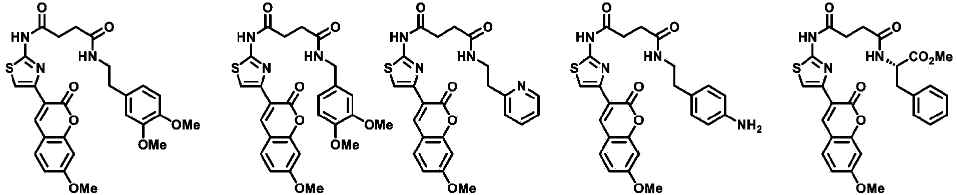

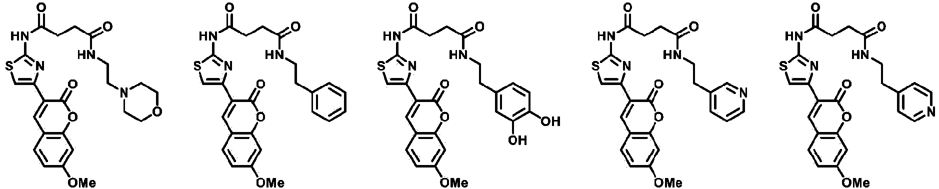

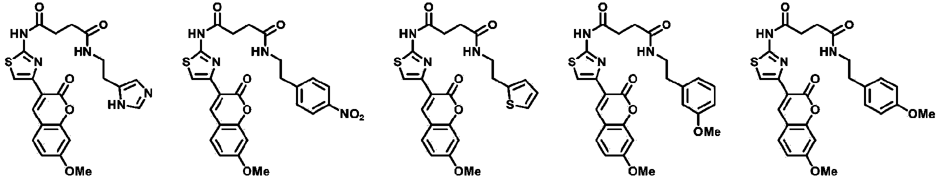

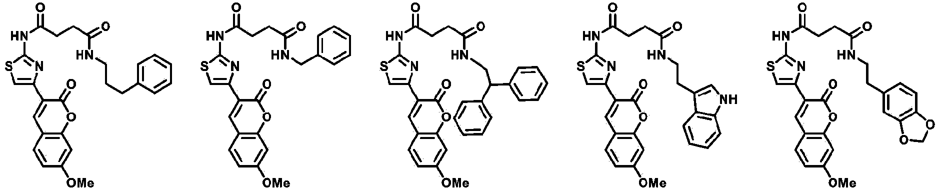

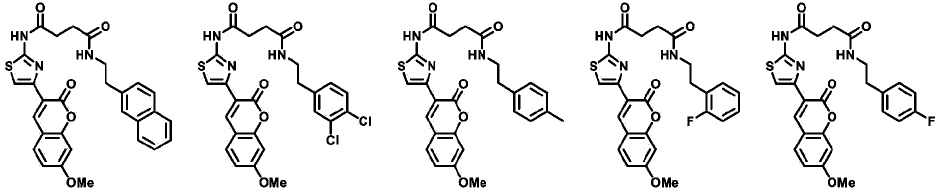

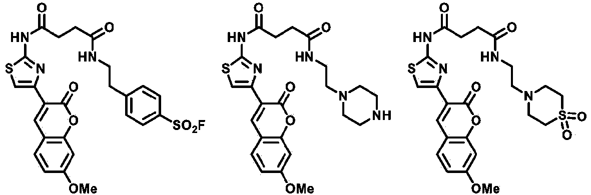

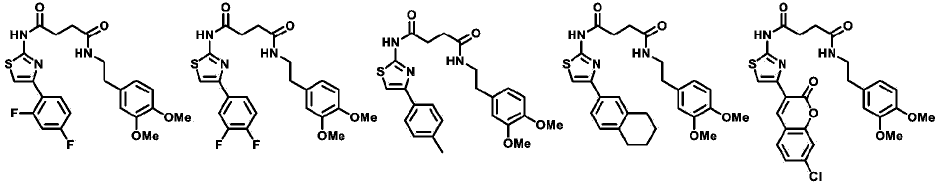

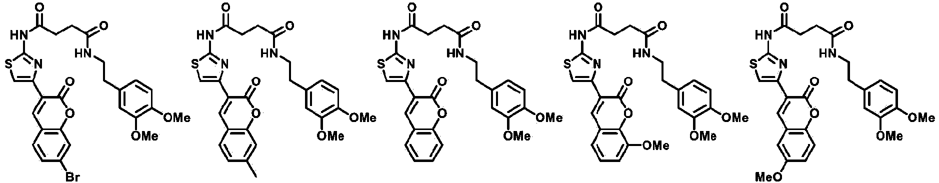

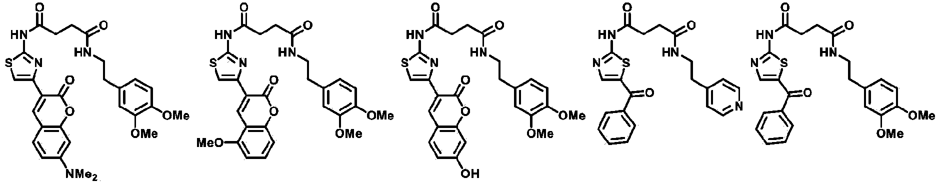

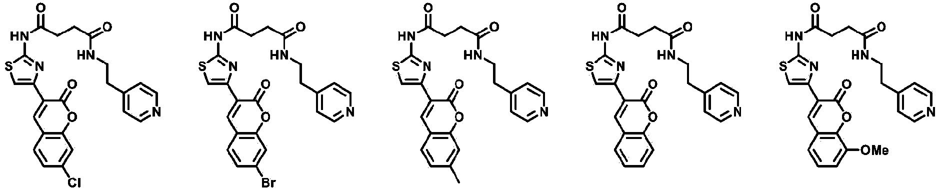

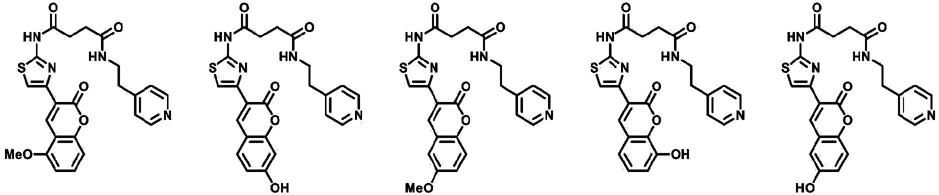

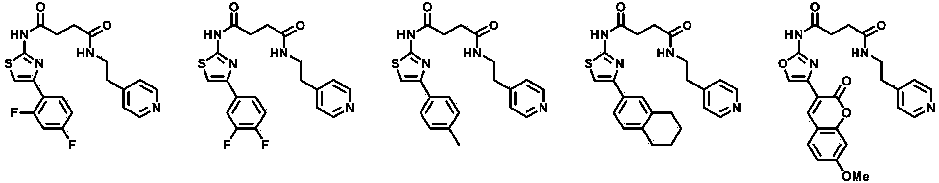

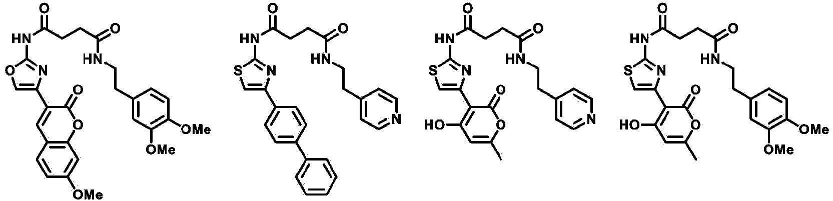

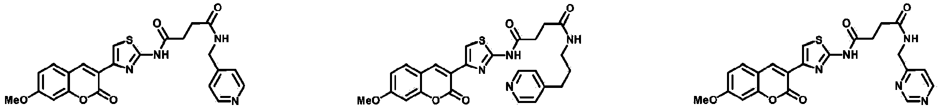

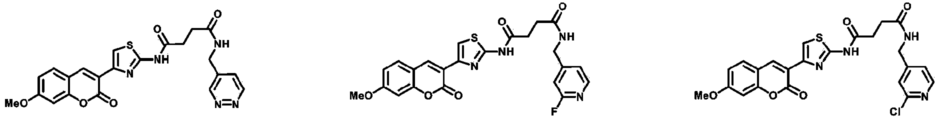

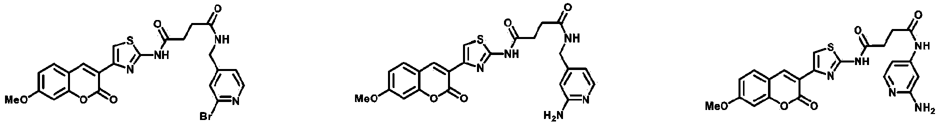

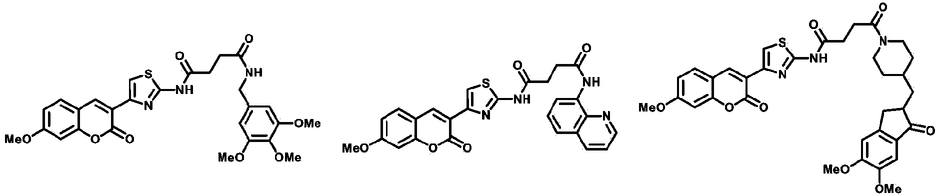

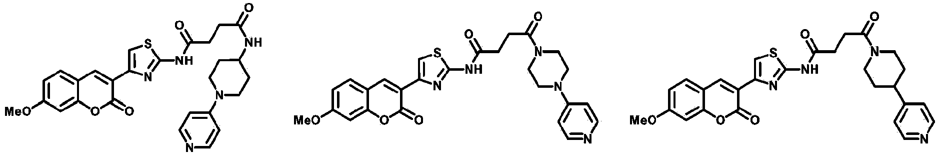

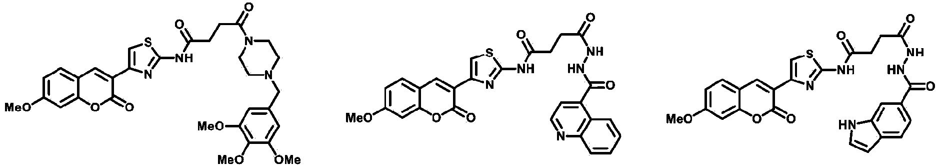

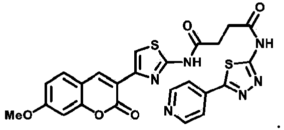

18. A pharmaceutical composition for preventing or treating diseases associated with reduced expression of SOX9 (SRY-Box Transcription Factor 9), comprising the compound according to any one of claims 1 to 17, or a stereoisomer, a solvate, or a pharmaceutically acceptable salt thereof.

19. The pharmaceutical composition according to claim 18,
wherein the disease associated with reduced expression of SOX9 is osteoarthritis.

20. A method of treating a disease associated with reduced expression of SOX9, comprising administering to an individual the compound according to any one of claims 1 to 17, or a stereoisomer, a solvate, or a pharmaceutically acceptable salt thereof.

21. Use of the compound according to any one of claims 1 to 17, or a solvate, a stereoisomer, or a pharmaceutically acceptable salt thereof, for use in the prevention or treatment of a disease associated with reduced expression of SOX9.

22. Use of the compound according to any one of claims 1 to 17, or a solvate, a stereoisomer, or a pharmaceutically acceptable salt thereof, for use in the manufacture of a medicament for preventing or treating a disease associated with reduced expression of SOX9.
